# EUROPEAN PATENT APPLICATION

(11) **EP 4 180 034 A1**
(43) Date of publication of application: **17.05.2023**
(21) Application number: 21838031.9
(22) Date of filing: 09.07.2021
(51) Int. Cl.: A61K 31/343, A61K 45/06, A61P 35/00

(54) **PHARMACEUTICAL COMPOSITION FOR PREVENTING OR TREATING CANCER COMPRISING NAPHTHOQUINONE-BASED COMPOUND AND IMMUNE CHECKPOINT INHIBITOR AS ACTIVE INGREDIENTS**

(30) Priority: 10.07.2020 KR 20200085711
(71) Applicant: NADIANBIO Ltd., Iksan-si Jeollabuk-do 54538 (KR)
(72) Inventor: SO, Hong Seob, Iksan-si Jeollabuk-do 54569 (KR); KWAK, Tae Hwan, Yongin-si Gyeonggi-do 17103 (KR)
(74) Representative: HGF
(86) International application number: PCT/KR2021/008830
(87) International publication number: WO 2022/010322

(57) **Abstract**

The present invention is a pharmaceutical composition for preventing or treating cancer, containing, as active ingredients, a naphthoquinone-based compound, and at least one selected from among an immune checkpoint inhibitor and an immunogenic cell death inducer, and the pharmaceutical composition has a significantly higher anticancer effect compared to the case where each formulation is administered alone. Accordingly, an anticancer synergistic effect of inhibiting the growth in cancer cells and inhibiting the metastasis of the cancer cells, in which an anticancer effect is insignificant when the existing anticancer agent is administered alone, may be expected. Therefore, the composition containing the naphthoquinone-based compound may be usefully used as a drug for preventing or treating cancer, together with the immune checkpoint inhibitor and/or the immunogenic cell death inducer.

## Description

### BACKGROUND

The present invention relates to a pharmaceutical composition for preventing or treating cancer, the pharmaceutical composition containing, as active ingredients: a naphthoquinone-based compound, a pharmaceutically acceptable salt thereof, a prodrug thereof, a solvate thereof, or an isomer thereof; and an immune checkpoint inhibitor and/or an immunogenic cell death (ICD) inducer.

Most human tumors evade the immune surveillance of a subject and are thus difficult to treat, and the causes thereof include 1) loss of cancer antigen expression, 2) a chronic inflammatory environment, 3) inhibition of T cell influx, 4) inhibition of the antigen presentation capability of dendritic cells by a cancer environment, 5) a cancer immunosuppressive environment composed of immune checkpoints, immune-suppressing cytokines, and immune-suppressing cells, and the like.

Meanwhile, anticancer immunotherapy has the advantage of using the patient's own immune system to obtain long-term antitumor immunity with few side effects. The purpose of the immunotherapy is to generate tumor-specific cytotoxic T lymphocytes (CTLs) capable of recognizing tumor cells or tumor antigens and eliminating tumor cells. That is, a tumor antigen peptide is loaded into a major histocompatibility complex (MHC) and presented to T lymphocytes by tumor cells themselves or antigen-presenting cells, to activate the T lymphocytes and induce differentiation into CTLs and increase in CTLs.

Recently, as one method for enhancing such an anticancer immune response and overcoming some cancer immunosuppressive environments, antibodies that inhibit immune checkpoints, such as CTLA-4 and PD-1, are being clinically applied as anticancer agents. However, single anticancer immunotherapy using a CTLA-4, PD-1, or PD-L1 inhibitor still has limitations, such as showing excellent effects only in specific cancer and showing therapeutic effects only in some patients (Korean Patent Publication No. 10-2020-0055116).

The immune checkpoint inhibitors are typically known to cause mild adverse reactions compared to existing cytotoxic anticancer agents or targeted therapeutic agents, but infrequently cause fatal or permanent functional impairment and may cause various autoimmune diseases due to the immunomodulatory action thereof. Among the immune checkpoint inhibitors, a CTLA-4 inhibitor is known to cause more adverse reactions than a PD-1 inhibitor or a PD-L1 inhibitor, and when a CTLA-4 inhibitor and a PD-1 inhibitor are combined, the frequency and severity of adverse reactions increase. Another problem is that when an immune checkpoint inhibitor is used, not only good therapeutic responses are shown, but also tumor hyperprogression, in which the disease condition rapidly worsens due to sudden growth of the tumor after administration of the immune checkpoint inhibitor, may be caused in some patients.

As a strategy to overcome the limitations of immune checkpoint inhibitors, combined clinical trials with anticancer chemotherapy, radiotherapy, and targeted therapeutic agents, which may promote immunogenic cell death of tumor cells, are being attempted. The immunogenic cell death is a form of cell death in which dying cells may produce *in vivo* antigens to activate adaptive immunity. Such immunogenic cell death may be induced by specific external stimuli such as anticancer agents or radiation therapy. Damage-associated molecular patterns (DAMPs) released by these stimuli are recognized by pathogen recognition receptors of the innate or adaptive immune response and transmit a danger signal to the living body.

So far, six types of DAMPs, including calreticulin, ATP, high mobility group box 1 (HMGB1), type 1 IFN, cancer cell-derived nucleic acid, and annexin A1, have been known to be involved in immune-induced cell death. For example, DAMPs such as calreticulin, ATP, and HMGB1 are normally located in cells. When a stimulus promoting immunogenic cell death of tumor cells is applied, novel cancer antigens are exposed and DAMPs are released out of the cells. Consequently, dendritic cells are attracted to tumor cells, the antigen presentation capability of the dendritic cells is enhanced, and activation of T cells and infiltration into the tumor are promoted, and thus the antitumor effect caused by the immune checkpoint inhibitor may be increased (Guido Kroemer et. al., Annu. Rev. Immunol., 31:51-72, 2013).

### SUMMARY

Accordingly, as a result of studying a composition for treating cancer and a treatment method, which may further effectively enhance an anticancer immune response, the present inventors have confirmed that a naphthoquinone-based compound exhibits an excellent anticancer effect when administered together with an immune checkpoint inhibitor and/or an immunogenic cell death inducer, thereby completing the present invention.

One aspect of the present invention provides a pharmaceutical composition for preventing or treating cancer, the pharmaceutical composition containing, as active ingredients: a naphthoquinone-based compound; and at least one selected from among an immune checkpoint inhibitor and an immunogenic cell death inducer.

When the naphthoquinone-based compound according to the present invention was administered in combination with the immune checkpoint inhibitor and/or the immunogenic cell death inducer, a significantly higher anticancer effect was exhibited compared to the case where each ingredient was administered alone. Accordingly, even in cancer cells in which an anticancer effect is insignificant when the existing anticancer agent is administered alone, the effect of inhibiting the metastasis of the cancer cells as well as inhibiting the growth of the cancer cells may be expected. Therefore, the composition containing the naphthoquinone-based compound may be usefully used as a drug for preventing or treating cancer, together with the immune checkpoint inhibitor and/or the immunogenic cell death inducer.

### BRIEF DESCRIPTION OF THE DRAWINGS

Exemplary embodiments may be understood in more detail from the following description taken in conjunction with the accompanying drawings, in which:
FIG. 1 is a graph showing the sizes of tumors in animal models (MC38) for colorectal cancer according to sole administration or co-administration of respective drugs (Dun: dunnione / Anti-PD-1: anti-PD-1 antibody / 5-FU: 5-fluorouracil / OX: oxaliplatin);
FIG. 2 is a graph showing the sizes of tumors in the animal models (MC38) for colorectal cancer according to sole administration or co-administration of respective drugs (Dun: dunnione / Anti-CTLA4: anti-CTLA4 antibody / 5-FU: 5-fluorouracil / OX: oxaliplatin);
FIG. 3 is a graph showing the sizes of tumors in the animal models (MC38) for colorectal cancer according to sole administration or co-administration of respective drugs (Dun: dunnione / Anti-PD-1: anti-PD-1 antibody / Anti-CTLA4: anti-CTLA4 antibody / 5-FU: 5-fluorouracil / OX: oxaliplatin);
FIG. 4 is a graph showing the sizes of tumors in the animal models (MC38) for colorectal cancer according to sole administration or co-administration of respective drugs (Dun: dunnione / Anti-LAG3: anti-LAG3 antibody / OX: oxaliplatin);
FIG. 5 is a graph showing the sizes of tumors in the animal models (MC38) for colorectal cancer according to sole administration or co-administration of respective drugs (Dun: dunnione / Anti-PD-1: anti-PD-1 antibody);
FIG. 6 is a graph showing the sizes of tumors in the animal models (MC38) for colorectal cancer according to sole administration or co-administration of respective drugs (Dun: dunnione / Anti-PD-1: anti-PD-1 antibody / Rego: regorafenib);
FIG. 7 is a graph showing the sizes of tumors in the animal models (MC38) for colorectal cancer according to sole administration or co-administration of respective drugs (Dun: dunnione / Anti-PD-1: anti-PD-1 antibody / Abe: abemaciclib);
FIG. 8 is a graph showing the sizes of tumors in animal models (CT26) for colorectal cancer according to sole administration or co-administration of respective drugs (Dun: dunnione / FOLFIRI: irinotecan + leucovorin + 5-fluorouracil);
FIG. 9 is a graph showing the sizes of tumors in the animal models (CT26) for colorectal cancer according to sole administration or co-administration of respective drugs (Dun: dunnione / Anti-PD-1: anti-PD-1 antibody);
FIG. 10 is a graph showing the sizes of tumors in animal models (4T1) for breast cancer according to sole administration or co-administration of respective drugs (Dun: dunnione / Anti-CTLA4: anti-CTLA4 antibody / MIT: mitoxantrone);
FIG. 11 is a graph showing the sizes of tumors in the animal models (4T1) for breast cancer according to sole administration or co-administration of respective drugs (Dun: dunnione / Anti-PD-1: anti-PD-1 antibody / ADR: adriamycin);
FIG. 12 is a graph showing the sizes of tumors in the animal models (4T1) for breast cancer according to sole administration or co-administration of respective drugs (Dun: dunnione / ACP: adriamycin + cyclophosphamide + paclitaxel);
FIG. 13 is a graph showing the sizes of tumors in the animal models (4T1) for breast cancer according to sole administration or co-administration of respective drugs (Dun: dunnione / TAC: docetaxel + adriamycin + cyclophosphamide);
FIG. 14 is a graph showing the sizes of tumors in animal models (EMT6) for breast cancer according to sole administration or co-administration of respective drugs (Dun: dunnione / Anti-CTLA4: anti-CTLA4 antibody / LAPA: lapatinib);
FIG. 15 is a graph showing the sizes of tumors in animal models (RENCA) for renal cancer according to sole administration or co-administration of respective drugs (Dun: dunnione / Anti-CTLA4: anti-CTLA4 antibody / EVE: everolimus);
FIG. 16 is a graph showing the sizes of tumors in the animal models (RENCA) for renal cancer according to sole administration or co-administration of respective drugs (Dun: dunnione / Sorafenib: sorafenib);
FIG. 17 is a graph showing the sizes of tumors in the animal models (RENCA) for renal cancer according to sole administration or co-administration of respective drugs (Dun: dunnione / Anti-CTLA4: anti-CTLA4 antibody / Sorafenib: sorafenib);
FIG. 18 is a graph showing the sizes of tumors in the animal models (RENCA) for renal cancer according to sole administration or co-administration of respective drugs (Dun: dunnione / Anti-PD-1: anti-PD-1 antibody / EVE: everolimus);
FIG. 19 is a graph showing the sizes of tumors in the animal models (RENCA) for renal cancer according to sole administration or co-administration of respective drugs (Dun: dunnione / Anti-CTLA4: anti-CTLA4 antibody / Sorafenib: sorafenib);
FIG. 20 is a graph showing the sizes of tumors in the animal models (RENCA) for renal cancer according to sole administration or co-administration of respective drugs (Dun: dunnione / Anti-CTLA4: anti-CTLA4 antibody / VIS: vistusertib);
FIG. 21 is a graph showing the sizes of tumors in animal models (B16F10) for skin cancer according to sole administration or co-administration of respective drugs (Dun: dunnione / Anti-PD-L1: anti-PD-L1 antibody);
FIG. 22 is a graph showing the sizes of tumors in the animal models (B16F10) for skin cancer according to sole administration or co-administration of respective drugs (Dun: dunnione / Anti-PD-L1: anti-PD-L1 antibody / PTX: paclitaxel);
FIG. 23 is a graph showing the sizes of tumors in the animal models (B16F10) for skin cancer according to sole administration or co-administration of respective drugs (Dun: dunnione / Anti-CTLA4: anti-CTLA4 antibody / PTX: paclitaxel);
FIG. 24 is a graph showing the sizes of tumors in the animal models (B16F10) for skin cancer according to sole administration or co-administration of respective drugs (Dun: dunnione / Anti-CTLA4: anti-CTLA4 antibody / Rego: regorafenib);
FIG. 25 is a graph showing the sizes of tumors in the animal models (B16F10) for skin cancer according to sole administration or co-administration of respective drugs (Dun: dunnione / Anti-PD-1: anti-PD-1 antibody / VIS: vistusertib);
FIG. 26 is a graph showing the sizes of tumors in the animal models (B16F10) for skin cancer according to sole administration or co-administration of respective drugs (Dun: dunnione / EVE: everolimus);
FIG. 27 is a graph showing the sizes of tumors in the animal models (B16F10) for skin cancer according to sole administration or co-administration of respective drugs (Dun: dunnione / Anti-PD-1: anti-PD-1 antibody / EVE: everolimus);
FIG. 28 is a graph showing the sizes of tumors in animal models (Hepa1-6) for liver cancer according to sole administration or co-administration of respective drugs (Dun: dunnione / Anti-CTLA4: anti-CTLA4 antibody / SORA: sorafenib);
FIG. 29 is a graph showing the sizes of tumors in the animal models (Hepa1-6) for liver cancer according to sole administration or co-administration of respective drugs (Dun: dunnione / Anti-CTLA4: anti-CTLA4 antibody / EVE: everolimus);
FIG. 30 is a graph showing the sizes of tumors in animal models (KLN205) for lung cancer according to sole administration or co-administration of respective drugs (Dun: dunnione / Anti-PD-1: anti-PD-1 antibody / CP: carboplatin + paclitaxel);
FIG. 31 is a graph showing the sizes of tumors in animal models (LL/2) for lung cancer according to sole administration or co-administration of respective drugs (Dun: dunnione / Anti-PD-1: anti-PD-1 antibody / CaPe: carboplatin + pemetrexed);
FIG. 32 is a photograph obtained by photographing photon emission amount, as the degree of tumor metastasis in animal models (4T1-luciferase) for breast cancer metastasis according to sole administration or co-administration of respective drugs (Dun: dunnione / Anti-CTLA4: anti-CTLA4 antibody / MIT: mitoxantrone / CTRL: control);
FIG. 33 is a graph showing the survival rates of the animal models (MC38) for colorectal cancer according to sole administration or co-administration of respective drugs (Dun: dunnione / Anti-PD-1: anti-PD-1 antibody / Anti-CTLA4: anti-CTLA4 antibody / 5-FU: 5-fluorouracil / OX: oxaliplatin);
FIG. 34 is a graph showing the survival rates of the animal models (MC38) for colorectal cancer according to sole administration or co-administration of respective drugs (Dun: dunnione / Anti-PD-1: anti-PD-1 antibody / 5-FU: 5-fluorouracil);
FIG. 35 schematically illustrates the construction of models for spontaneous non-small cell lung cancer and the schedule of sole administration or co-administration of respective drugs (Dun: dunnione / Anti-PD-1: anti-PD-1 antibody / Carboplatin: carboplatin / Paclitaxel: paclitaxel);
FIG. 36 schematically illustrates a drug administration schedule for the analysis of immune-related adverse events (irAEs) according to sole administration or co-administration of respective drugs (Dun: dunnione / Ipilimumab: ipilimumab / αPD-1: anti-PD-1 antibody / Anti-human IgG: anti-human IgG);
FIG. 37 shows the incidence of cardiac hypertrophy as an adverse reaction according to sole administration or co-administration of respective drugs (Anti-human IgG: anti-human IgG / Ipilimumab: ipilimumab / αPD-1: anti-PD-1 antibody / Dun: dunnione);
FIG. 38 shows the incidence of hepatitis as an adverse reaction according to sole administration or co-administration of respective drugs (Anti-human IgG: anti-human IgG / Ipilimumab: ipilimumab / αPD-1: anti-PD-1 antibody / Dun: dunnione / ALT: alanine aminotransferase / AST: aspartate aminotransferase);
FIG. 39 shows the incidence of pneumonia as an adverse reaction according to sole administration or co-administration of respective drugs (Anti-human IgG: anti-human IgG / Ipilimumab: ipilimumab / αPD-1: anti-PD-1 antibody / Dun: dunnione);
FIG. 40 shows the incidence of pancytopenia as an adverse reaction according to sole administration or co-administration of respective drugs (Anti-human IgG: anti-human IgG / Ipilimumab: ipilimumab / αPD-1: anti-PD-1 antibody / Dun: dunnione / WBC: white blood cell / Monocyte: monocyte / Lymphocyte: lymphocyte / HCT: hematocrit / RBC: red blood cell / Hemoglobin: hemoglobin);
FIG. 41 schematically illustrates a drug administration schedule for the analysis of immune-related adverse events according to co-administration of respective drugs. Here, the administration of cyclophosphamide is based on metronomic anticancer therapy (Dun: dunnione / Ipilimumab: ipilimumab / αPD-1: anti-PD-1 antibody / CTX: cyclophosphamide / Anti-human IgG: anti-human IgG);
FIG. 42 shows the incidence of heart failure as an adverse reaction according to co-administration of respective drugs (Anti-human IgG: anti-human IgG / Ip: ipilimumab / αPD-1: anti-PD-1 antibody / Dun: dunnione / CTX: cyclophosphamide / LVIDd: left ventricular internal diameter-diastolic / LVIDs: left ventricular internal diameter-systolic / Fraction Shortening: fractional shortening / Ejection Fraction: ejection fraction);
FIG. 43 shows the degree of decrease in ovary size as an adverse reaction according to co-administration of respective drugs (Anti-human IgG: anti-human IgG / Ipilimumab: ipilimumab / αPD-1: anti-PD-1 antibody / Dun: dunnione);
FIG. 44 shows the incidence of pneumonia as an adverse reaction according to co-administration of respective drugs (Anti-human IgG: anti-human IgG / Ipilimumab: ipilimumab / αPD-1: anti-PD-1 antibody / Dun: dunnione);
FIG. 45 shows the incidence of nephritis as an adverse reaction according to co-administration of respective drugs (Anti-human IgG: anti-human IgG / Ipilimumab: ipilimumab / αPD-1: anti-PD-1 antibody / Dun: dunnione); and
FIG. 46 shows the incidence of pancytopenia as an adverse reaction according to co-administration of respective drugs (Anti-human IgG: anti-human IgG / Ip: ipilimumab / αPD-1: anti-PD-1 antibody / CTX: cyclophosphamide / Dun: dunnione / WBC: white blood cell / Lymphocyte: lymphocyte / Monocyte: monocyte / RBC: red blood cell / Hemoglobin: hemoglobin).

### DETAILED DESCRIPTION OF EMBODIMENTS

Hereinafter, the present invention will be described in detail.

One aspect of the present invention provides a pharmaceutical composition for preventing or treating cancer, the pharmaceutical composition containing, as active ingredients: a naphthoquinone-based compound; and at least one selected from among an immune checkpoint inhibitor and an immunogenic cell death (ICD) inducer.

One embodiment of the pharmaceutical composition may contain a naphthoquinone-based compound and an immune checkpoint inhibitor. In addition, one embodiment of the pharmaceutical composition may contain a naphthoquinone-based compound and an immunogenic cell death inducer. Furthermore, one embodiment of the pharmaceutical composition may contain a naphthoquinone-based compound, an immune checkpoint inhibitor, and an immunogenic cell death inducer.

The "naphthoquinone-based compound" contained as an active ingredient of the composition provided in the present invention is known as an ingredient of a medicine for treating various diseases including cancer, hearing loss, diabetes, and the like. For example, U.S. Patent No. 5,969,163 discloses an anticancer composition containing a naphthoquinone-based compound, and Korean Patent No. 1739361 discloses a composition which is for preventing or ameliorating metabolic diseases and contains a naphthoquinone-based compound. However, in a case where the naphthoquinone-based compound is administered alone as an anticancer agent, the naphthoquinone-based compound does not serve as a targeted therapeutic agent, and thus has a problem that drug resistance or side effects caused by cytotoxicity are difficult to avoid in order to advance the treatment up to a dosage causing a significant anticancer effect.

Accordingly, the present inventors have made efforts to discover anticancer medicines that may show synergistic anticancer effects even with low dosages, and as a result, have confirmed that when two or three drugs are co-administered by mixing the naphthoquinone-based compound with the immune checkpoint inhibitor and/or an immunogenic cell death inducer, the effect inhibiting the proliferation of the cancer cells significantly increased compared to the case where each substance is administered alone, thereby completing the present invention.

The composition may be a composition containing a naphthoquinone-based compound and an immune checkpoint inhibitor. For example, the composition may contain, as active ingredients, 1 to 5, 2 to 4, or 3 kinds of immune checkpoint inhibitors and a naphthoquinone-based compound. In addition, the composition may be a composition containing a naphthoquinone-based compound and an immunogenic cell death inducer. For example, the composition may contain, as active ingredients, 1 to 5, 2 to 4, or 3 kinds of immunogenic cell death inducers and a naphthoquinone-based compound. Furthermore, the composition may be a composition containing a naphthoquinone-based compound, an immune checkpoint inhibitor, and an immunogenic cell death inducer. For example, the composition may contain, as active ingredients, 1 to 5, 2 to 4, or 3 kinds of each of the immune checkpoint inhibitors and the immunogenic cell death inducers together with the naphthoquinone-based compound.

The composition may be in the form of a complex dosage form in which the naphthoquinone-based compound is mixed with at least one selected from among the immune checkpoint inhibitor and the immunogenic cell death inducer. For example, the composition may be in a single dosage form prepared by mixing the naphthoquinone-based compound and the immune checkpoint inhibitor with a pharmaceutically acceptable adjuvant, diluent, or carrier. In addition, the composition may be in a single dosage form prepared by mixing the naphthoquinone-based compound and the immunogenic cell death inducer with a pharmaceutically acceptable adjuvant, diluent, or carrier. Furthermore, the composition may be in a single dosage form prepared by mixing the naphthoquinone-based compound, the immune checkpoint inhibitor, and the immunogenic cell death inducer with a pharmaceutically acceptable adjuvant, diluent, or carrier. Here, the naphthoquinone-based compound, the immune checkpoint inhibitor, and the immunogenic cell death inducer may each include various components within the scope of the corresponding medicine. In addition, the single dosage form may be administered together with a separate formulation, containing another therapeutic agent, as a dosage form separated from such a dosage form.

In addition, the composition may be in the form in which the naphthoquinone-based compound and at least one selected from among the immune checkpoint inhibitor and the immunogenic cell death inducer are each formulated, and simultaneously or sequentially administered. For example, the naphthoquinone-based compound and the immune checkpoint inhibitor are provided as separate pharmaceutical dosage forms, and the respective formulations may be administered at the same time or at different times. In addition, the naphthoquinone-based compound and the immunogenic cell death inducer are provided as separate pharmaceutical dosage forms, and the respective formulations may be administered at the same time or at different times. Furthermore, the naphthoquinone-based compound, the immune checkpoint inhibitor, and the immunogenic cell death inducer are all provided as separate pharmaceutical dosage forms, and the respective formulations may be administered at the same time or at different times. Here, the various components of the naphthoquinone-based compound, the immune checkpoint inhibitor, and the immunogenic cell death inducer within the scope of the corresponding medicine may be respectively formulated, and simultaneously or sequentially administered, and may be administered once or multiple times.

For example, the naphthoquinone-based compound may be in a dosage form for oral administration, and may be administered once a week to 7 times a week, 2 times a week to 5 times a week, or 3 times a week. In addition, the immune checkpoint inhibitor may be in a dosage form for intraperitoneal administration, and may be administered daily, at intervals of 2, 3, 4, 5, or 6 days, or once a week to 3 times a week. Furthermore, the immunogenic cell death inducer may be in a dosage form for oral administration or intraperitoneal administration, and may be administered daily, at intervals of 2, 3, 4, 5, or 6 days, or once a week to 3 times a week. The naphthoquinone-based compound, the immune checkpoint inhibitor, and the immunogenic cell death inducer may be independently administered according to the schedules for the respective substances.

In addition, the naphthoquinone-based compound and the immune checkpoint inhibitor may be co-administered in a weight ratio of 1,000:1 to 1:10, 500:1 to 1:1, 250:1 to 5:1, 200:1 to 2.5:1, or 100:1. In addition, the naphthoquinone-based compound and the immunogenic cell death inducer may be co-administered in a weight ratio of 5:1 to 1:5, 3:1 to 1:3, 2:1 to 1:2, 3:2 to 2:3, or 1:0.001 to 0.001:1. For example, based on a single dosage of the composition, the naphthoquinone-based compound, the immune checkpoint inhibitor, and the immunogenic cell death inducer may be co-administered in a weight ratio of 1 : 0.001 to 500 : 0.1 to 1,000, in a weight ratio of 1 : 0.001 to 300 : 0.1 to 500, or in a weight ratio of 1 : 0.001 to 0.1 : 0.2 to 5.

### Naphthoquinone-based compound

The naphthoquinone-based compound used as an active ingredient in the composition according to the present invention may be at least one selected from among compounds represented by Chemical Formulae 1 to 5, pharmaceutically acceptable salts thereof, prodrugs thereof, solvates thereof, or isomers thereof.

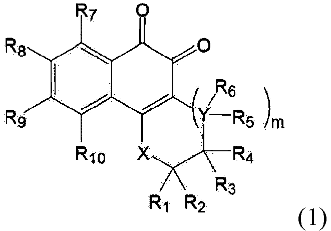

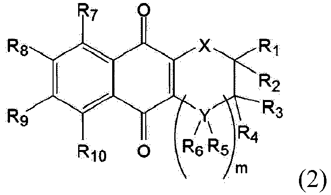

In Chemical Formulae 1 and 2, R₁ to R₆ are each independently selected from the group consisting of hydrogen, hydroxy, halogen, amino, substituted or unsubstituted C₁-C₁₀ alkylamino, substituted or unsubstituted C₁-C₁₀ dialkylamino, substituted or unsubstituted C₁-C₁₀ alkyl, substituted or unsubstituted C₂-C₁₀ alkenyl, substituted or unsubstituted C₂-C₁₀ alkynyl, substituted or unsubstituted C₁-C₁₀ alkoxy, substituted or unsubstituted C₁-C₁₀ alkoxycarbonyl, substituted or unsubstituted C₁-C₁₀ acyl, substituted or unsubstituted C₃-C₈ cycloalkyl, substituted or unsubstituted 3- to 10-membered heterocycloalkyl, substituted or unsubstituted C₆-C₁₀ aryl, substituted or unsubstituted 5- to 10-membered heteroaryl, and substituted or unsubstituted -(CH₂)ₙ-aryl, or may be each substituted or unsubstituted double bond or C₃-C₆ ring structure, which is formed by mutually linking two substituents among them, and the ring structure may be a saturated structure or a partially or fully unsaturated structure, wherein the substituent may be one or more selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, 3- to 10-membered heterocycloalkyl, C₆-C₁₀ aryl, and 5- to 10-membered heteroaryl; R₇ to R₁₀ are each independently selected from the group consisting of hydrogen, hydroxy, halogen, amino, substituted or unsubstituted C₁-C₁₀ alkylamino, substituted or unsubstituted C₁-C₁₀ dialkylamino, substituted or unsubstituted C₁-C₁₀ alkyl, substituted or unsubstituted C₂-C₁₀ alkenyl, substituted or unsubstituted C₂-C₁₀ alkynyl, substituted or unsubstituted C₁-C₁₀ alkoxy, substituted or unsubstituted C₁-C₁₀ alkoxycarbonyl, substituted or unsubstituted C₁-C₁₀ acyl, substituted or unsubstituted C₃-C₈ cycloalkyl, substituted or unsubstituted 3- to 10-membered heterocycloalkyl, substituted or unsubstituted C₆-C₁₀ aryl, substituted or unsubstituted 5- to 10-membered heteroaryl, and substituted or unsubstituted - (CH₂)ₙ-aryl, or may be each substituted or unsubstituted C₃-C₆ ring structure formed by mutually linking two substituents among them, and the ring structure may be a saturated structure or a partially or fully unsaturated structure, wherein the substituent may be one or more selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, 3- to 10-membered heterocycloalkyl, C₆-C₁₀ aryl, and 5- to 10-membered heteroaryl; X is O, S, or NR', where R' is hydrogen or C₁-C₆ alkyl; Y is C, S, N, or O, where when Y is S or O, R₅ and R₆ are not any substituents, and when Y is N, R₅ is hydrogen or C₁-C₆ alkyl and R₆ is not any substituent; m is 0 or 1, and when m is 0, adjacent carbon atoms of m are directly boned to each other to form a cyclic structure; and n is an integer of 0 to 10.

Preferably, in Chemical Formulae 1 and 2, X may be O or S, and Y may be C or O.

As a preferred example, in Chemical Formulae 1 and 2, R₁ to R₆ may be each independently selected from the group consisting of hydrogen, hydroxy, halogen, substituted or unsubstituted C₁-C₁₀ alkyl, substituted or unsubstituted C₂-C₁₀ alkenyl, substituted or unsubstituted C₁-C₁₀ alkoxy, and -(CH₂)ₙ-phenyl, or may be a double bond or a C₄-C₆ ring structure, which is formed by mutually linking R₁ and R₄ or R₂ and R₃, wherein the substituent may be C₁-C₁₀ alkyl.

As another preferred example, in Chemical Formulae 1 and 2, R₇ to R₁₀ may be each independently selected from the group consisting of hydrogen, hydroxy, halogen, substituted or unsubstituted C₁-C₁₀ alkyl, and substituted or unsubstituted C₁-C₁₀ alkoxy.

Preferred examples among the compounds represented by Chemical Formula 1 or 2 include a compound represented by Chemical Formula 1-1 or 2-1 where X is O and Y is C, or a compound represented by Chemical Formula 1-2 or 2-2 where X is S.

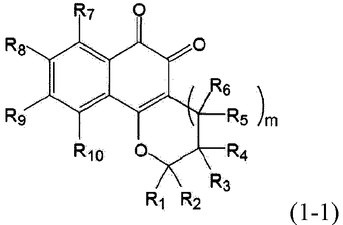

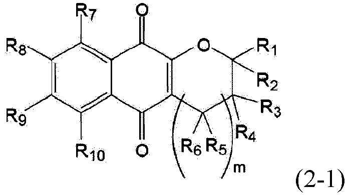

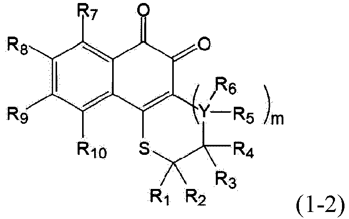

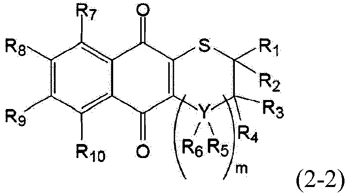

In the Chemical Formulae, R₁ to R₁₀, Y, and m are as defined in Chemical Formulae 1 and 2.

As yet another preferred example, the compound represented by Chemical Formula 1 or 2 may be a compound represented by Chemical Formula 1-3 or 2-3 where m is 0 and adjacent carbon atoms are directly boned to each other to form a cyclic structure (furan ring). Hereinafter, the compound is sometimes referred to as a `furan compound' or a `furano-o-naphthoquinone derivative'.

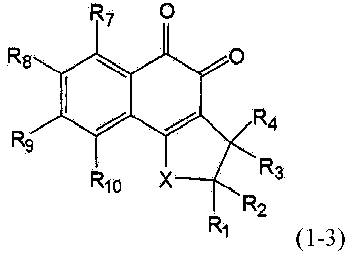

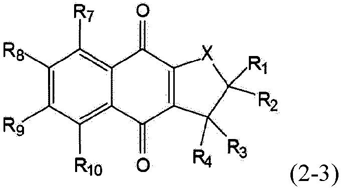

In the Chemical Formulae, R₁ to R₄, R₇ to R₁₀, and X are as defined in Chemical Formula 1.

In addition, the compound represented by Chemical Formula 1 or 2 may be a compound represented by Chemical Formula 1-4 or 2-4 where m is 1. Hereinafter, the compound is sometimes referred to as a `pyran compound' or a `pyrano-o-naphthoquinone derivative'.

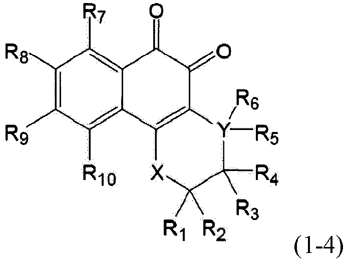

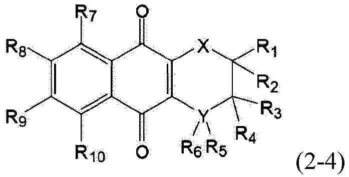

In the Chemical Formulae, R₁ to R₁₀, X, and Y are as defined in Chemical Formula 1.

As still another preferred example, the compound represented by Chemical Formula 1 or 2 may be a compound represented by Chemical Formula 1-5 or 1-6 or a compound represented by Chemical Formula 2-5 or 2-6, wherein R₇ and Rs are bonded to each other to form a ring structure.

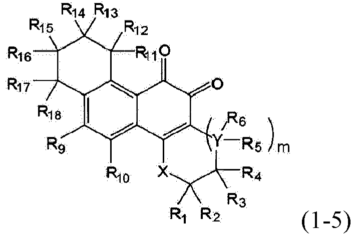

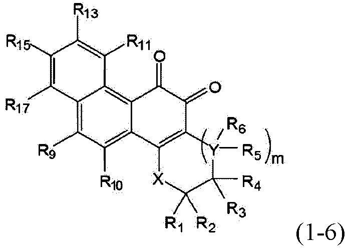

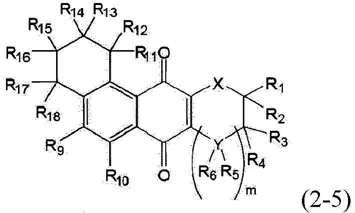

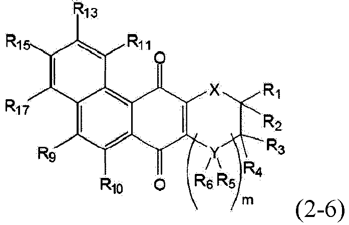

R₁ to R₁₀, X, Y, and m in the formulae are as defined in Chemical Formula 1, and R₁₁ to R₁₈ are each hydrogen, hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, 3- to 10-membered heterocycloalkyl, C₆-C₁₀ aryl, or 5- to 10-membered heteroaryl.

Preferably, R₁₁ to R₁₈ may be each independently hydrogen, hydroxy, halogen, C₁-C₁₀ alkyl, C₂-C₁₀ alkenyl, C₂-C₁₀ alkynyl, C₁-C₁₀ alkoxy, C₃-C₈ cycloalkyl, or phenyl.

Particularly preferred examples among the naphthoquinone-based compounds represented by Chemical Formula 1 or 2 include compounds in Table 1 below, but are not limited thereto.

**[Table 1]**

| **No.** | **Compound** | **No.** | **Compound** | **No.** | **Compound** |
|---|---|---|---|---|---|
| 1 | 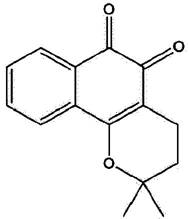 | 2 | 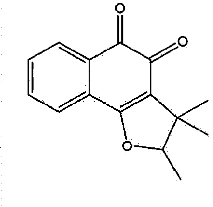 | 3 | 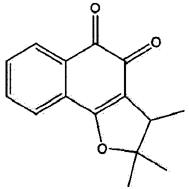 |
| 4 | 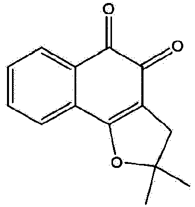 | 5 | 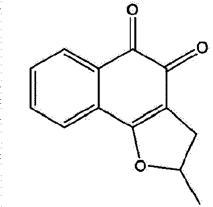 | 6 | 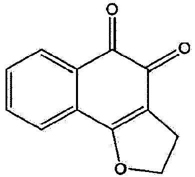 |
| 7 | 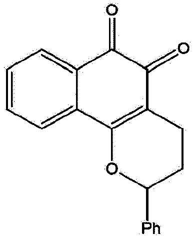 | 8 | 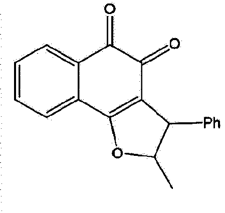 | 9 | 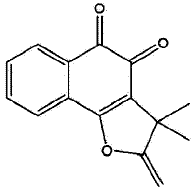 |
| 10 | 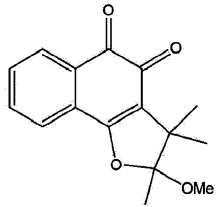 | 11 | 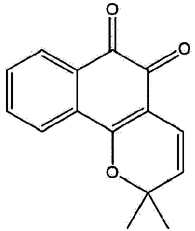 | 12 | 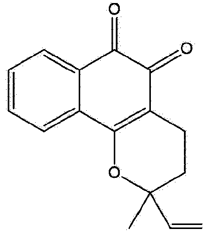 |
| 13 | 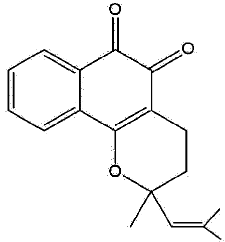 | 14 | 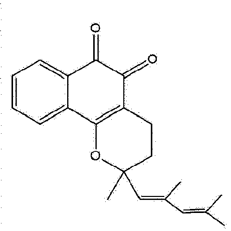 | 15 | 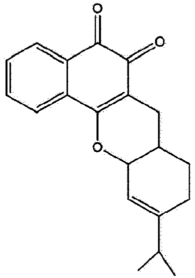 |
| 16 | 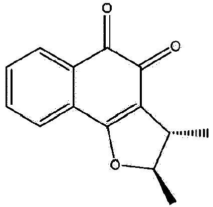 | 17 | 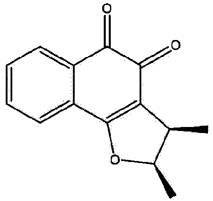 | 18 | 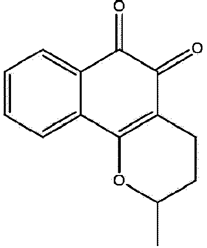 |
| 19 | 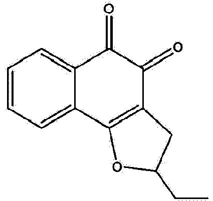 | 20 | 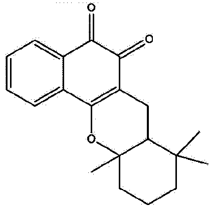 | 21 | 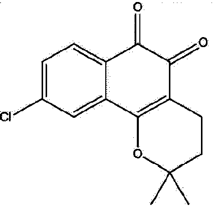 |
| 22 | 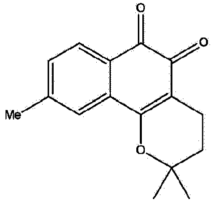 | 23 | 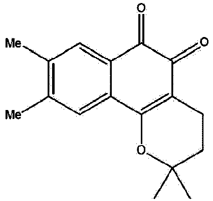 | 24 | 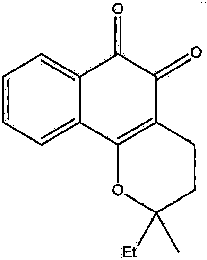 |
| 25 | 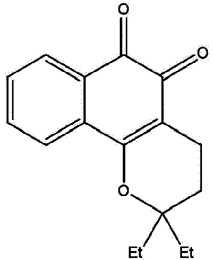 | 26 | 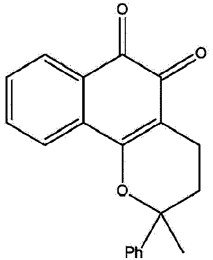 | 27 | 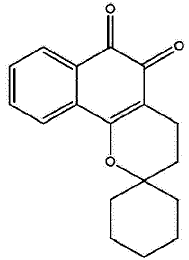 |
| 28 | 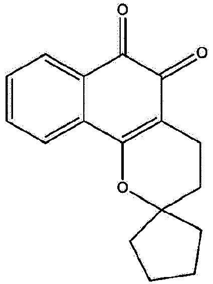 | 29 | 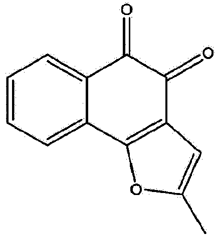 | 30 | 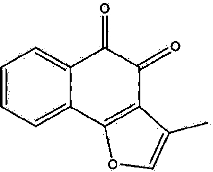 |
| 31 | 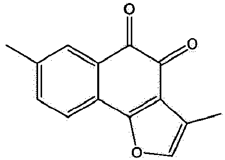 | 32 | 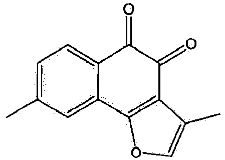 | 33 | 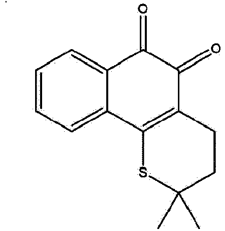 |
| 34 | 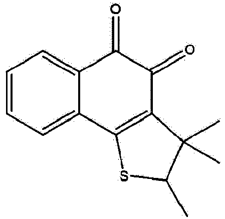 | 35 | 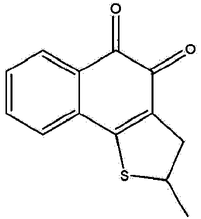 | 36 | 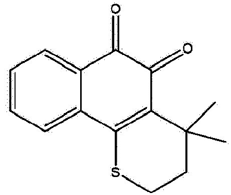 |
| 37 | 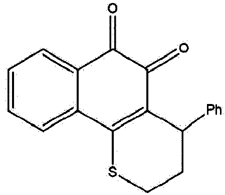 | 38 | 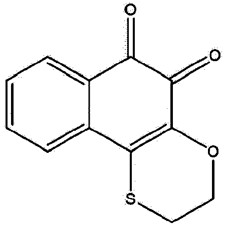 | 39 | 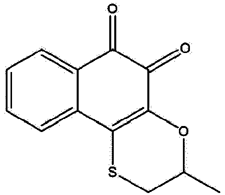 |
| 40 | 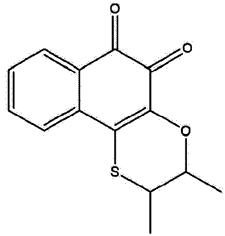 | 41 | 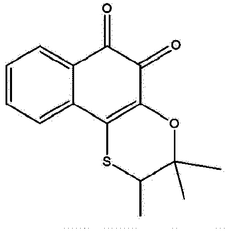 | 42 | 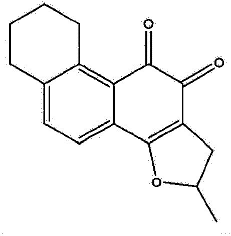 |
| 43 | 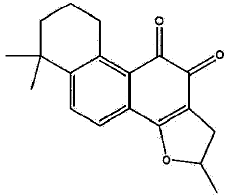 | 44 | 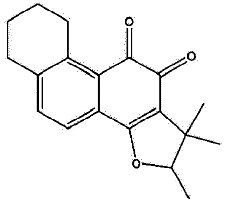 | 45 | 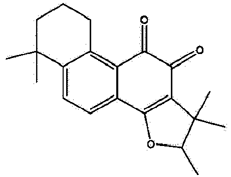 |
| 46 | 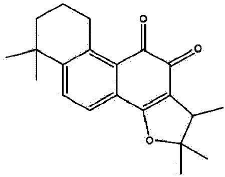 | 47 | 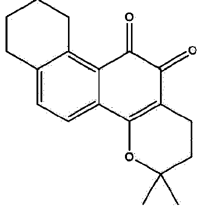 | 48 | 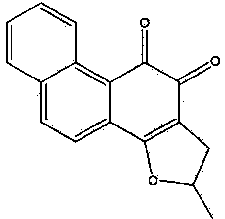 |
| 49 | 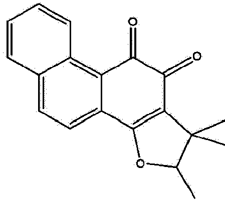 | 50 | 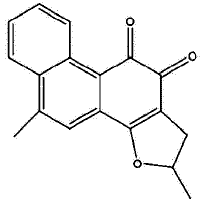 | 51 | 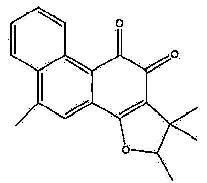 |
| 52 | 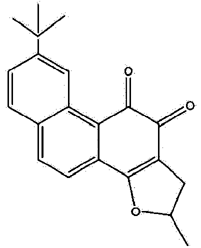 | 53 | 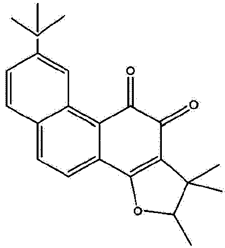 | 54 | 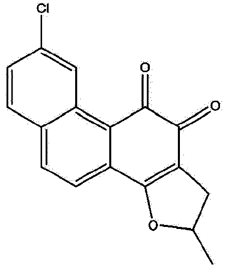 |
| 55 | 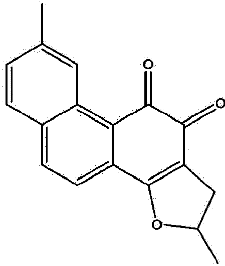 | 56 | 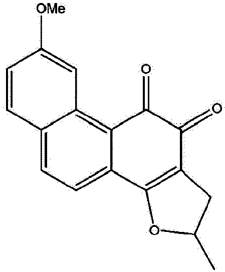 | 57 | 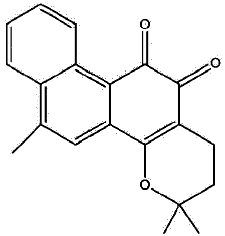 |
| 58 | 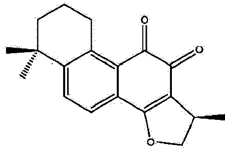 | 59 | 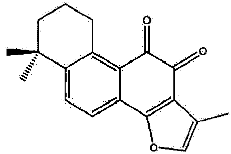 | 60 | 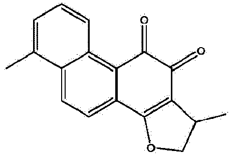 |
| 61 | 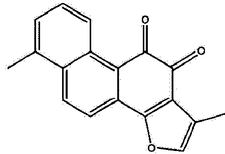 | | | | |

The naphthoquinone-based compound represented by Chemical Formula 1 or 2 used in the composition according to the present invention may be prepared by the methods disclosed in International Publication Nos. WO 2006/088315 and WO 2006/020719, and may be prepared by other known methods and/or various methods based on techniques in the field of organic synthesis. Various derivatives may be synthesized using an appropriate synthesis method according to the kind of a substituent based on the aforementioned methods.

In addition, the naphthoquinone-based compound may include, as a component, a compound represented by Chemical Formula 3, a pharmaceutically acceptable salt thereof, a prodrug thereof, an isomer thereof, or a solvate thereof.

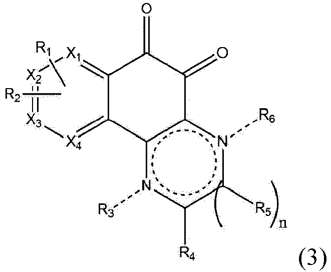

In Chemical Formula 3, R₁ and R₂ are each independently hydrogen, halogen, substituted or unsubstituted C₁-C₂₀ alkoxy, substituted or unsubstituted C₁-C₆ alkyl, substituted or unsubstituted C₆-C₁₀ aryl, substituted or unsubstituted C₆-C₁₀ aryloxy, substituted or unsubstituted 5- to 10-membered heteroaryl, -NO₂, -NR'₁R'₂, -NR'₁(CO(O)R'₂), -NR'₁(C(O)NR'₁R'₂), -C(O)NR'₁R'₂, -CN, -SO(O)R'₁, - SO(O)NR'₁R'₂, -NR'₁(SO(O)R'₂), or -CSNR'₁R'₂, or R₁ and R₂ may be bonded to each other to form a cyclic structure of substituted or unsubstituted C₆-C₁₀ aryl or a cyclic structure of substituted or unsubstituted 5- to 10-membered heteroaryl; wherein R'₁ and R'₂ are each independently hydrogen, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ aryloxy, substituted or unsubstituted 5- to 10-membered heteroaryl, substituted or unsubstituted - (CR"₁R"₂)_{m'}-C₆-C₁₀ aryl, or substituted or unsubstituted NR"₁R"₂; wherein R"₁ and R"₂ are each independently hydrogen or C₁-C₃ alkyl, or R"₁ and R"₂ may be bonded to each other to form a cyclic structure of substituted or unsubstituted C₆-C₁₀ aryl; R₃, R₄, R₅, and R₆ are each independently hydrogen, halogen, substituted or unsubstituted C₁-C₉ alkyl, substituted or unsubstituted C₂-C₂₀ alkene, substituted or unsubstituted C₁-C₂₀ alkoxy, substituted or unsubstituted C₃-C₈ cycloalkyl, substituted or unsubstituted 3- to 10-membered heterocycloalkyl, substituted or unsubstituted C₆-C₁₀ aryl, substituted or unsubstituted C₆-C₁₀ aryloxy, substituted or unsubstituted 5- to 10-membered heteroaryl, substituted or unsubstituted - (CR'₅R'₆)ₘ-C₆-C₁₀ aryl, substituted or unsubstituted -(CR'₅R'₆)ₘ-C₆-C₁₀ aryloxy, substituted or unsubstituted -(CR'₅R'₆)ₘ-5- to 10-membered heteroaryl, substituted or unsubstituted -(CR'₅R'₆)ₘ-3- to 10-membered heterocycloalkyl, substituted or unsubstituted -(CR'₅R'₆)ₘ-NR'₃R'₄, substituted or unsubstituted -(CR'₅R'₆)ₘ-OR'₃, - CO(O)R'₃, -CONR'₃R'₄, -NR'₃R'₄, -NR'₃(C(O)R'₄), -SO(O)R'₃, -SO(O)NR'₃R'₄, - NR'₃(SO(O)R'₄), -CSNR'₃R'₄, -CH₂A when the compound represented by Chemical Formula 3 is "A", or -A when the compound represented by Chemical Formula 3 is "A"; wherein R'₃ and R'₄ are each independently hydrogen, substituted or unsubstituted C₁-C₆ alkyl, substituted or unsubstituted C₃-C₈ cycloalkyl, substituted or unsubstituted C₆-C₁₀ aryl, substituted or unsubstituted -(CH₂)ₘ-C₆-C₁₀ aryl, substituted or unsubstituted -(CH₂)ₘ-C₆-C₁₀ aryloxy, or -CO(O)R"₃, or R'₃ and R'₄ may be bonded to each other to form a cyclic structure of substituted or unsubstituted 3- to 10-membered heterocycloalkyl, or a cyclic structure of substituted or unsubstituted 5- to 10-membered heteroaryl; R'₅ and R'₆ are each independently hydrogen or C₁-C₃ alkyl; and R"₃ is C₁-C₆ alkyl; wherein the substituent is one or more selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₃-C₈ cycloalkyl, 3-to 10-membered heterocycloalkyl, C₆-C₁₀ aryl, and 5- to 10-membered heteroaryl; with a proviso that structures in which R₃ and R₄ are each independently C₆-C₁₀ aryl are excluded, structures in which R₄ and R₆ are each independently C₆-C₁₀ aryl are excluded, structures in which R₄ is hydrogen, methyl, halogen-substituted methyl, or piperidinylmethyl when R₃ has the structure defined above are excluded, and structures in which R₅ is phenyl are excluded; m and m' are each independently a natural number of 1 to 4; the heteroatom is one or more selected from among N, O, and S; X₁, X₂, X₃, and X₄ are each independently CH or N; and n is 0 or 1, and when n is 0, adjacent carbon atoms of n are directly boned to each other to form a cyclic structure.

In a preferred example, the compound represented by Chemical Formula 3 may be a compound represented by Chemical Formula 3-1.

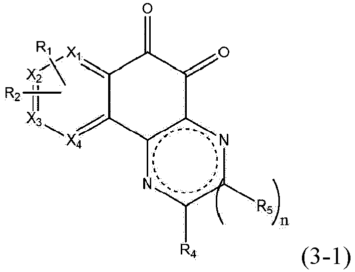

In the Chemical Formula 3-1, R₁, R₂, R₄, R₅, X₁, X₂, X₃, and X₄ are as defined in Chemical Formula 3.

Examples of the compound represented by Chemical Formula 3-1 include a compound represented by Chemical Formula 3-1-1, but the following compound does not limit the present invention.

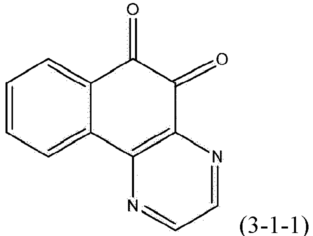

In another preferred example, the compound represented by Chemical Formula 3 may be a compound represented by Chemical Formula 3-2 or a compound represented by Chemical Formula 3-3.

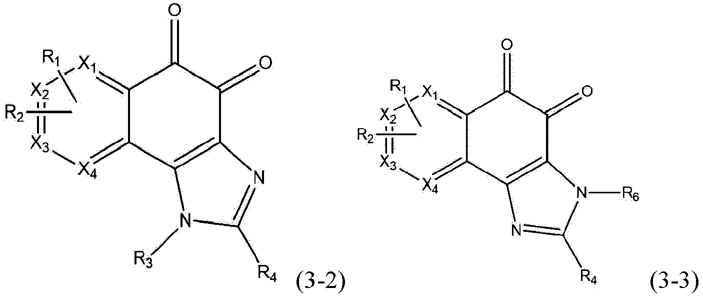

In the Chemical Formulae 3-2 and 3-3, R₁ to R₄, R₆, X₁, X₂, X₃, and X₄ are as defined in Chemical Formula 3.

Specifically, in Chemical Formulae 3-2 and 3-3, R₁ and R₂ may be each independently hydrogen, halogen, substituted or unsubstituted C₁-C₂₀ alkoxy, substituted or unsubstituted C₁-C₆ alkyl, substituted or unsubstituted C₆-C₁₀ aryl, substituted or unsubstituted 5- to 10-membered heteroaryl, -NO₂, -NR'₁R'₂, - NR'₁(C(O)R'₂), -NR'₁(SO₂R'₂), -NR'₁(CO₂R'₂), -NR'₁(C(O)NR'₁R'₂), -COOR'₁, - C(O)NR'₁R'₂, or -CN, or R₁ and R₂ may be bonded to each other to form a cyclic structure of substituted or unsubstituted C₆-C₁₀ aryl or a cyclic structure of substituted or unsubstituted 5- to 10-membered heteroaryl, wherein R'₁ and R'₂ are each independently hydrogen, substituted or unsubstituted C₁-C₆ alkyl, substituted or unsubstituted C₃-C₈ cycloalkyl, substituted or unsubstituted C₆-C₁₀ aryl, or substituted or unsubstituted -(CH₂)ₘ-C₆-C₁₀ aryl; wherein, the substituent may be one or more selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₃-C₈ cycloalkyl, 3-to 10-membered heterocycloalkyl, C₆-C₁₀ aryl, and 5- to 10-membered heteroaryl.

More specifically, in Chemical Formulae 3-2 and 3-3, R₁ and R₂ may be each independently H, F, Cl, -NO₂, NH₂, -N(CH₃)₂, -NHCOCH₃, -NHCOC₃H₅, or -NHCH₂C₆H₅F, and X₂ and X₃ may be each CH.

Further more specifically, in Chemical Formulae 3-2 and 3-3, R₁ and R₂ are each independently H, F, Cl, -NO₂, NH₂, -N(CH₃)₂, -NHCOCH₃, -NHCOC₃H₅, or -NHCH₂C₆H₅F; X₂ and X₃ are each CH; R₃ to R₆ are each independently H, halogen, substituted or unsubstituted C₁-C₉ alkyl, substituted or unsubstituted C₃-C₈ cycloalkyl, substituted or unsubstituted -(CR'₅R'₆)ₘ-C₆-C₁₀ aryl, substituted or unsubstituted -(CR'₅R'₆)ₘ-C₆-C₁₀ aryloxy, substituted or unsubstituted -(CR'₅R'₆)ₘ-5- to 10-membered heteroaryl, substituted or unsubstituted -(CR'₅R'₆)ₘ-3- to 10-membered heterocycloalkyl, substituted or unsubstituted -(CHR'₅)ₘ-NR'₃R'₄, - CO(O)R'₃, -CONR'₃R'₄, -NR'₃R'₄, -NR'₃(C(O)R'₄), or -CH₂A when the compound represented by Chemical Formula 3 is "A"; R₄ is halogen, substituted or unsubstituted C₂-C₉ alkyl, substituted or unsubstituted C₁-C₁₀ alkoxy, substituted or unsubstituted C₃-C₈ cycloalkyl, substituted or unsubstituted 3- to 10-membered heterocycloalkyl, substituted or unsubstituted C₆-C₁₀ aryl, substituted or unsubstituted C₆-C₁₀ aryloxy, substituted or unsubstituted 5- to 10-membered heteroaryl, substituted or unsubstituted -(CR'₅R'₆)ₘ-C₆-C₁₀ aryl, substituted or unsubstituted -(CR'₅R'₆)ₘ-C₆-C₁₀ aryloxy, substituted or unsubstituted -(CR'₅R'₆)ₘ-5- to 10-membered heteroaryl, substituted or unsubstituted -(CHR'₅)ₘ-NR'₃-C₆-C₁₀ aryl, substituted or unsubstituted -(CR'₅R'₆)ₘ-3- to 10-membered heterocycloalkyl, substituted or unsubstituted -(CR'₅R'₆)ₘ-NR'₃R'₄, substituted or unsubstituted - (CR'₅R'₆)ₘ-OR'₃, -NR'₃R'₄, or -A when the compound represented by Chemical Formula 3 is "A"; wherein R'₃ and R'₄ are each independently hydrogen, substituted or unsubstituted C₁-C₆ alkyl, substituted or unsubstituted C₃-C₈ cycloalkyl, substituted or unsubstituted -(CH₂)ₘ-C₆-C₁₀ aryl, substituted or unsubstituted - (CH₂)ₘ-C₆-C₁₀ aryloxy, or -CO(O)R"₃, or R'₃ and R'₄ may be bonded to each other to form a cyclic structure of substituted or unsubstituted 3- to 10-membered heterocycloalkyl or a cyclic structure of substituted or unsubstituted 5- to 10-membered heteroaryl; R'₅ and R'₆ are each independently hydrogen or C₁-C₃ alkyl; R"₃ is C₁-C₆ alkyl; wherein the substituent is one or more selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₃-C₈ cycloalkyl, 3- to 10-membered heterocycloalkyl, C₆-C₁₀ aryl, and 5- to 10-membered heteroaryl; m is a natural number of 1 to 4; and the heteroatom may be one or more selected from among N, O, and S.

Still further more specifically, in Chemical Formulae 3-2 and 3-3, R₁ and R₂ are each independently H, F, Cl, -NO₂, NH₂, -N(CH₃)₂, -NHCOCH₃, - NHCOC₃H₅, or -NHCH₂C₆H₅F; X₂ and X₃ are each CH; R₃ and R₆ are each independently H, halogen, substituted or unsubstituted C₁-C₉ alkyl, substituted or unsubstituted -(CH₂)ₘ-C₆-C₁₀ aryl, substituted or unsubstituted -(CH₂)ₘ-C₆-C₁₀ aryloxy, substituted or unsubstituted -(CHR'₅)ₘ-5- to 10-membered heteroaryl, substituted or unsubstituted -(CHR'₅)ₘ-3- to 10-membered heterocycloalkyl, substituted or unsubstituted -(CHR'₅)ₘ-NR'₃R'₄, -CO(O)R'₃, -CONR'₃R'₄, -NR'₃R'₄, -NR'₃(C(O)R'₄), or -CH₂A when the compound represented by Chemical Formula 3 is "A"; R₄ is halogen, substituted or unsubstituted C₂-C₉ alkyl, substituted or unsubstituted C₁-C₁₀ alkoxy, substituted or unsubstituted C₃-C₈ cycloalkyl, substituted or unsubstituted 3- to 10-membered heterocycloalkyl, substituted or unsubstituted C₆-C₁₀ aryl, substituted or unsubstituted C₆-C₁₀ aryloxy, substituted or unsubstituted 5- to 10-membered heteroaryl, substituted or unsubstituted -(CH₂)ₘ-C₆-C₁₀ aryl, substituted or unsubstituted -(CH₂)ₘ-C₆-C₁₀ aryloxy, substituted or unsubstituted -(CHR'₅)ₘ-5- to 10-membered heteroaryl, substituted or unsubstituted - (CHR'₅)ₘ-NR'₃-C₆-C₁₀ aryl, substituted or unsubstituted -(CHR'₅)ₘ-3- to 10-membered heterocycloalkyl, substituted or unsubstituted -(CHR'₅)ₘ-NR'₃R'₄, - NR'₃R'₄, or -A when the compound represented by Chemical Formula 3 is "A"; wherein R'₃ and R'₄ are each independently hydrogen, substituted or unsubstituted C₁-C₆ alkyl, substituted or unsubstituted C₃-C₈ cycloalkyl, substituted or unsubstituted -(CH₂)ₘ-C₆-C₁₀ aryl, substituted or unsubstituted -(CH₂)ₘ-C₆-C₁₀ aryloxy, or -COOC(CH₃)₃, or R'₃ and R'₄ may be bonded to each other to form a cyclic structure of substituted or unsubstituted 3- to 10-membered heterocycloalkyl or a cyclic structure of substituted or unsubstituted 5- to 10-membered heteroaryl; R'₅ is hydrogen or C₁-C₃ alkyl; wherein the substituent is one or more selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₃-C₈ cycloalkyl, 3- to 10-membered heterocycloalkyl, C₆-C₁₀ aryl, and 5- to 10-membered heteroaryl; m is a natural number of 1 to 4; and the heteroatom may be one or more selected from among N, O, and S.

In addition, more specifically, in Chemical Formulae 3-2 and 3-3, R₃ and R₆ are each independently H, substituted or unsubstituted C₁-C₃ alkyl, substituted or unsubstituted -(CH₂)ₘ-C₅-C₆ aryl, substituted or unsubstituted - (CH₂)ₘ-C₅-C₆ aryloxy, substituted or unsubstituted -(CHR'₅)ₘ-C₄-C₆ heteroaryl, substituted or unsubstituted -(CHR'₅)ₘ-C₄-C₆ heterocycloalkyl, substituted or unsubstituted -(CHR'₅)ₘ-NR'₃R'₄, -CO(O)R'₃, or -CH₂A when the compound represented by Chemical Formula 3 is "A"; wherein R'₃ and R'₄ are each independently hydrogen, C₁-C₅ alkyl, or C₃-C₅ cycloalkyl, or R'₃ and R'₄ may be bonded to each other to form a cyclic structure of substituted or unsubstituted 3- to 10-membered heterocycloalkyl; R'₅ is H; the substituent is methyl, halogen, or hydroxy; and m may be 1 to 3. Further more specifically, the halogen may be fluorine or chlorine and the aryl may be C₆ aryl.

Furthermore, more specifically, in Chemical Formulae 3-2 and 3-3, R₄ is halogen, substituted or unsubstituted C₂-C₅ alkyl, substituted or unsubstituted C₁-C₃ alkoxy, substituted or unsubstituted C₃-C₆ cycloalkyl, substituted or unsubstituted C₄-C₆ aryl, substituted or unsubstituted -(CH₂)ₘ-C₅-C₆ aryl, substituted or unsubstituted C₆-C₁₀ aryloxy, substituted or unsubstituted -(CH₂)ₘ-C₅-C₆ aryloxy, substituted or unsubstituted C₅-C₆ heteroaryl, substituted or unsubstituted -(CHR'₅)ₘ-C₅-C₆ heteroaryl, substituted or unsubstituted C₄-C₆ heterocycloalkyl, substituted or unsubstituted -(CHR'₅)ₘ-C₃-C₆ heterocycloalkyl, -NR'₃R'₄, substituted or unsubstituted -(CHR'₅)ₘ-NR'₃-C₅-C₆ aryl, substituted or unsubstituted -(CHR'₅)ₘ-NR'₃R'₄, or -A when the compound represented by Chemical Formula 3 is "A"; R'₃ and R'₄ are each independently hydrogen, methyl, ethyl, or -COOC(CH₃)₃, or R'₃ and R'₄ may be bonded to each other to form a cyclic structure of substituted or unsubstituted C₄-C₆ heterocycloalkyl; R'₅ is H, methyl, ethyl, propyl, or butyl; the substituent is methyl, halogen, or hydroxy; and m may be 1 or 2. Further more specifically, the halogen may be fluorine and the aryl may be C₆ aryl.

In addition, particularly preferred examples among the naphthoquinone-based compounds represented by Chemical Formula 3 include the following compounds, but are not limited thereto.

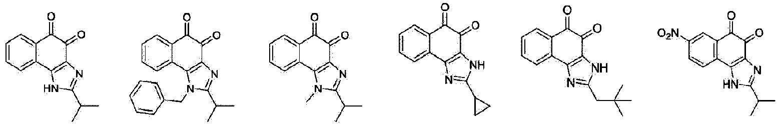

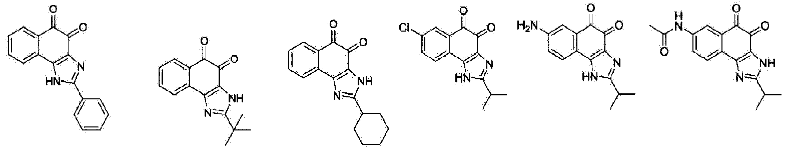

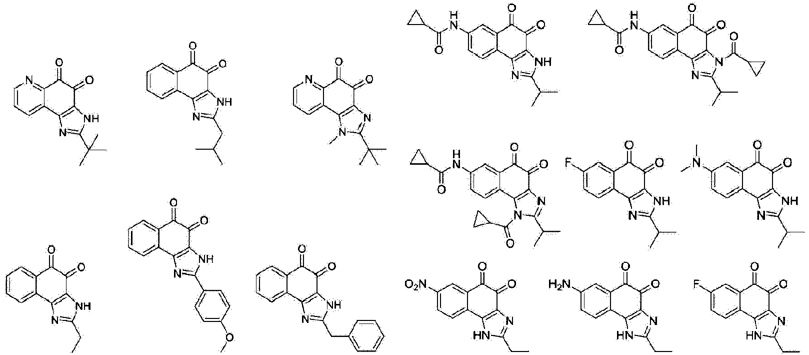

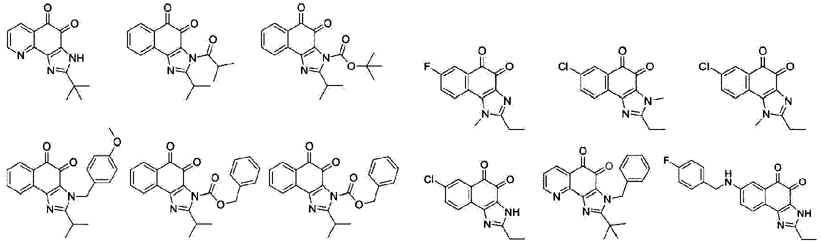

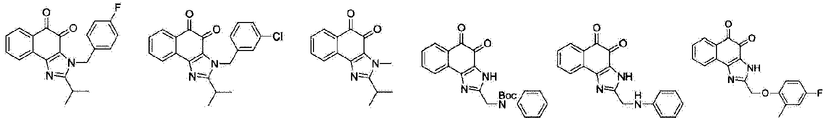

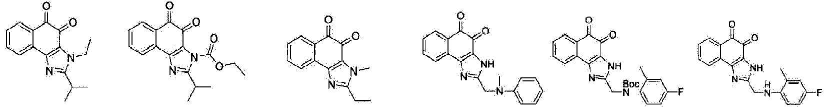

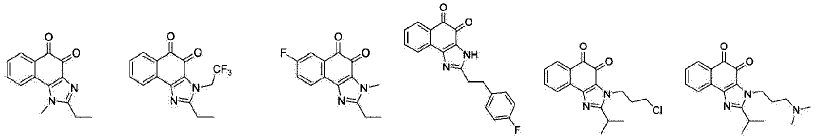

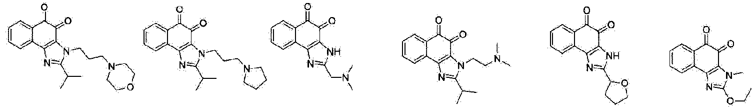

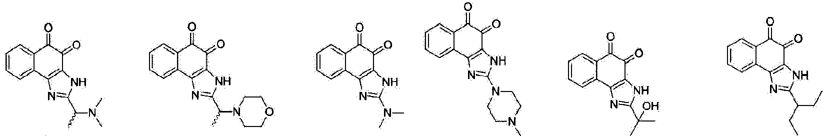

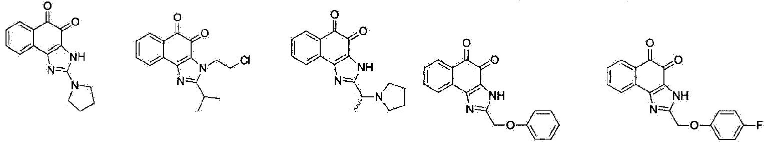

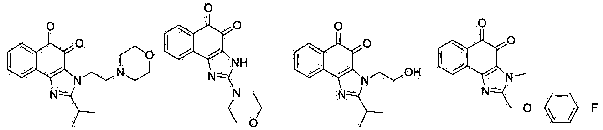

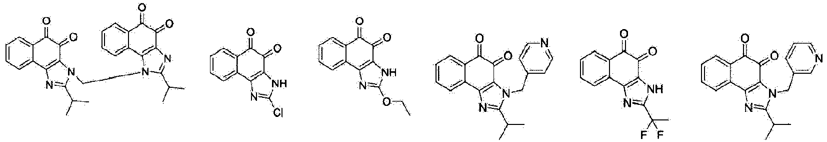

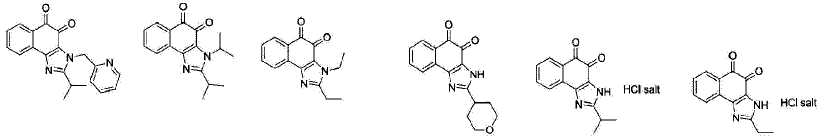

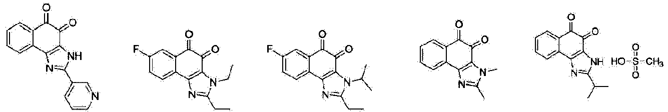

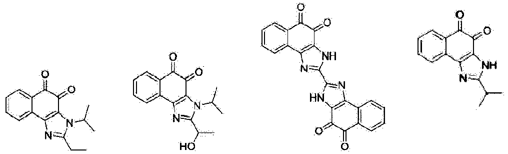

The naphthoquinone-based compound represented by Chemical Formula 3 used in the composition according to the present invention may be prepared by the method disclosed in Korean Patent No. 10-1739361, and may be prepared by other known methods and/or various methods based on techniques in the field of organic synthesis. Various derivatives may be synthesized using an appropriate synthesis method according to the kind of a substituent based on the aforementioned methods.

In addition, the naphthoquinone-based compound may include, as a component, a compound represented by Chemical Formula 4, a pharmaceutically acceptable salt thereof, a prodrug thereof, an isomer thereof, or a solvate thereof.

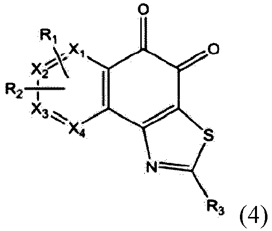

In Chemical Formula 4, R₁ and R₂ are each independently hydrogen, a halogen atom, hydroxy, substituted or unsubstituted C₁-C₂₀ alkoxy, substituted or unsubstituted C₁-C₁₀ alkyl, substituted or unsubstituted C₄-C₁₀ aryl, substituted or unsubstituted C₄-C₁₀ aryloxy, substituted or unsubstituted C₂-C₁₀ heteroaryl, -NO₂, - NR'₁R'₂, -NR'₁(CO(O)R'₂), -NR'₁(C(O)NR'₁R'₂), -CO(O)R'₁, -C(O)NR'₁R'₂, -CN, -SO(O)R'₁, -SO(O)NR'₁R'₂, -NR'₁(SO(O)R'₂), or -CSNR'₁R'₂, or R₁ and R₂ may be bonded to each other to form a cyclic structure of substituted or unsubstituted C₄-C₁₀ aryl or a cyclic structure of substituted or unsubstituted C₂-C₁₀ heteroaryl; wherein R'₁ and R'₂ are each independently hydrogen, substituted or unsubstituted C₁-C₆ alkyl, substituted or unsubstituted C₃-C₈ cycloalkyl, substituted or unsubstituted C₄-C₁₀ aryl, substituted or unsubstituted C₄-C₁₀ aryloxy, substituted or unsubstituted C₁-Cs heteroaryl, substituted or unsubstituted -(CR"₁R"₂)ₘ,-C₄-C₁₀ aryl, substituted or unsubstituted -(CR"₁R"₂)_{m'}-C₄-C₁₀ heteroaryl, or substituted or unsubstituted NR"₁R"₂; wherein R"₁ and R"₂ are each independently hydrogen or C₁-C₃ alkyl, or R" ₁ and R"₂ may be bonded to each other to form a cyclic structure of substituted or unsubstituted C₄-C₁₀ aryl; R₃ is hydrogen, hydroxy, a halogen atom, substituted or unsubstituted C₁-C₁₀ alkyl, substituted or unsubstituted C₂-C₂₀ alkene, substituted or unsubstituted C₁-C₂₀ alkoxy, substituted or unsubstituted C₃-C₈ cycloalkyl, substituted or unsubstituted C₂-C₈ heterocycloalkyl, substituted or unsubstituted C₄-C₁₀ aryl, substituted or unsubstituted C₄-C₁₀ aryloxy, substituted or unsubstituted C₁-C₁₀ heteroaryl, substituted or unsubstituted -(CR'₅R'₆)ₘ-C₄-C₁₀ aryl, substituted or unsubstituted -(CR'₅R'₆)ₘ-C₄-C₁₀ aryloxy, substituted or unsubstituted -(CR'₅R'₆)ₘ-C₁-C₁₀ heteroaryl, substituted or unsubstituted -(CR'₅R'₆)ₘ-NR'₃R'₄, substituted or unsubstituted -(CR'₅R'₆)ₘ-C₂-C₁₀ heterocycloalkyl, substituted or unsubstituted - (CR'₅R'₆)ₘ-OR'₃, substituted or unsubstituted -(CR'₅R'₆)ₘ(O)COR'₃, -CO(O)R'₃, - CONR'₃R'₄, -NR'₃R'₄, -NR'₃(C(O)R'₄), -CH₂A when the compound represented by Chemical Formula 4 is "A", or -A when the compound represented by Chemical Formula 4 is "A"; wherein R'₃ and R'₄ are each independently hydrogen, substituted or unsubstituted C₁-C₆ alkyl, substituted or unsubstituted C₃-C₈ cycloalkyl, substituted or unsubstituted C₄-C₁₀ aryl, substituted or unsubstituted -(CR'₅R'₆)ₘ-C₄-C₁₀ aryl, substituted or unsubstituted -(CR'₅R'₆)ₘ-C₄-C₁₀ aryloxy, substituted or unsubstituted -(CR'₅R'₆)ₘ-C₁-C₁₀ heteroaryl, or -CO(O)R‴₃, or R'₃ and R'₄ may be bonded to each other to form a cyclic structure of substituted or unsubstituted C₄-C₁₀ heterocycloalkyl or a cyclic structure of substituted or unsubstituted C₁-C₁₀ heteroaryl; R'₅ and R'₆ are each independently hydrogen or C₁-C₃ alkyl; R‴₃ is C₁-C₆ alkyl; wherein the substituent may be one or more selected from the group consisting of hydroxy, a nitro group, a halogen atom, C₁-C₁₀ alkyl, C₂-C₁₀ alkenyl, C₂-C₁₀ alkynyl, C₁-C₁₀ alkoxy, C₁-C₁₀ alkoxycarbonyl, C₃-C₈ cycloalkyl, C₃-C₈ heterocycloalkyl, C₄-C₁₀ aryl, and C₅-C₁₀ heteroaryl; X₁, X₂, X₃, and X₄ are each independently CH or N; m and m' are each independently a natural number of 1 to 4; and the heteroatom is one or more selected from among N, O, and S.

In a preferred example, X₁ and X₂ are each independently CH, CO, or N(R₃'); where R₃' is hydrogen or C₁-C₃ alkyl; and X₃ and X₄ may be each CH.

In addition, R₁ and R₂ may be each independently hydrogen, a halogen atom, -OCH₃, -OCH₂CH₃, -O(CH₂)₂CH₃, -CH₃, -NO₂, -CN, -NR'₁R'₂, or-OH (in the formula, R'₁ and R'₂ are each independently hydrogen, C₁-C₃ alkyl, substituted or unsubstituted -CH₂-C₄-C₁₀ aryl, substituted or unsubstituted -C₂H₄-C₄-C₁₀ aryl, or substituted or unsubstituted C₂-C₁₀ heteroaryl, and the substituent is a halogen atom).

Further more specifically, R₁ and R₂ may be each independently hydrogen, Cl, -NO₂, -NH₂, or -NR'₁R'₂ (in the formula, R'₁ and R'₂ are each independently hydrogen or substituted or unsubstituted -CH₂-C₄-C₆ aryl, and the substituent is a halogen atom).

In addition, R₃ is hydrogen, substituted or unsubstituted methyl, ethyl, n-propyl, isopropyl, butyl, isobutyl, t-butyl, pentyl, neopentyl, substituted or unsubstituted C₄-C₈ aryl, substituted or unsubstituted C₄-C₈ aryloxy, substituted or unsubstituted C₁-C₈ heteroaryl, substituted or unsubstituted -(CR'₅R'₆)ₘ-C₄-C₁₀ aryl, substituted or unsubstituted -(CR'₅R'₆)ₘ-C₄-C₁₀ aryloxy, substituted or unsubstituted -(CR'₅R'₆)ₘ-C₁-C₁₀ heteroaryl, substituted or unsubstituted -(CR'₅R'₆)ₘ-C₄-C₁₀ heterocycloalkyl, or substituted or unsubstituted -(CH₂)ₘ-NR'₅R'₆; R'₅ and R'₆ are each independently hydrogen or C₁-C₃ alkyl, wherein the substituent may be one or more selected from the group consisting of a halogen atom, hydroxy, C₁-C₁₀ alkyl, C₂-C₁₀ alkenyl, C₂-C₁₀ alkynyl, C₁-C₁₀ alkoxy, C₁-C₁₀ alkoxycarbonyl, C₃-C₈ cycloalkyl, C₃-C₈ heterocycloalkyl, C₄-C₁₀ aryl, and C₅-C₁₀ heteroaryl; the heteroatom is N, O, or S; and m may be a natural number of 1 to 4.

Further more specifically, R₃ is hydrogen, substituted or unsubstituted methyl, ethyl, n-propyl, isopropyl, butyl, isobutyl, t-butyl, pentyl, neopentyl, substituted or unsubstituted C₄-C₈ aryl, substituted or unsubstituted C₄-C₈ aryloxy, substituted or unsubstituted -(CH₂)ₘ-C₄-C₁₀ heterocycloalkyl, or substituted or unsubstituted -(CH₂)ₘ-NR'₅R'₆; and R'₅ and R'₆ are each independently hydrogen or C₁-C₃ alkyl, still further more specifically, R₃ may be methyl, ethyl, n-propyl, isopropyl, n-butyl, isobutyl, t-butyl, pentyl, neopentyl, phenyl, or phenyl substituted with a halogen atom, and even further more specifically, R₃ may be methyl, isopropyl, t-butyl, phenyl, or neopentyl.

Furthermore, particularly preferred examples among the naphthoquinone-based compounds represented by Chemical Formula 4 include the following compounds, but are not limited thereto.

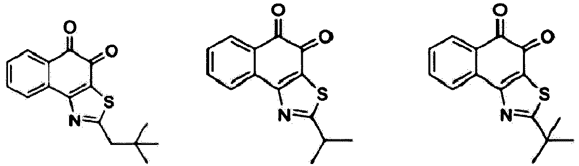

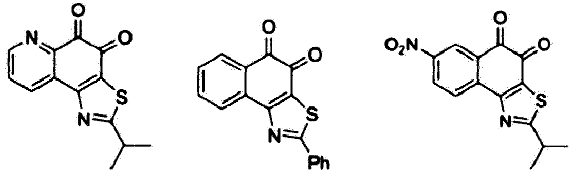

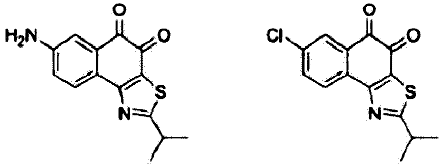

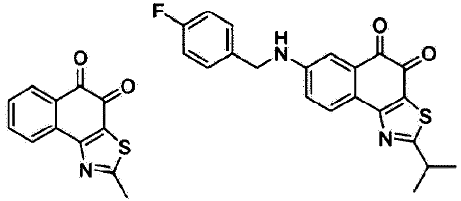

The naphthoquinone-based compound represented by Chemical Formula 4 used in the composition according to the present invention may be prepared by the method disclosed in Korean Patent No. 10-1644778, and may be prepared by other known methods and/or various methods based on techniques in the field of organic synthesis. Various derivatives may be synthesized using an appropriate synthesis method according to the kind of a substituent based on the aforementioned methods.

In addition, the naphthoquinone-based compound may include, as a component, a compound represented by Chemical Formula 5, a pharmaceutically acceptable salt thereof, a prodrug thereof, an isomer thereof, or a solvate thereof.

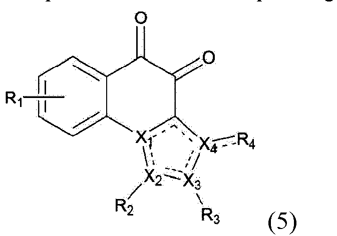

In Chemical Formula 5, X₁, X₂, X₃, and X₄ are each independently selected from the group consisting of carbon, nitrogen, oxygen, and sulfur atoms, and at least two among X₁, X₂, X₃, and X₄ are heteroatoms selected from among nitrogen, oxygen, and sulfur atoms, with a proviso that when X₁ and X₂ are each carbon atom, X₃ and X₄ may not be nitrogen atoms at the same time; R₁ is one or more selected from the group consisting of hydrogen, alkyl, alkyloxy, halo, nitro, hydroxy, cyano, and -NR₅R₆; R₂ is absent or selected from the group consisting of hydrogen, oxygen, alkyl, alkyloxy, C₆-₁₀ aryl, and heterocyclyl, wherein the alkyl may be substituted with C₆-₁₀ aryl, and the heterocyclyl may be substituted with -C(O)Rs; R₃ is absent or selected from the group consisting of hydrogen, oxygen, halo, alkyl, alkyloxy, C₆-₁₀ aryl, heterocyclyl, -SO₂NR₇R₁₂, -NR₉R₁₀, and -C(O)R₁₁, wherein when the alkyl is substituted, the substituent is selected from the group consisting of halo, alkyloxy, C₆-₁₀ aryl, C₆-₁₀ aryloxy, heterocyclyl, -C(O)R₈, R₁₂C(O)O-, and -NR₁₃R₁₄, and the heterocyclyl may be substituted with -C(O)Rs; R₄ is absent or selected from the group consisting of hydrogen, oxygen, alkyl, alkyloxy, C₆-₁₀ aryl, C₆-₁₀ aryloxy, heterocyclyl, and -C(O)R₁₅, when the alkyl is substituted, the substituent is selected from the group consisting of halo, C₆-₁₀ aryl, heterocyclyl, and -C(O)R₈, and the heterocyclyl may be substituted with -C(O)Rs; R₅ and R₆ are each independently selected from the group consisting of hydrogen, alkyl, and -C(O)R₇; R₇ and R₁₂ are each alkyl; R₁₁ is heterocyclyl or -NR₁₃R₁₄; R₁₅ is alkyl, alkyloxy, C₆-₁₀ aryloxy, heterocyclyl, or -NR₁₃R₁₄; R₉, R₁₀, R₁₃, and R₁₄ are each independently selected from the group consisting of hydrogen, alkyl, unsubstituted or halo-substituted C₆-₁₀ aryl, and -C(O)Rs; Rs's are each alkyloxy; wherein alkyl may be linear or branched alkyl having 1 to 10 carbon atoms or cyclic alkyl having 3 to 7 carbon atoms, the heterocyclyl is a 3- to 7-membered heterocyclic group having, in the ring, one or more heteroatoms selected from the group consisting of a nitrogen atom, an oxygen atom, and a sulfur atom, and when the aryl is substituted, the substituents are each one or more selected from the group consisting of halo, C₁-₆ alkyl, halo-substituted alkyl, and alkyloxy; and - - - is a single bond or a double bond depending on R₂, R₃, R₄, X₁, X₂, X₃, and X₄, with a proviso that when X₁ and X₄ are each carbon atom and X₂ and X₃ are each nitrogen atom, one among R₂ and R₄ is not alkyl, aryl, or heterocyclyl, wherein when R₂ is alkyl, aryl, or heterocyclyl, R₄ is not -C(O)R₁₅.

In a preferred example, the compound represented by Chemical Formula 5 may be a compound in which X₁ and X₄ are carbon atoms and X₂ and X₃ are nitrogen atoms. Here, in Chemical Formula 5, R₂ is absent or is alkyl, alkyloxy, or C₆-₁₀ aryl, R₃ is absent or is hydrogen, alkyl, or C₆-₁₀ aryl, and R₄ is absent or is oxygen, alkyl, or alkyloxy, wherein one among R₂ and R₄ is not alkyl or aryl, and when the alkyl or aryl is substituted, the substituent is as defined above.

In another preferred example, the compound represented by Chemical Formula 5 may be a compound in which X₁ and X₃ are carbon atoms and X₂ and X₄ are nitrogen atoms. Here, in Chemical Formula 5, R₂ may be absent or may be alkyl, R₃ may be selected from the group consisting of halo, alkyl, alkyloxy, C₆-₁₀ aryl, and heterocyclyl, and R₄ may be absent or may be selected from the group consisting of hydrogen, alkyl, C₆-₁₀ aryl, C₆-₁₀ aryloxy, heterocyclyl, and -C(O)R₁₅. Here, when R₁₅ and the alkyl, aryl, or heterocyclyl are substituted, the substituents are as defined above.

In yet another preferred example, the compound represented by Chemical Formula 5 may be a compound in which X₁ and X₃ are carbon atoms, X₂ is a nitrogen atom, and X₄ is a sulfur atom. Here, in Chemical Formula 5, R₂ and R₄ are absent and R₃ is alkyl or C₆-₁₀ aryl, wherein when the alkyl or aryl is substituted, the substituent is as defined above.

In still another preferred example, the compound represented by Chemical Formula 5 may be a compound in which X₁ and X₃ are carbon atoms, and one among X₂ and X₄ is a nitrogen atom and the other is an oxygen atom. Here, in Chemical Formula 5, R₂ is absent, R₃ is oxygen, alkyl, or C₆-₁₀ aryl, and R₄ is absent or is hydrogen or alkyl, wherein when the alkyl or aryl is substituted, the substituent is as defined above.

In yet still another preferred example, the compound represented by Chemical Formula 5 may be a compound in which X₂, X₃, and X₄ are nitrogen atoms. Here, in Chemical Formula 5, R₂ is absent or is alkyl or heterocyclyl, R₃ is absent or selected from the group consisting of alkyl, C₆-₁₀ aryl, heterocyclyl, -SO₂R₇, -NR₉R₁₀, and -C(O)R₁₁, and R₄ is absent or is selected from the group consisting of alkyl, heterocyclyl, and -C(O)R₁₅. Here, when R₇, R₉, R₁₀, R₁₁, or R₁₅ and the alkyl, aryl, or heterocyclyl are substituted, the substituents are as defined above.

In yet still another preferred example, the compound represented by Chemical Formula 5 may be a compound in which X₂ and X₃ are carbon atoms and X₁ and X₄ are nitrogen atoms. Here, in Chemical Formula 5, R₂ is C₆-₁₀ aryl and R₃ and R₄ are absent, where when the aryl is substituted, the substituent is as defined above.

Particularly preferred examples among the naphthoquinone-based compounds represented by Chemical Formula 5 include compounds in Table 2 below, but are not limited thereto.

**[Table 2]**

| **No.** | **Compound** | **No.** | **Compound** | **No.** | **Compound** |
|---|---|---|---|---|---|
| 1 | 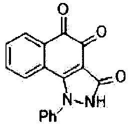 | 2 | 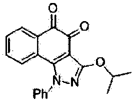 | 3 | 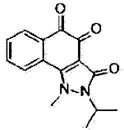 |
| 4 | 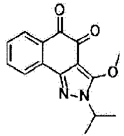 | 5 | 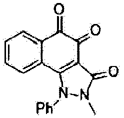 | 6 | 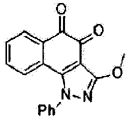 |
| 7 | 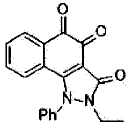 | 8 | 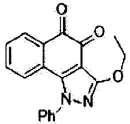 | 9 | 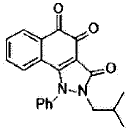 |
| 10 | 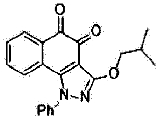 | 11 | 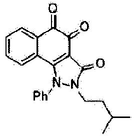 | 12 | 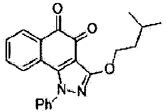 |
| 13 | 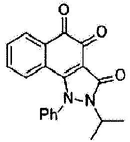 | 14 | 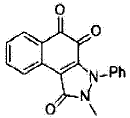 | 15 | 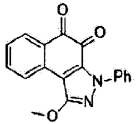 |
| 16 | 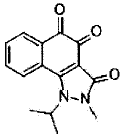 | 17 | 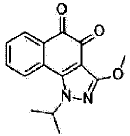 | 18 | 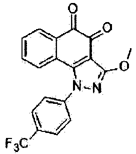 |
| 19 | 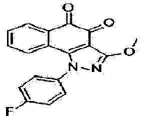 | 20 | 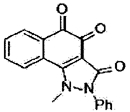 | 21 | 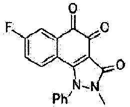 |
| 22 | 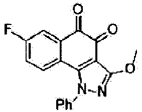 | 23 | 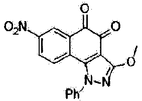 | 24 | 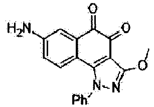 |
| 25 | 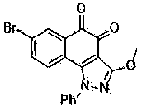 | 26 | 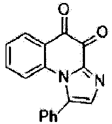 | 27 | 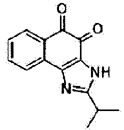 |
| 28 | 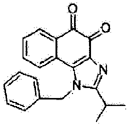 | 29 | 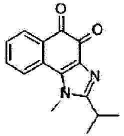 | 30 | 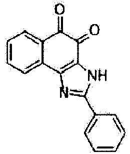 |
| | | | | | |
| 31 | 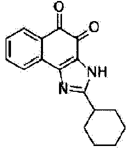 | 32 | 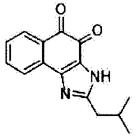 | 33 | 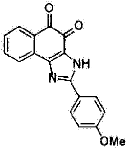 |
| 34 | 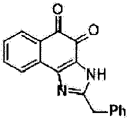 | 35 | 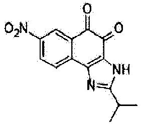 | 36 | 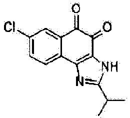 |
| 37 | 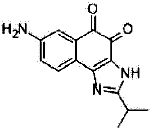 | 38 | 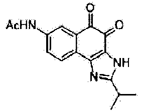 | 39 | 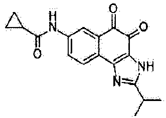 |
| 40 | 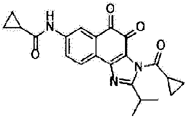 | 41 | 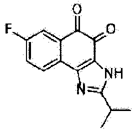 | 42 | 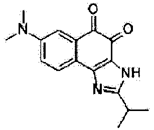 |
| 43 | 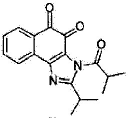 | 44 | 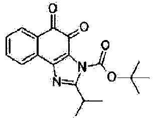 | 45 | 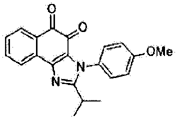 |
| 46 | 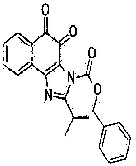 | 47 | 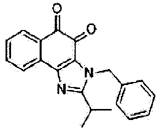 | 48 | 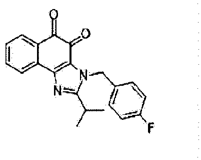 |
| 49 | 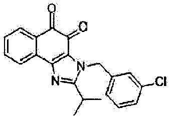 | 50 | 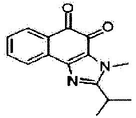 | 51 | 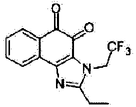 |
| 52 | 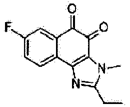 | 53 | 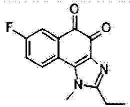 | 54 | 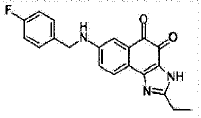 |
| 55 | 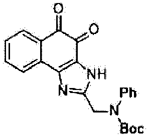 | 56 | 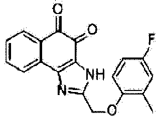 | 57 | 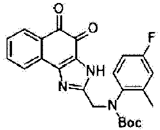 |
| 58 | 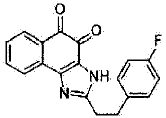 | 59 | 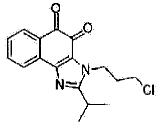 | 60 | 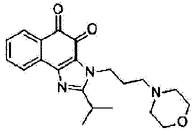 |
| 61 | 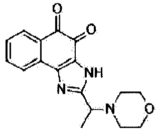 | 62 | 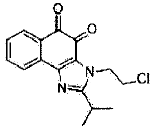 | 63 | 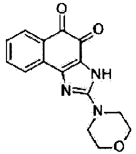 |
| 64 | 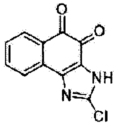 | 65 | 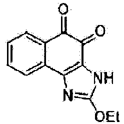 | 66 | 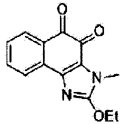 |
| 67 | 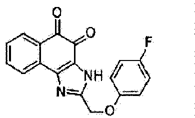 | 68 | 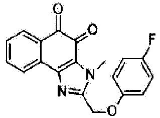 | 69 | 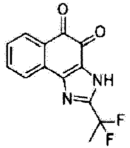 |
| 70 | 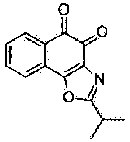 | 71 | 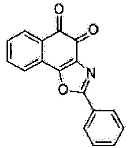 | 72 | 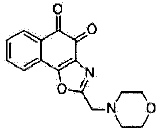 |
| 73 | 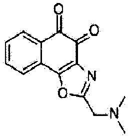 | 74 | 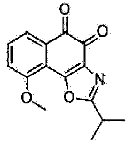 | 75 | 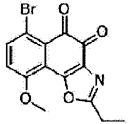 |
| 76 | 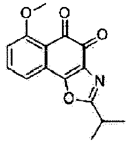 | 77 | 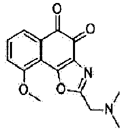 | 78 | 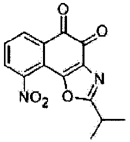 |
| 79 | 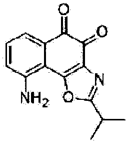 | 80 | 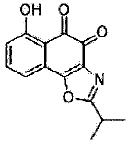 | 81 | 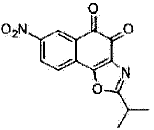 |
| 82 | 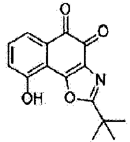 | 83 | 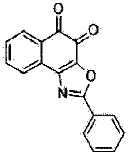 | 84 | 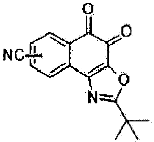 |
| 85 | 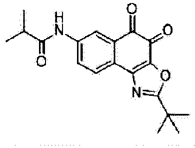 | 86 | 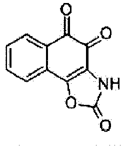 | 87 | 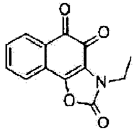 |
| 88 | 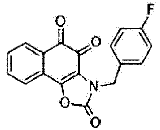 | 89 | 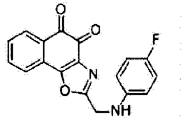 | 90 | 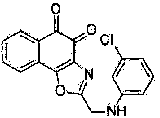 |
| 91 | 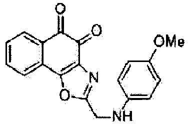 | 92 | 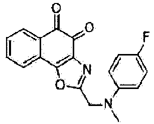 | 93 | 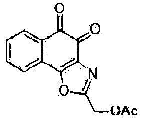 |
| 94 | 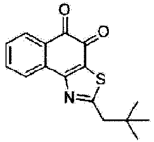 | 95 | 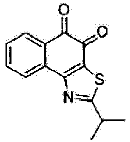 | 96 | 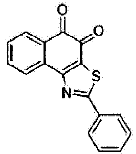 |
| 97 | 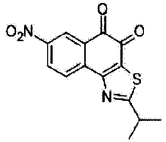 | 98 | 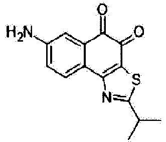 | 99 | 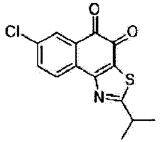 |
| 100 | 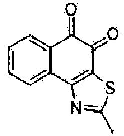 | 101 | 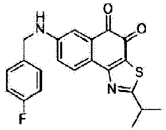 | 102 | 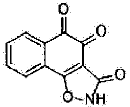 |
| 103 | 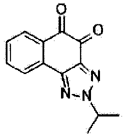 | 104 | 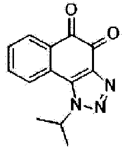 | 105 | 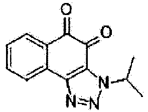 |
| 106 | 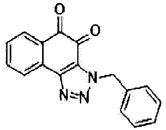 | 107 | 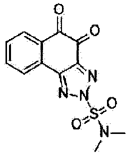 | 108 | 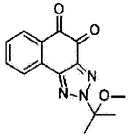 |
| 109 | 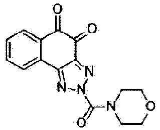 | 110 | 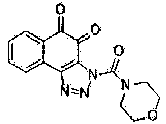 | 111 | 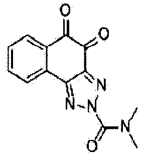 |
| 112 | 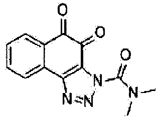 | 113 | 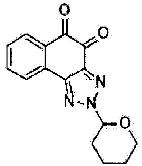 | 114 | 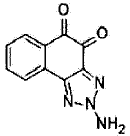 |
| 115 | 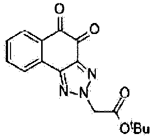 | 116 | 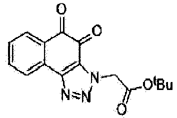 | 117 | 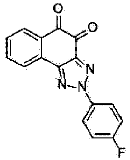 |
| 118 | 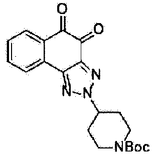 | 119 | 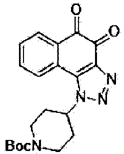 | 120 | 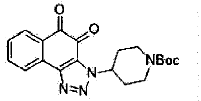 |
| 121 | 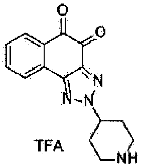 | 122 | 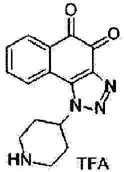 | 123 | 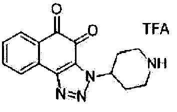 |
| 124 | 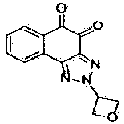 | 125 | 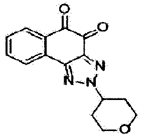 | 126 | 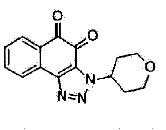 |
| 127 | 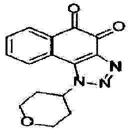 | 128 | 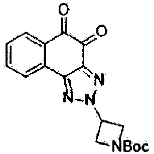 | 129 | 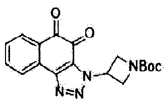 |
| 130 | 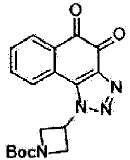 | | | | |

The naphthoquinone-based compound represented by Chemical Formula 5 used in the composition according to the present invention may be prepared by the method disclosed in Korean Patent Publication No. 10-2247694, and may be prepared by other known methods and/or various methods based on techniques in the field of organic synthesis. Various derivatives may be synthesized using an appropriate synthesis method according to the kind of a substituent based on the aforementioned methods.

As used herein, the term "alkyl" refers to a radical which does not have an unsaturated group and contains carbon and hydrogen. The alkyl radical may be linear (line shape) or branched (branch shape). Exemplary alkyl includes methyl, ethyl, propyl, isopropyl, butyl, t-butyl, sec-butyl, and the like, but is not limited thereto. C₁-C₁₀ alkyl is an alkyl group having 1 to 10 carbon atoms in the main chain of linear or branched alkyl. The alkyl group may be optionally substituted. When the alkyl group is substituted, the alkyl group may be substituted with 4 or less substituents at any specific bonding point (at any prescribed carbon atom). Meanwhile, the alkyl group is substituted with an alkyl group, it is used synonymously with a "branched alkyl group".

The term "alkenyl" refers to an unsaturated aliphatic group which is similar to the alkyl in terms of length and substitutability but contains one or more carbon-carbon double bonds. For example, the "alkenyl" includes a linear alkenyl group (for example, ethenyl, propenyl, butenyl, pentenyl, hexenyl, heptenyl, octenyl, nonenyl, or decenyl), a branched alkenyl group, and a cycloalkenyl group (for example, cyclopropenyl, cyclopentenyl, cyclohexenyl, cycloheptenyl, or cyclooctenyl). In addition, the "alkenyl" may further include an alkenyl group containing oxygen, nitrogen, sulfur, or phosphorus atoms substituting for one or more carbon atoms of the hydrocarbon main chain. In a specific example, a linear or branched alkenyl group may have 6 or less carbon atoms in the main chain (for example, C₂-C₆ for a linear alkenyl group, and C₃-C₆ for a branched alkenyl group). Similarly, the cycloalkenyl group may have 3 to 8 carbon atoms and more preferably 5 or 6 carbon atoms in a ring structure.

The term "alkynyl" refers to an unsaturated aliphatic group which is similar to the alkyl in terms of length and substitutability but contains one or more carbon-carbon triple bonds. For example, the "alkynyl" includes a linear alkynyl group (for example, ethynyl, propynyl, butynyl, pentynyl, hexynyl, heptynyl, octynyl, nonynyl, or decynyl) and a branched alkynyl group (including an alkyl- or alkenylsubstituted alkynyl group). In addition, the "alkynyl" may further include an alkynyl group containing oxygen, nitrogen, sulfur, or phosphorus atoms substituting for one or more carbon atoms of the hydrocarbon main chain. In a specific example, a linear or branched alkynyl group has 6 or less carbon atoms in the main chain (for example, C₂-C₆ for a linear alkynyl group, and C₃-C₆ for a branched alkynyl group).

When the alkyl, alkynyl, and alkenyl are each substituted, the substituent may be, for example, hydroxy, carboxylate, oxo, halogen (for example, F, Cl, Br, or I), C₁-C₆ haloalkyl (for example, CCl₃ or CF₃), carbamoyl (-NHCOOR or - OCONHR), urea (-NHCONHR), thiol, cyano, nitro, amino, acylamino, C₁-C₁₀ alkylthio, C₆-C₁₀ arylthio, C₁-C₁₀ alkyl, C₁-C₁₀ alkoxy, C₆-C₁₀ aryloxy, C₁-C₁₀ alkylcarbonyloxy, C₆-C₁₀ arylcarbonyloxy, C₃-C₈ cycloalkyl, C₃-C₈ cycloalkyloxy, C₂-C₁₀ alkenyl, C₂-C₁₀ alkynyl, C₆-C₁₀ aryl, aminocarbonyl, C₁-C₁₀ alkylcarbonyl, C₃-C₈ cycloalkylcarbonyl, 3- to 10-membered heterocycloalkylcarbonyl, C₆-C₁₀ arylcarbonyl, C₆-C₁₀ aryloxycarbonyl, C₁-C₁₀ alkoxycarbonyl, C₃-C₈ cycloalkyloxycarbonyl, 3- to 10-membered heterocycloalkyloxycarbonyl, C₁-C₁₀ alkylsulfonyl, C₆-C₁₀ arylsulfonyl, C₁-C₁₀ alkylamino, 3- to 10-membered heterocycloalkyl, 5- to 10-membered heteroaryl, or the like.

Preferably, the substituent may be one or more selected from among hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, 3- to 10-membered heterocycloalkyl, C₆-C₁₀ aryl, and 5- to 10-membered heteroaryl.

The term "cycloalkyl" refers to an alkyl species containing 3 to 15 carbon atoms and preferably 3 to 8 carbon atoms, without alternating or resonant double bonds between carbon atoms. The cycloalkyl may contain 1 to 4 rings. Exemplary cycloalkyl includes cyclopropyl, cyclobutyl, cyclopentyl, cyclohexyl, adamantyl, and the like. An exemplary substituent of the cycloalkyl may be halogen, C₁-C₁₀ alkyl, C₁-C₁₀ alkoxy, amino, nitro, cyano, thiol, C₁-C₁₀ alkylthio, and the like.

The term "heterocycloalkyl" refers to a substitution product in which a carbon atom in a ring is substituted with a heteroatom such as nitrogen, sulfur, or oxygen, and refers to a saturated or unsaturated 7- to 10-membered bicyclic heterocyclic ring or a stable non-aromatic 3- to 8-membered monocyclic heterocyclic ring, and an additional ring may be formed through the fusion, spiro, or crosslinking between the rings. Each heterocyclic ring consists of one or more carbon atoms and one to four heteroatoms. The heterocycloalkyl may be bonded to any endocyclic ring that creates a stable structure. Preferred examples thereof include furan, thiophene, pyrrole, pyrroline, pyrrolidine, oxazole, thiazole, imidazole, imidazoline, imidazolidine, pyrazole, pyrazoline, pyrazolidine, isothiazole, triazole, thiadiazole, pyran, pyridine, piperidine, morpholine, thiomorpholine, pyridazine, pyrimidine, pyrazine, piperazine, and triazine, but are not limited thereto.

The term "aryl" refers to an aromatic substituent which has at least one ring with a shared pi electron system and includes carbocyclic aryl (for example, phenyl) and heterocyclic aryl (for example, pyridine). The term includes monocyclic or fused-ring polycyclic (that is, rings that share adjacent pairs of carbon atoms) groups. The aryl group may be carbocyclic, or may optionally contain 1 to 4 heteroatoms (for example, nitrogen, sulfur, or oxygen) in the aromatic ring, and such an aryl group is also referred to as "heteroaryl".

Examples of the aryl or heteroaryl include phenyl, naphthyl, pyridyl, pyrimidyl, pyrrolyl, isothiazolyl, triazolyl, tetrazolyl, pyrazolyl, oxazolyl, isoxazolyl, pyrazinyl, pyridazinyl, triazinyl, quinazolinyl, thiazolyl, benzothiophenyl, furanyl, imidazolyl, and thiophenyl, but are not limited thereto.

The cycloalkyl, heterocycloalkyl, aryl, and heteroaryl may be optionally substituted, and examples of the substituent include hydroxy, halogen, thiol, cyano, nitro, amino, acylamino, C₁-C₁₀ alkylthio, C₆-C₁₀ arylthio, C₁-C₁₀ alkyl, C₁-C₁₀ alkoxy, C₆-C₁₀ aryloxy, C₁-C₁₀ alkylcarbonyloxy, C₆-C₁₀ arylcarbonyloxy, C₃-C₈ cycloalkyl, C₃-C₈ cycloalkyloxy, C₂-C₁₀ alkenyl, C₂-C₁₀ alkynyl, C₆-C₁₀ aryl, carboxylate, aminocarbonyl, C₁-C₁₀ alkylcarbonyl, C₃-C₈ cycloalkylcarbonyl, 3- to 10-membered heterocycloalkylcarbonyl, C₆-C₁₀ arylcarbonyl, C₆-C₁₀ aryloxycarbonyl, C₁-C₁₀ alkoxycarbonyl, C₃-C₈ cycloalkyloxycarbonyl, 3- to 10-membered heterocycloalkyloxycarbonyl, C₆-C₁₀ aryloxycarbonyl, C₁-C₁₀ alkylsulfonyl, C₁-C₁₀ alkylamino, C₆-C₁₀ arylsulfonyl, 3- to 10-membered heterocycloalkyl, and 5- to 10-membered heteroaryl, but are not limited thereto.

In addition, the heterocycloalkyl and heteroaryl have 1 to 3 heteroatoms selected from the group consisting of N, O, and S.

The range of the active ingredients contained in the composition according to the present invention includes all of the naphthoquinone-based compounds, pharmaceutically acceptable salts thereof, prodrugs thereof, solvates thereof, or isomers thereof. Unless otherwise specified in the present specification, the term "naphthoquinone-based compound" may be used as a concept including all of the compound itself, a pharmaceutically acceptable salt thereof, a prodrug thereof, a solvate thereof, and an isomer thereof.

As used herein, the term "pharmaceutically acceptable salt" refers to a compound dosage form which does not cause serious irritation to the organism to which a compound is administered and does not impair the biological activity and physical properties of the compound. The pharmaceutically acceptable salt includes an acid addition salt formed of an acid that forms a non-toxic acid addition salt containing a pharmaceutically acceptable anion, and examples of the acid include an inorganic acid such as hydrochloric acid, sulfuric acid, nitric acid, phosphoric acid, hydrobromic acid, or hydroiodic acid, an organic carboxylic acid such as tartaric acid, formic acid, citric acid, acetic acid, trichloroacetic acid, trifluoroacetic acid, gluconic acid, benzoic acid, lactic acid, fumaric acid, maleic acid, or salicylic acid, and a sulfonic acid such as methanesulfonic acid, ethanesulfonic acid, benzenesulfonic acid, or p-toluenesulfonic acid. Meanwhile, examples of a base addition salt include an alkali metal salt or alkaline earth metal salt formed with lithium, sodium, potassium, calcium, magnesium, or the like, an amino acid salt such as lysine, arginine, or guanidine, and an organic salt such as dicyclohexylamine, N-methyl-D-glucamine, tris(hydroxymethyl)methylamine, diethanolamine, choline, or triethylamine. As used herein, the naphthoquinone-based compound may be converted into a salt thereof by a conventional method.

The term "prodrug" refers to a substance which is transformed into a parent drug *in vivo.* The prodrug is easier to administer than the parent drug, and is thus frequently used. For example, the prodrug may obtain physiological activity through oral administration, but the parent drug may not. The prodrug may also have improved solubility in the medicine composition compared to the parent drug. For example, the prodrug would be a compound of which the water solubility is detrimental to mobility, but which is once hydrolyzed into carboxylic acid, which is an activator, through metabolism in cells with favorable water solubility, and is administered as an ester that facilitates the permeation of cell membranes. Another example of the prodrug may be a short peptide (polyamino acid) bound to an acidic group that is converted by metabolism so as to expose the active site of the peptide. The prodrug according to the present invention includes the compounds described in International Publication No. WO 2006/020719, but is not limited to the compounds.

As used herein, the term "solvate" refers to a compound according to the present invention or a salt thereof, which contains a stoichiometric or non-stoichiometric amount of a solvent bound by a non-covalent intermolecular force. Preferred solvents include solvents which are volatile, non-toxic, and/or suitable for administration to humans, and when the solvent is water, it means a hydrate.

The term "isomer" refers to compounds according to the present invention or salts thereof, which have the same chemical formula or molecular formula but are optically or sterically different. For example, the compounds according to the present invention may have a chiral carbon center, and thus may be present in the form of an R or S isomer, a racemic compound, individual enantiomers or a mixture thereof, or individual diastereomers or a mixture thereof, and all such stereoisomers and mixtures thereof may fall within the scope of the present invention.

### Immune checkpoint inhibitor

As used herein, the term "immune checkpoint" refers to an intracellular signaling system that maintains self-tolerance and protects tissues from excessive immune responses that cause damage. The immune checkpoint protein is a cellular membrane protein that regulates immune checkpoints and may inhibit the differentiation, proliferation, and activity of immune cells. Specifically, the immune checkpoint protein is expressed in activated T cells, and has a function of reducing proliferation, cytokine secretion, and cytotoxicity of T cells and inhibiting excessive activity of T cells. Some immune checkpoints are known as one of the main mechanisms by which tumor cells induce immune evasion. Therefore, the "immune checkpoint inhibitor" targets the immune checkpoint protein and inhibits or blocks immune checkpoints to increase T cell activation, thereby enhancing antitumor immunity and exhibiting anticancer effects. In addition to the advantages of having fewer side effects such as vomiting and hair loss and greater therapeutic effect compared to typical cytotoxic anticancer agents, the immune checkpoint inhibitor uses an immune response system with excellent memory ability, and is thus known to have a long-lasting therapeutic effect even after drug administration is stopped.

Specifically, the immune checkpoint inhibitor may target any one immune checkpoint selected from the group consisting of cytotoxic T-lymphocyte-associated antigen 4 (CTLA4), programmed cell death protein 1 (PD-1), programmed death-ligand 1 (PD-L1), programmed death-ligand 2 (PD-L2), lymphocyte activation gene 3 (LAG3), B7-H3 (CD276), B7-H4 (VTCN1; V-set domain containing T cell activation inhibitor 1), herpesvirus entry mediator (HVEM), killer cell immunoglobulin-like receptor (KIR), OX40 (CD134, TNFRSF4; TNF receptor superfamily member 4), IgG, indoleamine-2,3-dioxygenase 1 (IDO-1), indoleamine-2,3-dioxygenase 2 (IDO-2), carcinoembryonic antigen cell adhesion molecule 1 (CEACAM1), B- and T-lymphocyte attenuator (BTLA), OX40 ligand (OX40L), T cell membrane protein 3 (TIM3), galectin 9 (GAL9), V-domain Ig suppressor of T cell activation (VISTA), T cell immunoglobulin and ITIM domain (TIGIT), and combinations thereof. The immune checkpoint inhibitor may be purchased from a conventional manufacturer or the like and then used, or may be prepared according to a known production method and then used.

As used herein, the immune checkpoint inhibitor may be an antibody that binds to the proteins of cancer cells, for example, a monoclonal antibody, and may inhibit immune checkpoints to induce a response in which T cells kill cancer cells.

Is an embodiment, the immune checkpoint inhibitor may be any one selected from among an anti-CTLA4 antibody, an anti-PD-1 antibody, an anti-PD-L1 antibody, an anti-PD-L2 antibody, an anti-LAG3 antibody, an anti-B7-H3 antibody, an anti-B7-H4 antibody, an anti-HVEM antibody, an anti-KIR antibody, an anti-OX40 antibody, an anti-IgG antibody, an anti-IDO-1 antibody, an anti-IDO-2 antibody, an anti-CEACAM1 antibody, an anti-BTLA antibody, an anti-OX40L antibody, an anti-TIM3 antibody, an anti-GAL9 antibody, an anti-VISTA antibody, an anti-TIGIT antibody, and combinations thereof.

Specifically, the immune checkpoint inhibitor may be any one selected from the group consisting of: an anti-CTLA4 antibody, an antigen-binding fragment thereof, or a variant thereof; an anti-PD-L1 antibody, an antigen-binding fragment thereof, or a variant thereof; an anti-PD-1 antibody, an antigen-binding fragment thereof, or a variant thereof; an anti-LAG3 antibody, an antigen-binding fragment thereof, or a variant thereof; and combinations thereof.

As used herein, the term "cytotoxic T-lymphocyte-associated antigen 4 (CTLA-4)" is also referred to as CD152, and CTLA-4 is expressed on the membrane surface of activated T cells. CTLA-4 binds to CD80 (B7-1) and CD86 (B7-2) of antigen-presenting cells to inhibit the activity of T cells. The CTLA-4 inhibitors may be ipilimumab (YERVOY^{®}) and tremelimumab.

As used herein, the term "programmed death-ligand 1 (PD-L1)" is also referred to as CD274 or B7-H1, and refers to a protein present on the surface of cancer cells or in hematopoietic cells. PD-L1 on the surface of cancer cells may bind to PD-1 on the surface of T cells. The PD-L1 inhibitors may be, for example, atezolizumab, avelumab (BABENCIO^{®}), durvalumab (IMFINZI^{®}), KN035, CK-301, AUNP12, CA-170, and BMS-986189.

As used herein, the term "programmed cell death protein 1 (PD-1)" is also referred to as CD279, and refers to a protein expressed on the surface of activated T cells. PD-1 reacts with PD-L1 (B7-H1) and PD-L2 (B7-DC), which are proteins present on the surface of cancer cells, to inhibit T cell receptor (TCR)- and CD28-mediated T-cell activation and the production of growth factors and cytokines, thereby inducing negative signaling. The PD-1 inhibitors are, for example, pembrolizumab (KEYTRUDA^{®}), MK-3475, nivolumab (OPDIVO^{®}), cemiplimab (LIBTAYO^{®}), JTX-4014, spartalizumab, camrelizumab, sintilimab, tislelizumab, toripalimab, dostarlimab, INCMGA00012, AMP-224, and AMP-514.

As used herein, the term "lymphocyte activation gene 3 (LAG3)" is also referred to as CD223, and LAG3 binds to major histocompatibility complex (MHC) class II to inhibit the proliferation and activity of T cells. The LAG3 inhibitors may be IMP321, relatlimab, and GSK2831781.

In an embodiment of the present invention, there is provided the composition containing the naphthoquinone-based compound and immune checkpoint inhibitor, a combination having an excellent effect of inhibiting cancer cell proliferation compared to the case where each ingredient is administered alone. For example, the naphthoquinone-based compound may be compound No. 2 (dunnione; 2,3,3-trimethyl-2H-benzo[g][1]benzofuran-4,5-dione) of Table 1, and the immune checkpoint inhibitor may be any one selected from the group consisting of an anti-CTLA4 antibody, an anti-PD-L1 antibody, an anti-PD-1 antibody, an anti-LAG3 antibody, and combinations thereof.

The "dunnione" is a naphthoquinone-based compound, and is divided into two structures: alpha-dunnione (2,3-dihydro-2,3,3-trimethyl naphtho[1,2-b]furan-4,9-dione); and dunnione (2,3-dihydro-2,3,3-trimethyl naphtho[1,2-b]furan-4,5-dione). In addition, the dunnione is obtained from the leaves of *Streptocarpus dunnii,* which grows naturally in South America, or from several kinds of Calceolaria. According to the pharmacological action of the dunnione that has been reported so far, the dunnione has been reported to increase NQO1 (NAD(P)H: quinone oxidoreductase 1) enzyme activity to induce an increase in NAD⁺ and the like in cells, and effective in preventing and treating damage of the small intestine mucosa caused by anticancer agents, and acute pancreatitis or the like which is caused by alcohol, gallstones in the duct of the pancreas, or the like, through activation of deacetylases, such as Sirtuin 1, which uses the NAD⁺ as a coenzyme (Pandit et al., Biochem Biophys Res Commun 2015; 467:697-703/Shen et al., Sci Rep 2017; 7:3006). In addition, U.S. Patent No. 9,066,922 B2 discloses that the dunnione may be used for preventing and treating obesity, diabetes, metabolic syndrome, neurodegenerative diseases, or mitochondrial dysfunction-related diseases.

### Immunogenic cell death inducer

As used herein, the term "immunogenic cell death" refers to a type of cell death caused by cell proliferation inhibitors such as oxaliplatin, cyclophosphamide, paclitaxel, and docetaxel, or radiation therapy and photodynamic therapy. The immunogenic cell death is different from typical cell death, and the immunogenic cell death of cancer cells may induce an effective anticancer immune response through the activation of dendritic cells and the subsequent activation of specific T cell responses. A substance that induces immunogenic cell death is referred to as an immunogenic cell death inducer. The details of the immunogenic cell death and the immunogenic cell death inducer are well summarized in Kroemer et al. (Annu. Rev. Immunol., 31: 51-72, 2013). This document is hereby incorporated by reference in its entirety.

The immunogenic cell death inducer used as an active ingredient in the composition according to the present invention may be a general-purpose anticancer agent and/or a targeted anticancer agent that attacks only cancer cells through respective cancer-specific molecular targets. Specifically, the immunogenic cell death inducer may be an anthracycline-based anticancer agent, a taxane-based anticancer agent, an anti-EGFR antibody, a BK channel agonist, bortezomib, a cardiac glycoside, a cyclophosphamide-based anticancer agent, a GADD34/PP1 inhibitor, LV-tSMAC, measles virus, or oxaliplatin. The anthracycline-based anticancer agent may be daunorubicin, doxorubicin, epirubicin, idarubicin, pixantrone, sabarubicin, or valrubicin, the taxane-based anticancer agent may be paclitaxel or docetaxel, and the anti-EGFR antibody may be cetuximab.

In a more specific example, the immunogenic cell death inducer may be any one selected from the group consisting of capecitabine, 5-fluorouracil, thioguanine, chlorambucil, oxaliplatin, cisplatin, carboplatin, paclitaxel, docetaxel, irinotecan, doxorubicin, vinorelbine, gemcitabine, pemetrexed, adriamycin, etoposide, vincristine, cytarabine, cyclophosphamide, ifosfamide, tamoxifen, anastrozole, letrozole, exemestane, fulvestrant, temozolomide, carmustine, lomustine, epirubicin, eribulin, toremifene, goserelin, megestrol, vinblastine, bendamustine, thiotepa, bleomycin, topotecan, leucovorin, trifluridine, tipiracil, mitoxantrone, mitomycin C, aldesleukin, temsirolimus, everolimus, mechlorethamine, methotrexate, pemetrexed, trastuzumab, bevacizumab, cetuximab, aflibercept, pertuzumab, ramucirumab, panitumumab, nivolumab, necitumumab, pembrolizumab, obinutuzumab, ofatumumab, erlotinib, gefitinib, sorafenib, lapatinib, dinaciclib, palbociclib, regorafenib, imatinib, sunitinib, axitinib, pazopanib, afatinib, ceritinib, crizotinib, osimertinib, bosutinib, dasatinib, nilotinib, ponatinib, hydroxyurea, procarbazine, abemaciclib, vistusertib, and combinations thereof.

In one embodiment of the present invention, there is provided the composition containing the naphthoquinone-based compound and immunogenic cell death inducer, a combination having an excellent effect of inhibiting cancer cell proliferation compared to the case where each ingredient is administered alone. For example, the naphthoquinone-based compound may be compound No. 2 (dunnione) of Table 1, and the immunogenic cell death inducer may be any one selected from the group consisting of 5-fluorouracil, oxaliplatin, paclitaxel, irinotecan, leucovorin, adriamycin, docetaxel, cyclophosphamide, mitoxantrone, everolimus, gefitinib, sorafenib, dinaciclib, regorafenib, afatinib, carboplatin, abemaciclib, vistusertib, lapatinib, pemetrexed, and combinations thereof.

In addition, in one embodiment of the present invention, there is provided a composition for co-administration, which is for treating cancer and contains a naphthoquinone-based compound, an immune checkpoint inhibitor, and an immunogenic cell death inducer.

For example, the naphthoquinone-based compound may be compound No. 2 (dunnione) of Table 1.

In addition, the immune checkpoint inhibitor may be any one selected from among an anti-CTLA4 antibody, an anti-PD-1 antibody, an anti-PD-L1 antibody, an anti-PD-L2 antibody, an anti-LAG3 antibody, an anti-B7-H3 antibody, an anti-B7-H4 antibody, an anti-HVEM antibody, an anti-KIR antibody, an anti-OX40 antibody, an anti-IgG antibody, an anti-IDO-1 antibody, an anti-IDO-2 antibody, an anti-CEACAM1 antibody, an anti-BTLA antibody, an anti-OX40L antibody, an anti-TIM3 antibody, an anti-GAL9 antibody, an anti-VISTA antibody, an anti-TIGIT antibody, and combinations thereof. Preferably, the immune checkpoint inhibitor may be any one selected from the group consisting of an anti-CTLA4 antibody, an anti-PD-L1 antibody, an anti-PD-1 antibody, an anti-LAG3 antibody, and combinations thereof.

In addition, the immunogenic cell death inducer may be any one selected from the group consisting of capecitabine, 5-fluorouracil, thioguanine, chlorambucil, oxaliplatin, cisplatin, carboplatin, paclitaxel, docetaxel, irinotecan, doxorubicin, vinorelbine, gemcitabine, pemetrexed, adriamycin, etoposide, vincristine, cytarabine, cyclophosphamide, ifosfamide, tamoxifen, anastrozole, letrozole, exemestane, fulvestrant, temozolomide, carmustine, lomustine, epirubicin, eribulin, toremifene, goserelin, megestrol, vinblastine, bendamustine, thiotepa, bleomycin, topotecan, leucovorin, trifluridine, tipiracil, mitoxantrone, mitomycin C, aldesleukin, temsirolimus, everolimus, mechlorethamine, methotrexate, pemetrexed, trastuzumab, bevacizumab, cetuximab, aflibercept, pertuzumab, ramucirumab, panitumumab, nivolumab, necitumumab, pembrolizumab, obinutuzumab, ofatumumab, erlotinib, gefitinib, sorafenib, lapatinib, dinaciclib, palbociclib, regorafenib, imatinib, sunitinib, axitinib, pazopanib, afatinib, ceritinib, crizotinib, osimertinib, bosutinib, dasatinib, nilotinib, ponatinib, hydroxyurea, procarbazine, abemaciclib, vistusertib, and combinations thereof. Preferably, the immunogenic cell death inducer may be any one selected from the group consisting of 5-fluorouracil, oxaliplatin, paclitaxel, irinotecan, leucovorin, adriamycin, docetaxel, cyclophosphamide, mitoxantrone, everolimus, gefitinib, sorafenib, dinaciclib, regorafenib, afatinib, carboplatin, abemaciclib, vistusertib, and combinations thereof.

Cancer, which may be prevented or treated with the composition according to the present invention, may be any one selected from the group consisting of liver cancer, gastric cancer, colon cancer, breast cancer, lung cancer, non-small cell lung cancer, bone cancer, pancreatic cancer, skin cancer, head or neck cancer, skin or intraocular melanoma, uterine cancer, ovarian cancer, colorectal cancer, small intestine cancer, rectal cancer, perianal cancer, fallopian tube carcinoma, endometrial carcinoma, cervical carcinoma, vaginal carcinoma, vulva carcinoma, Hodgkin's disease, esophageal cancer, lymph gland cancer, bladder cancer, gallbladder cancer, endocrine gland cancer, thyroid cancer, parathyroid cancer, adrenal cancer, soft tissue sarcoma, urethral cancer, penile cancer, prostate cancer, adenocarcinoma, chronic or acute leukemia, lymphocytic lymphoma, kidney or ureter cancer, renal cell carcinoma, renal pelvic carcinoma, central nervous system (CNS) tumor, primary CNS lymphoma, spinal cord tumor, brainstem glioma, pituitary adenoma, and combinations thereof, but is not limited thereto.

Another aspect of the present invention provides a pharmaceutical composition for preventing or treating cancer, the pharmaceutical composition containing a naphthoquinone-based compound as an active ingredient, wherein the naphthoquinone-based compound is used in combination with at least one selected from among an immune checkpoint inhibitor and an immunogenic cell death inducer. In other words, the naphthoquinone-based compound may be utilized for the use in combination with the immune checkpoint inhibitor and/or the immunogenic cell death inducer in order to prevent or treat cancer.

Here, for the descriptions of the naphthoquinone-based compound, the immune checkpoint inhibitor, and the immunogenic cell death inducer, refer to the aforementioned descriptions.

Specifically, for the purpose of preventing or treating cancer, the naphthoquinone-based compound may be used in the form of a combination drug in which the naphthoquinone-based compound is mixed with at least one selected from among the immune checkpoint inhibitor and the immunogenic cell death inducer, or may be used in the form in which the naphthoquinone-based compound and at least one selected from among the immune checkpoint inhibitor and the immunogenic cell death inducer are formulated, and simultaneously or sequentially administered.

For example, the naphthoquinone-based compound may be in a dosage form for oral administration, and may be administered once a week to 7 times a week, 2 times a week to 5 times a week, or 3 times a week. Here, the naphthoquinone-based compound may be used in combination with an immune checkpoint inhibitor, which is in a dosage form for intraperitoneal administration, and the immune checkpoint inhibitor may be administered daily, at intervals of 2, 3, 4, 5, or 6 days, or once a week to 3 times a week. In addition, the naphthoquinone-based compound may be used in combination with an immunogenic cell death inducer, which is in a dosage form for intraperitoneal administration or oral administration, and the immunogenic cell death inducer may be administered daily, at intervals of 2, 3, 4, 5, or 6 days, or once a week to 3 times a week. The naphthoquinone-based compound may be administered independently of the immune checkpoint inhibitor and/or the immunogenic cell death inducer according to the schedules for the respective substances.

In addition, the naphthoquinone-based compound, the immune checkpoint inhibitor, and the immunogenic cell death inducer may be co-administered in a weight ratio of 1 : 0.001 to 500 : 0.1 to 1,000, in a weight ratio of 1 : 0.001 to 300 : 0.1 to 500, or in a weight ratio of 1 : 0.001 to 0.1 : 0.2 to 5, based on a single dosage. For example, the naphthoquinone-based compound and the immune checkpoint inhibitor may be co-administered in a weight ratio of 1,000:1 to 1:10, 500:1 to 1:1, 250:1 to 5:1, 200:1 to 2.5:1, or 100:1. In addition, the naphthoquinone-based compound and the immunogenic cell death inducer may be co-administered in a weight ratio of 5:1 to 1:5, 3:1 to 1:3, 2:1 to 1:2, 3:2 to 2:3, or 1:0.001 to 0.001:1.

The pharmaceutical composition for preventing or treating cancer according to the present invention may be prepared in a dosage form for oral administration, and orally administered. Examples of the dosage form for oral administration include a tablet, a pill, a hard/soft capsule, a liquid medicine, a suspension, an emulsifier, a syrup, a granule, and an elixir, and such a dosage form contains a diluent (for example, lactose, dextrose, sucrose, mannitol, sorbitol, cellulose, and/or glycine) and a lubricant (for example, silica, talc, stearic acid and a magnesium or calcium salt thereof, and/or polyethylene glycol), in addition to the active ingredient. The tablet may also contain binders such as magnesium aluminum silicate, starch paste, gelatin, methyl cellulose, sodium carboxymethyl cellulose, and/or polyvinylpyrrolidone, and may optionally contain disintegrants or effervescent mixtures such as starch, agar, and alginic acid or a sodium salt thereof, and/or an absorbent, a colorant, a flavoring agent, and a sweetening agent.

In addition, the pharmaceutical composition for preventing or treating cancer according to the present invention may be parenterally administered, and the parenteral administration is performed by a method for injecting a subcutaneous injection, an intravenous injection, an intramuscular injection, or an intrathoracic injection. Here, in order to be formulated into a dosage form for parenteral administration, the pharmaceutical composition containing the naphthoquinone-based compound as an active ingredient, and a stabilizer or buffer may be mixed with water to prepare a solution or a suspension, which may be prepared in an ampoule or vial unit-administration form. The composition may be sterilized and/or may contain an adjuvant such as a preservative, a stabilizer, a wetting agent, an emulsification accelerator, or a salt and/or a buffer for regulating osmotic pressure, and other therapeutically useful substances. In addition, the composition may be formulated according to a conventional method such as mixing, granulating, or coating. In the same manner, the naphthoquinone-based compound and the immune checkpoint inhibitor and/or the immunogenic cell death inducer may be formulated together or separately, and simultaneously or sequentially administered.

In yet another aspect of the present invention, there is provided a method for preventing or treating cancer, the method including administering, to a subject, a pharmaceutical composition containing a naphthoquinone-based compound, and at least one selected from among an immune checkpoint inhibitor (ICI) and an immunogenic cell death (ICD) inducer.

Specifically, the pharmaceutical composition may contain a naphthoquinone-based compound and an immune checkpoint inhibitor. In addition, the pharmaceutical composition may contain a naphthoquinone-based compound and an immunogenic cell death inducer. Furthermore, the pharmaceutical composition may contain a naphthoquinone-based compound, an immune checkpoint inhibitor, and an immunogenic cell death inducer.

The naphthoquinone-based compound, immune checkpoint inhibitor, immunogenic cell death inducer, and administration are as described above.

The term "prevention" refers to any action that inhibits the occurrence of cancer or delays the onset of cancer through the administration of the pharmaceutical composition. The term "treatment" refers to any action that improves or beneficially changes the symptoms of cancer through the administration of the pharmaceutical composition.

The pharmaceutical composition according to the present invention may be for treating any one or more cancers selected from the group consisting of colorectal cancer, breast cancer, renal cancer, skin cancer (melanoma), liver cancer, and lung cancer, but is not limited thereto.

More specifically, when the cancer is colorectal cancer, for example, using a combination of dunnione as the naphthoquinone-based compound, an anti-PD-1 antibody and/or an anti-CTLA4 antibody as the immune checkpoint inhibitor, and regorafenib or a combination drug of oxaliplatin and 5-fluorouracil as the immunogenic cell death inducer may bring out a remarkable effect on a tumor growth inhibition rate.

When the cancer is breast cancer, for example, using a combination of dunnione as the naphthoquinone-based compound, an anti-PD-1 antibody or an anti-CTLA4 antibody as the immune checkpoint inhibitor, and mitoxantrone or lapatinib as the immunogenic cell death inducer may bring out a remarkable effect on a tumor growth inhibition rate.

When the cancer is renal cancer, for example, using a combination of dunnione as the naphthoquinone-based compound, an anti-PD-1 antibody or an anti-CTLA4 antibody as the immune checkpoint inhibitor, and everolimus, sorafenib, or vistusertib as the immunogenic cell death inducer may bring out a remarkable effect on a tumor growth inhibition rate.

When the cancer is skin cancer, for example, using a combination of dunnione as the naphthoquinone-based compound, an anti-PD-L1 antibody, an anti-PD-1 antibody, or an anti-CTLA4 antibody as the immune checkpoint inhibitor, and paclitaxel, regorafenib, vistusertib, or everolimus as the immunogenic cell death inducer may bring out a remarkable effect on a tumor growth inhibition rate.

When the cancer is liver cancer, for example, using a combination of dunnione as the naphthoquinone-based compound, an anti-CTLA4 antibody as the immune checkpoint inhibitor, and sorafenib or everolimus as the immunogenic cell death inducer may bring out a remarkable effect on a tumor growth inhibition rate.

When the cancer is lung cancer, for example, using a combination of dunnione as the naphthoquinone-based compound, an anti-PD-1 antibody as the immune checkpoint inhibitor, and a combination drug of carboplatin and pemetrexed as the immunogenic cell death inducer may bring out a remarkable effect on a tumor growth inhibition rate.

The pharmaceutical composition according to the present invention may be administered to a patient in a therapeutically effective amount or in a pharmaceutically effective amount.

Here, the "therapeutically effective amount" or "pharmaceutically effective amount" is an amount of a compound or composition effective in preventing or treating a target disease, and refers to an amount which is sufficient to treat a disease with a reasonable benefit/risk ratio applicable to a medical treatment, and does not cause side effects. The level of the effective amount may be determined according to factors including health conditions of a patient, kinds of disease, severity, a drug activity, sensitivity to drugs, an administration method, an administration time, an administration route, an excretion rate, a treatment duration, a combination, or concurrently used drugs, and other factors well known in the medical field.

The subject may be a mammal such as a human, a cow, a horse, a pig, a dog, a sheep, a goat, or a cat. The subject may be a patient suffering from the cancer such as liver cancer, gastric cancer, colon cancer, breast cancer, or lung cancer, or a subject who is more likely to suffer from the cancer.

In still another aspect of the present invention, there is provided a use of a composition, which is for preparing a pharmaceutical composition for preventing or treating cancer, wherein the composition contains a naphthoquinone-based compound and at least one selected from among an immune checkpoint inhibitor (ICI) and an immunogenic cell death (ICD) inducer.

Yet still another aspect of the present invention provides a use of a composition, which is for preventing or treating cancer, wherein the composition contains a naphthoquinone-based compound and at least one selected from among an immune checkpoint inhibitor (ICI) and an immunogenic cell death (ICD) inducer.

The naphthoquinone-based compound, immune checkpoint inhibitor, and immunogenic cell death inducer are as described above.

Hereinafter, the present invention will be described in detail with reference to the following Examples. Here, the following Examples are only for illustratively describing the present invention, and the content of the present invention is not limited by the following Examples.

### Example 1. Tumor proliferation assessment

### Example 1.1. Preparation of tumor implantation animal model and assessment method

All mice used in the experiments were raised in a sterile animal room with constant temperature (22°C to 26°C) and constant humidity (55% to 60%), acclimatized for 1 week while sufficiently supplying typical solid feed (Samtako Inc., Korea) and water, and then used. All experiments were conducted after obtaining approval from the Institutional Animal Care and Use Committee in accordance with the laboratory animal care and ethics regulations of the Institutional Animal Care and Use Committee of Wonkwang University.

7-week-old BALB/c or C57BL/6 mice were used, and subcutaneously inoculated using a 1-mL insulin syringe within 30 minutes after mixing the mouse cancer cells with 0.2 mL of PBS. Information on animal models for respective cancer types is shown in Table 3 below.

**[Table 3]**

| Type | Mouse | Cell line |
|---|---|---|
| Colorectal cancer I | C57BL/6 | MC38 (2.5 × 10⁶ cells) |
| Colorectal cancer II | C57BL/6 | CT26 (2.5 × 10⁵ cells) |
| Breast cancer I | BALB/c | 4T1 (2.5 × 10⁶ cells) |
| Breast cancer II | BALB/c | EMT6 (5 × 10⁴ cells) |
| Renal cancer | BALB/c | RENCA (5 × 10⁵ cells) |
| Skin cancer (melanoma) | BALB/c | B16F10 (1 × 10⁴ cells) |
| Liver cancer | C57BL/6 | Hepal-6 (1 × 10⁷ cells) |
| Lung cancer I | DBA/2 | KLN205 (3 × 10⁵ cells) |
| Lung cancer II | C57BL/6 | LL/2 (1 × 10⁵ cells) |

After the cancer cell line was subcutaneously implanted, the administration of drugs was started when the size of the cancer reached 50 to 100 mm³. The volumes (mm³) of the tumors, which were generated after the inoculation, were measured at intervals of 3 days using a caliper, calculation was performed using the formula of length × width/2, and then the results thereof were compared.

### Example 1.2. Assessment of tumor proliferation according to sole administration and co-administration of respective drugs

Dunnione (2,3,3-trimethyl-2H-benzo[g][1]benzofuran-4,5-dione) represented by the following structural formula was used as the naphthoquinone-based compound (C₁₅H₁₄O₃, 242.274 g/mol):

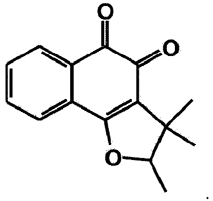

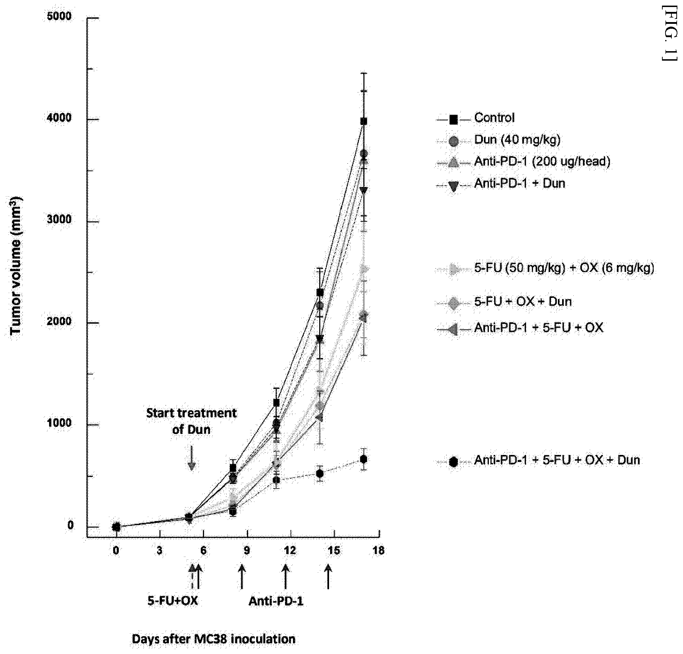

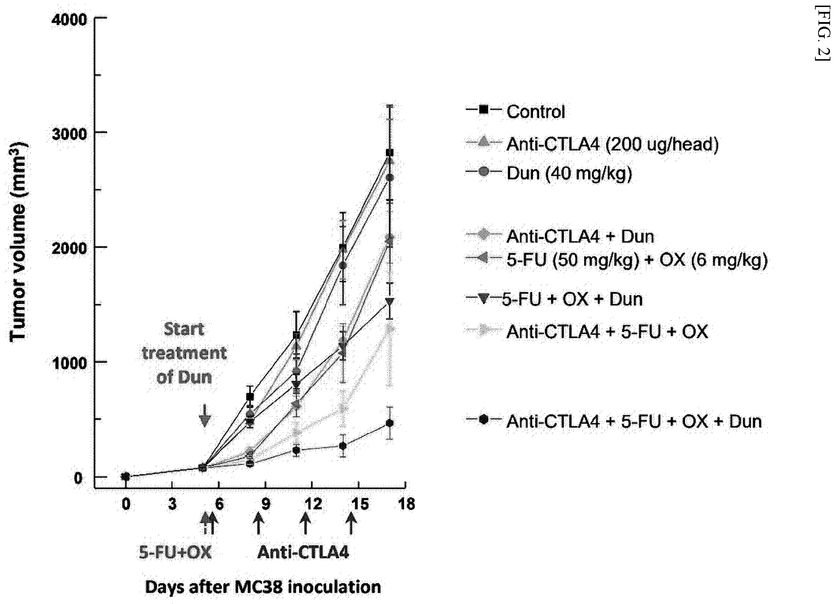

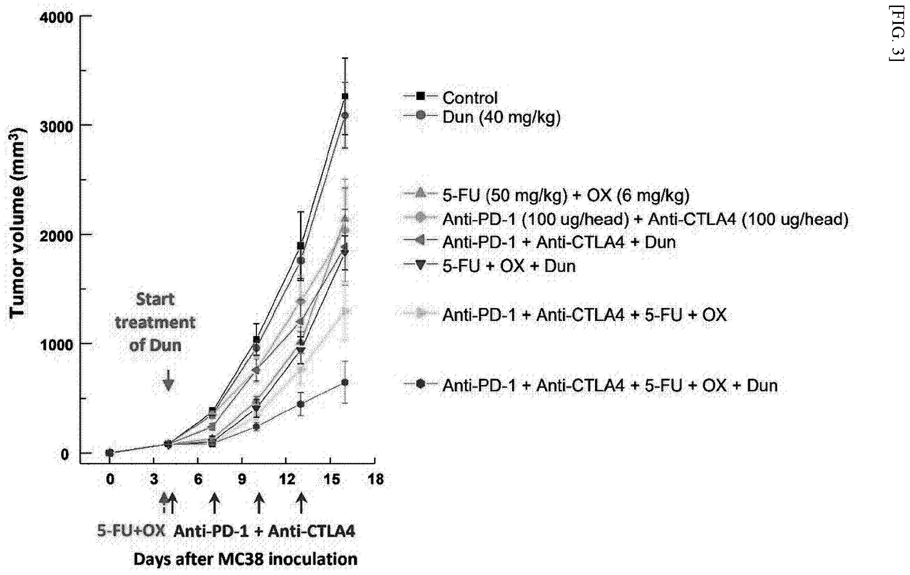

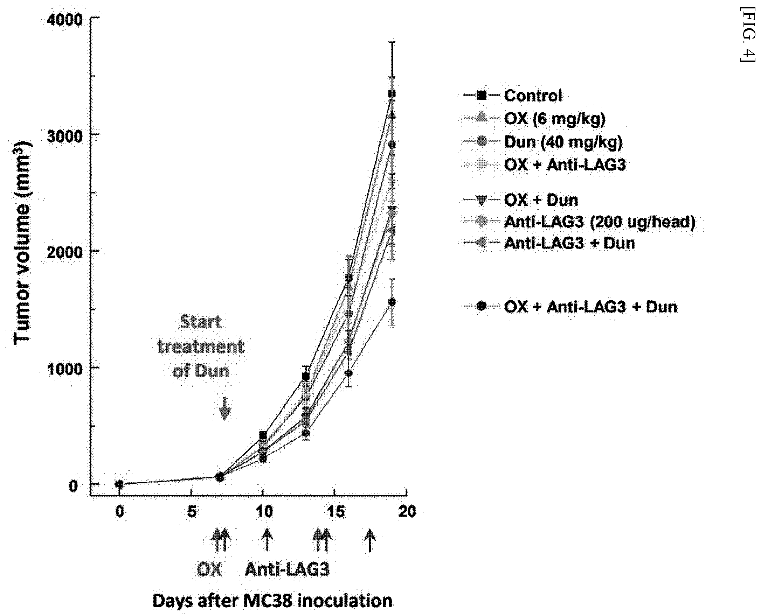

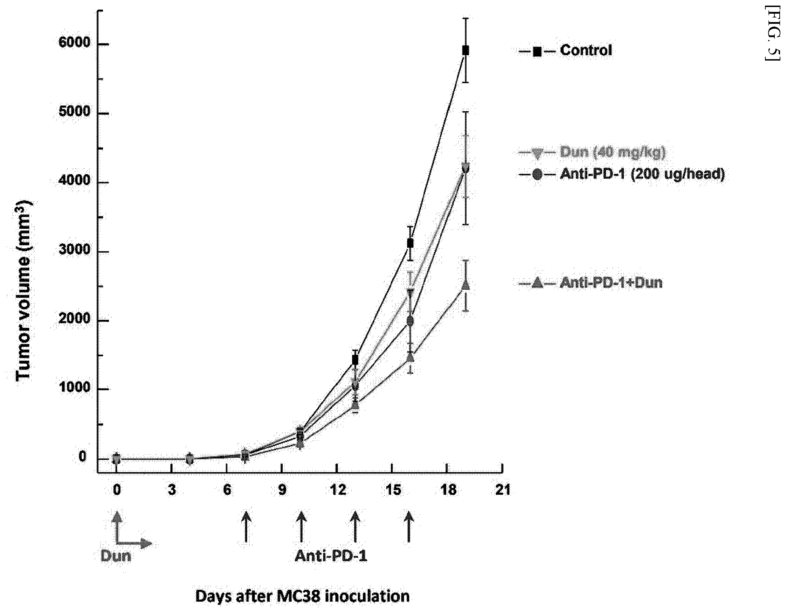

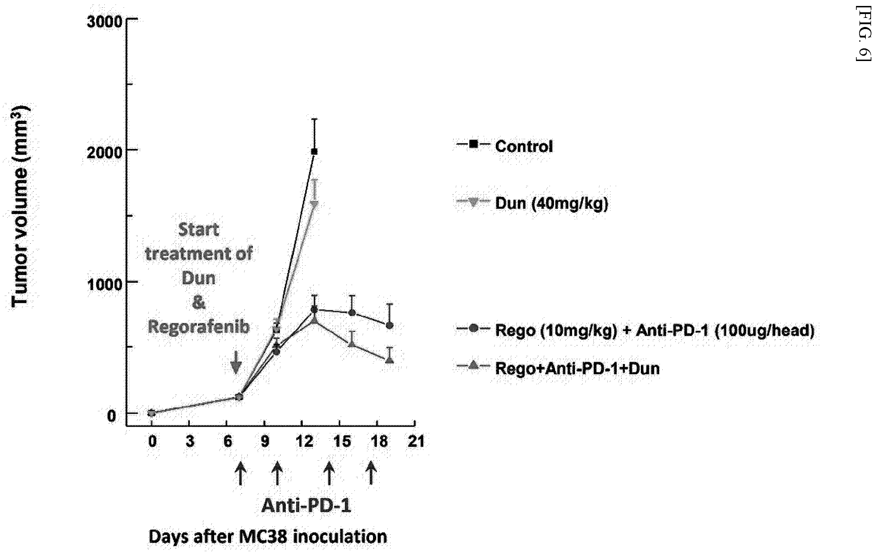

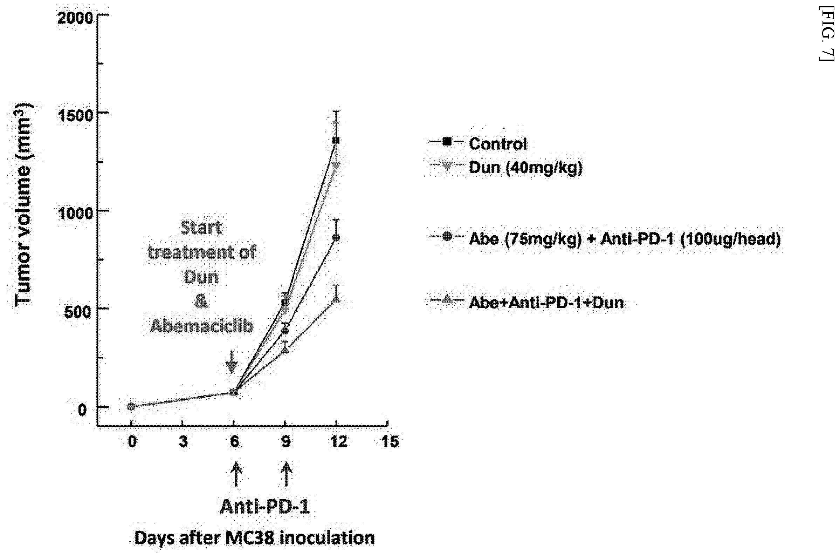

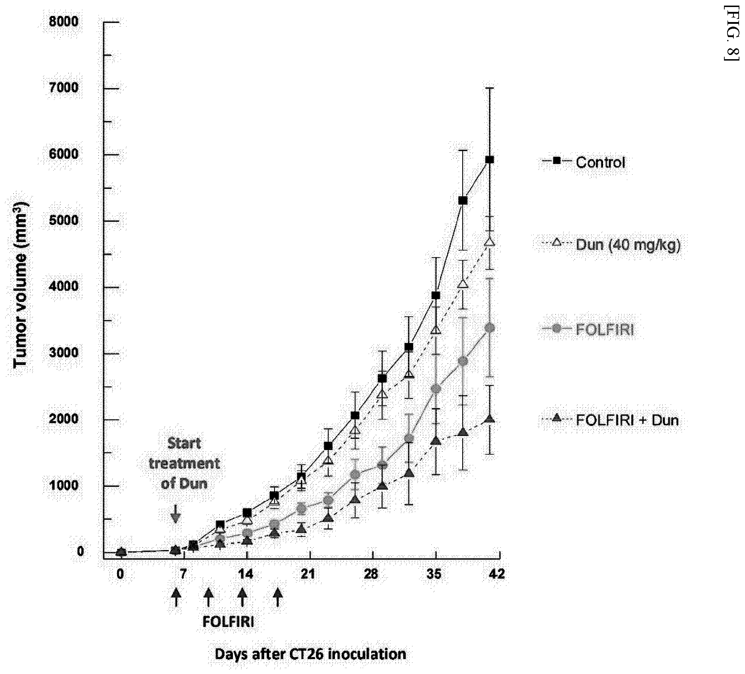

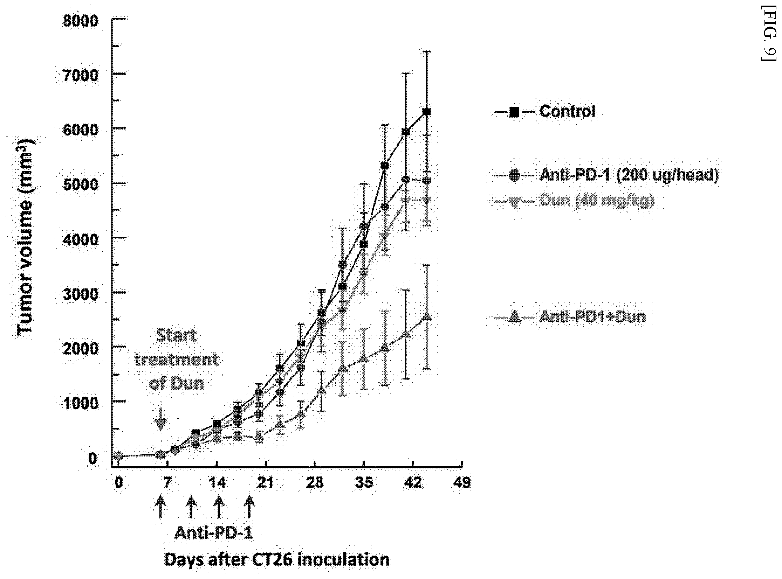

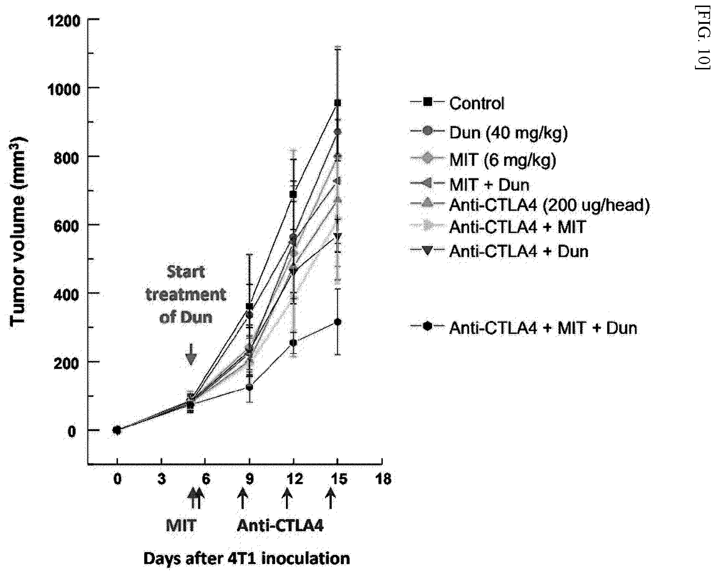

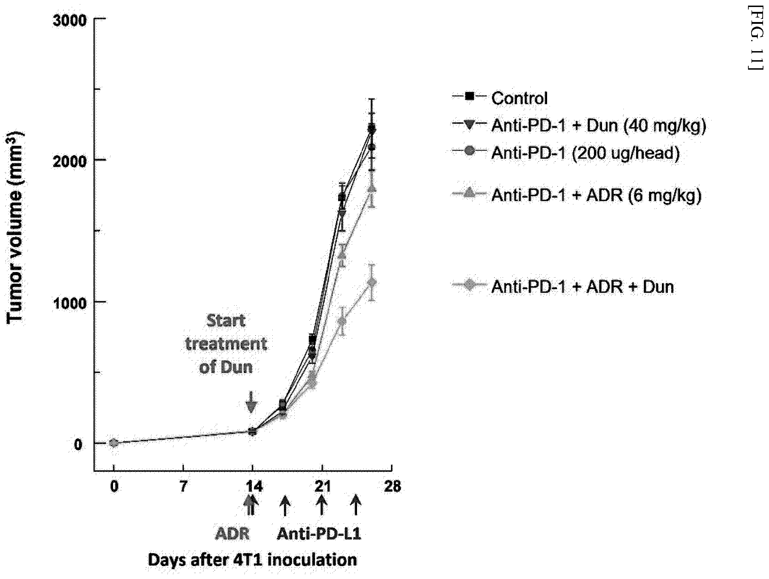

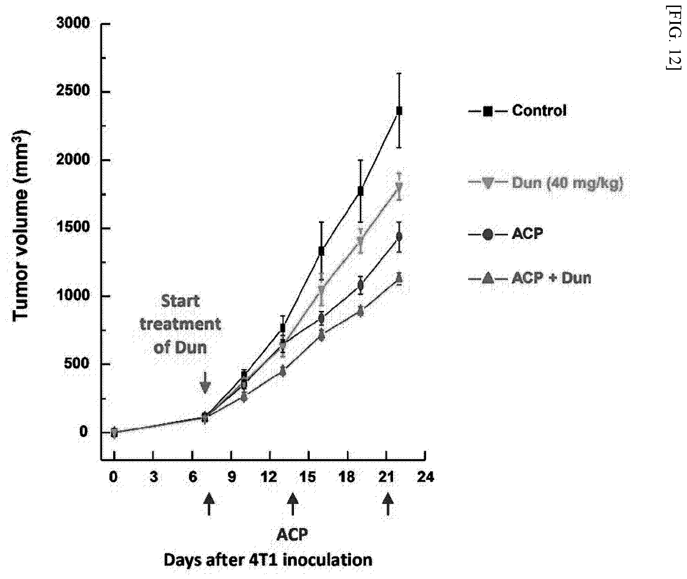

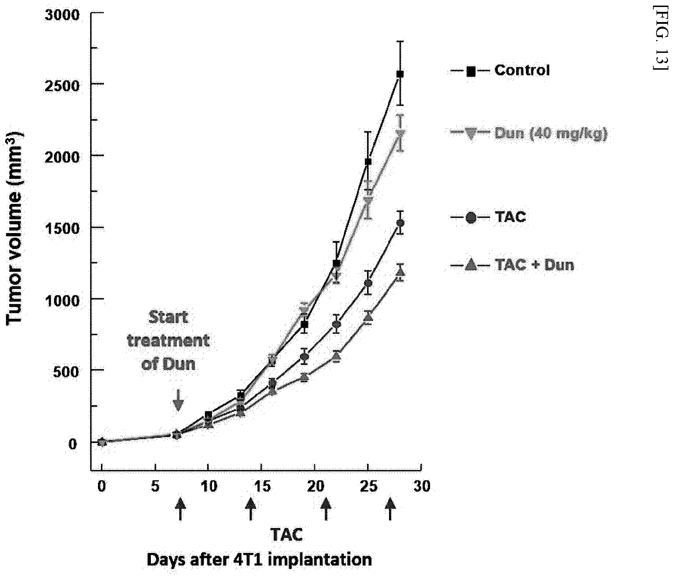

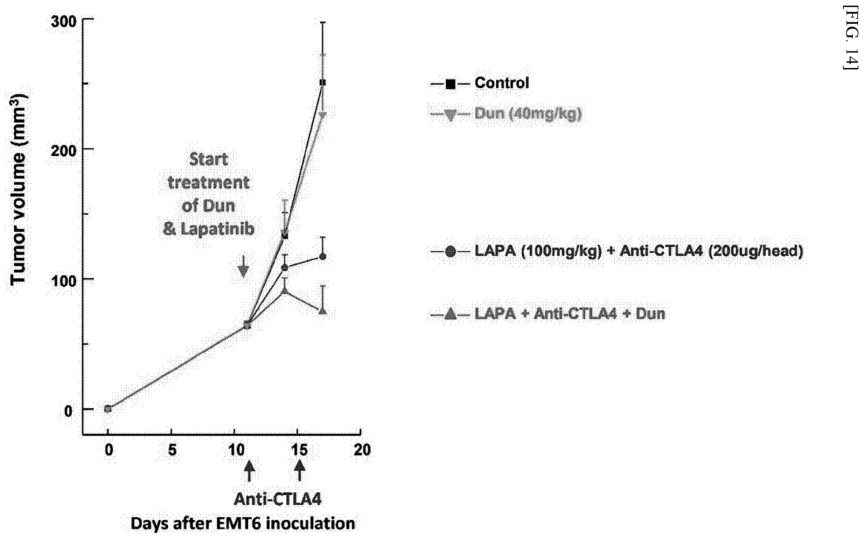

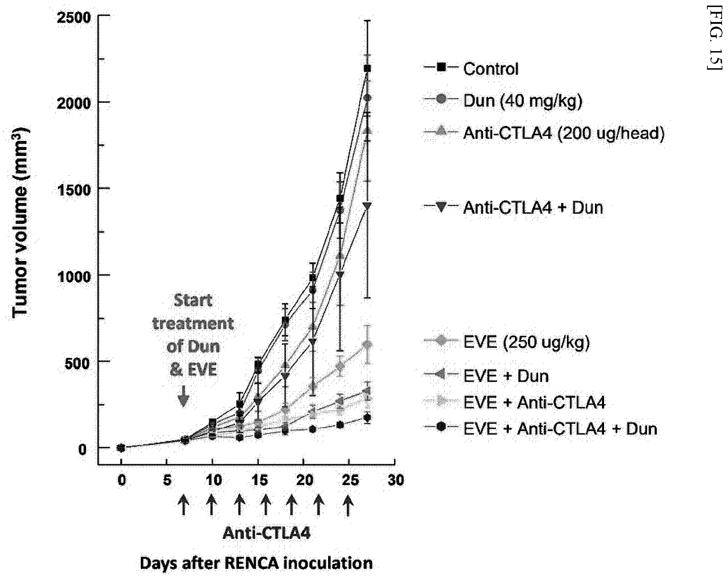

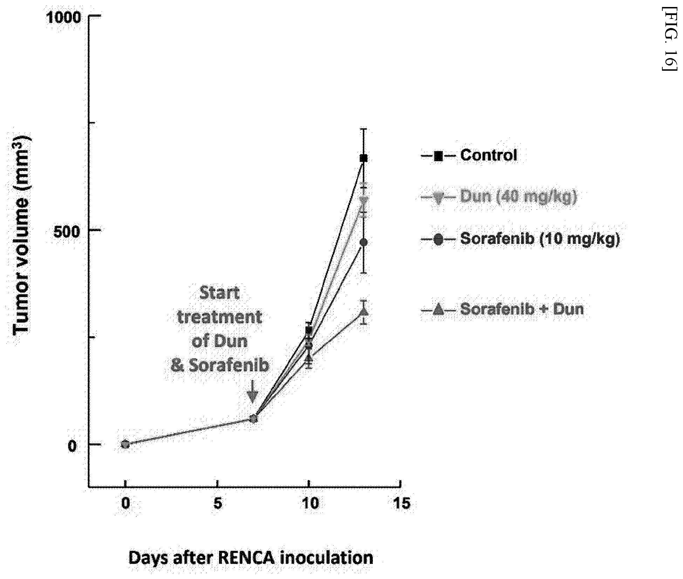

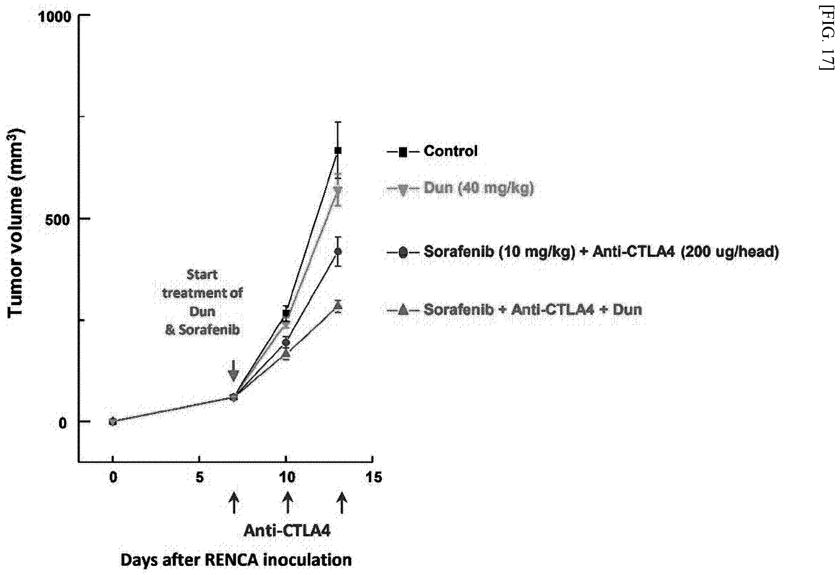

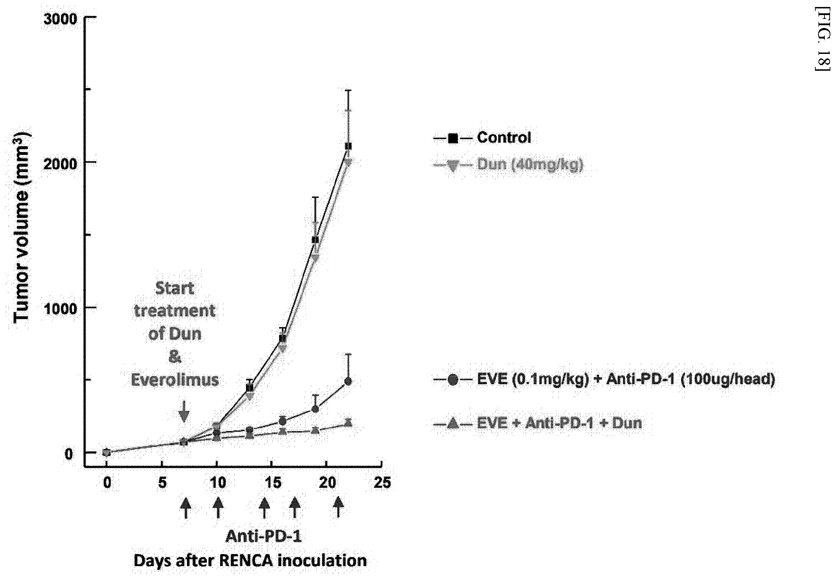

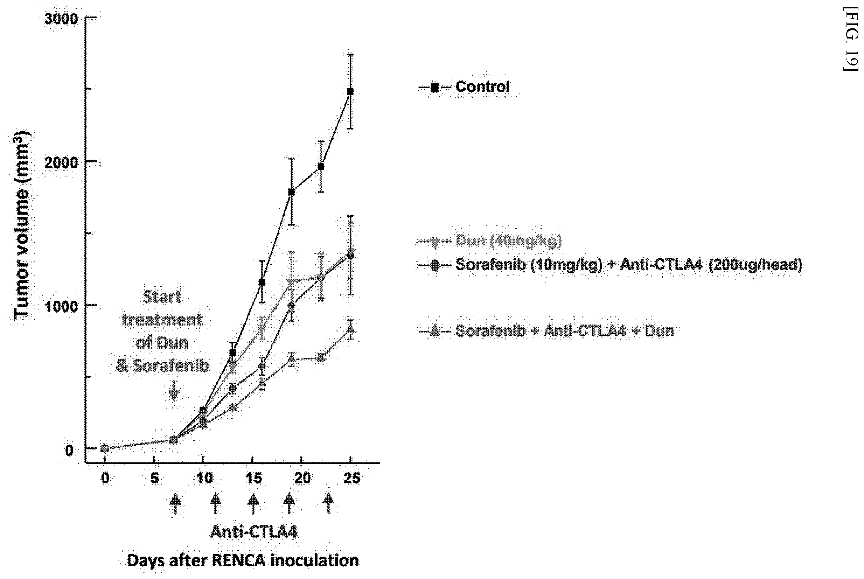

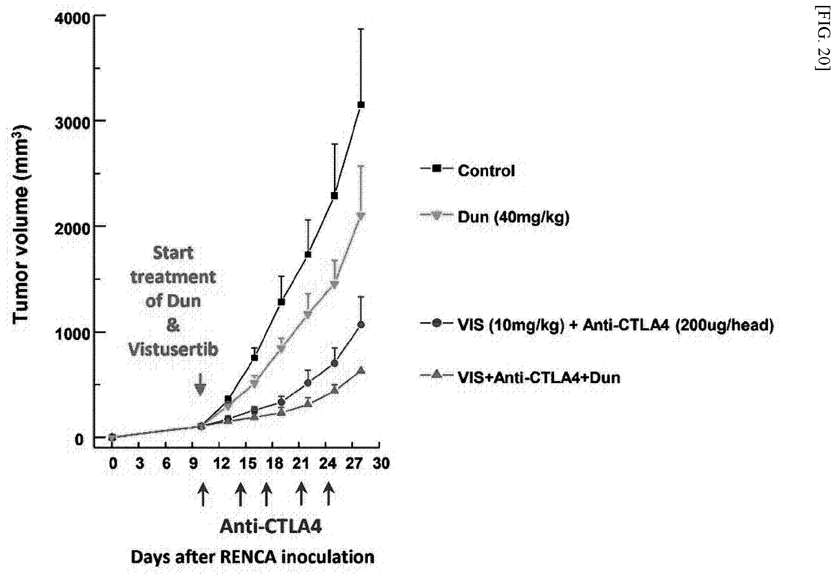

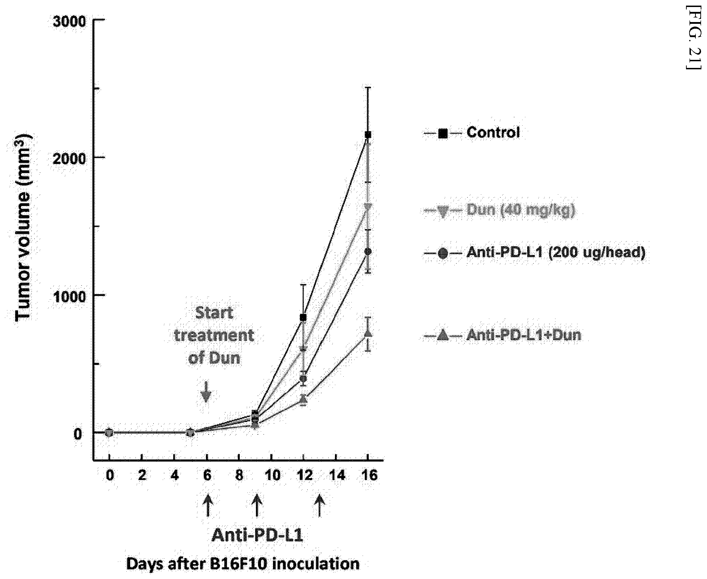

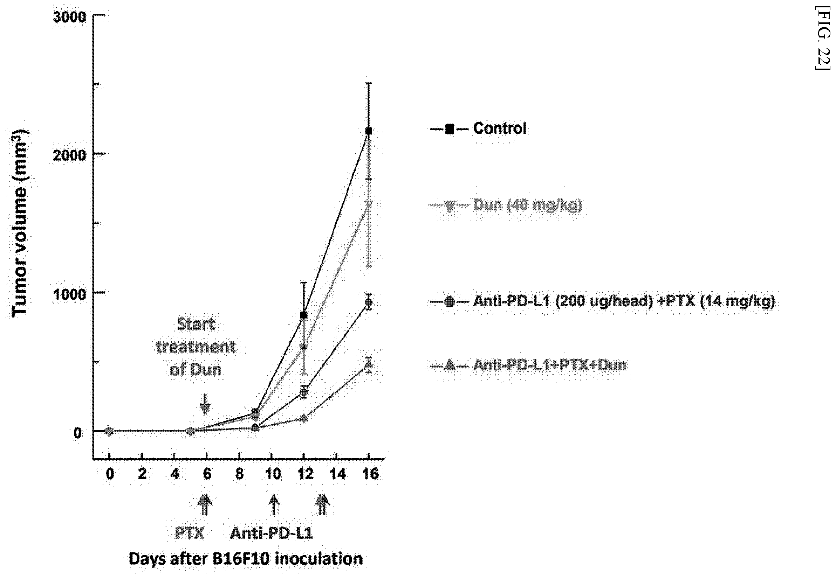

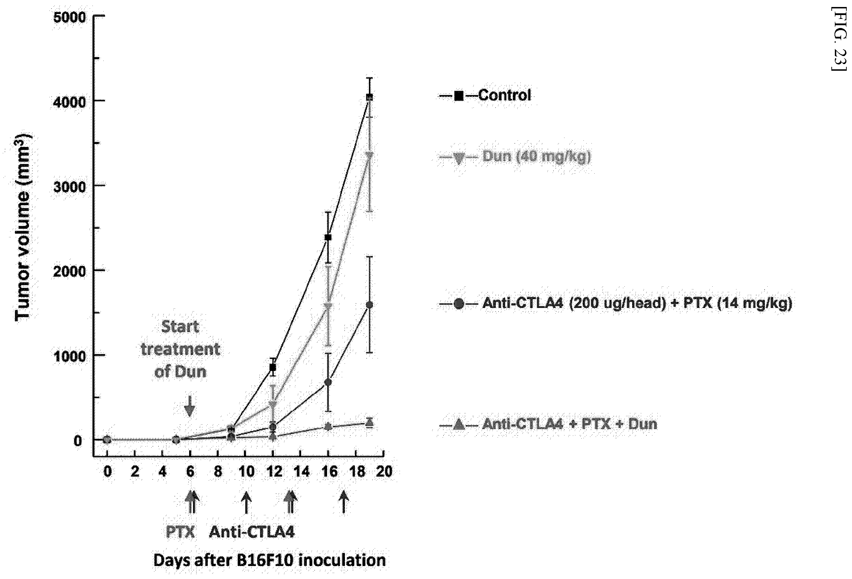

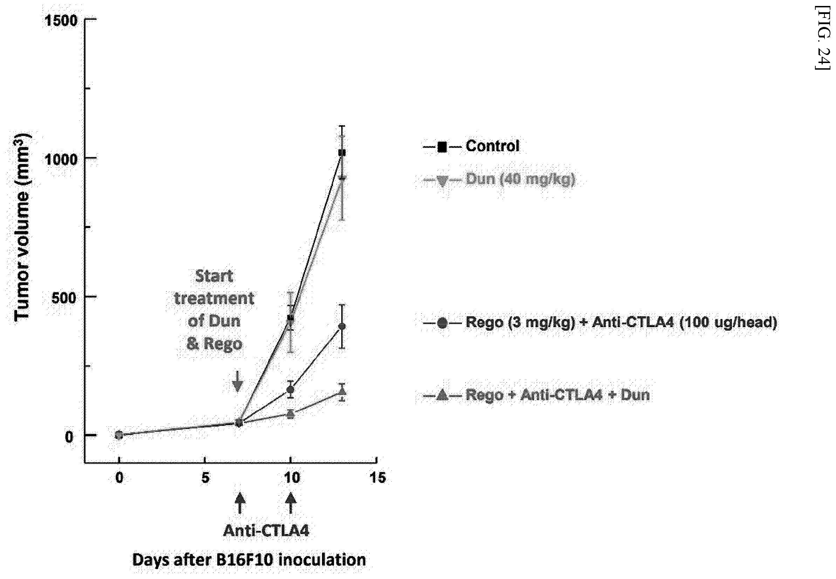

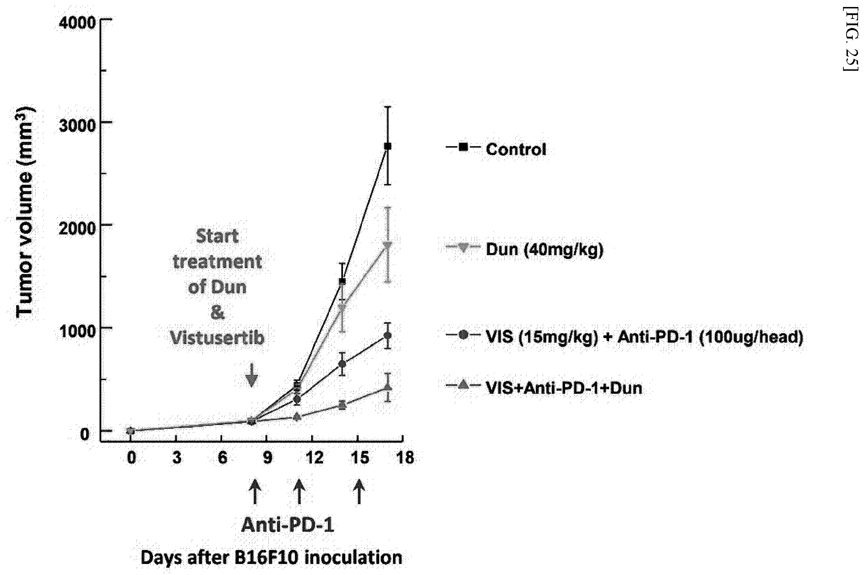

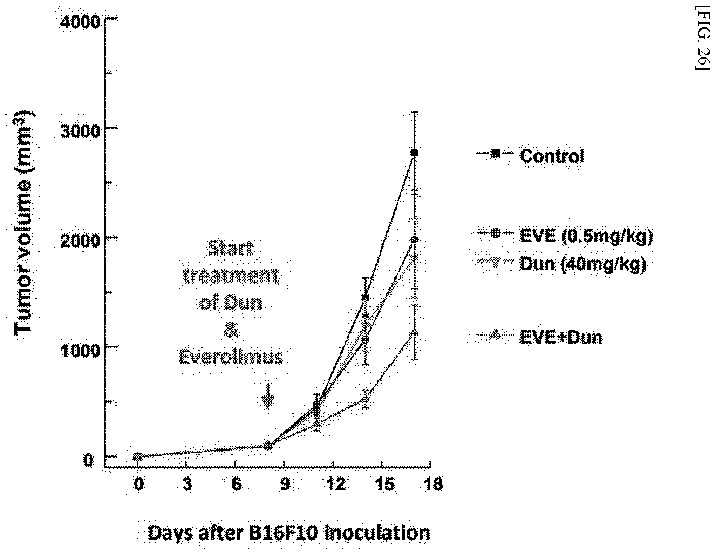

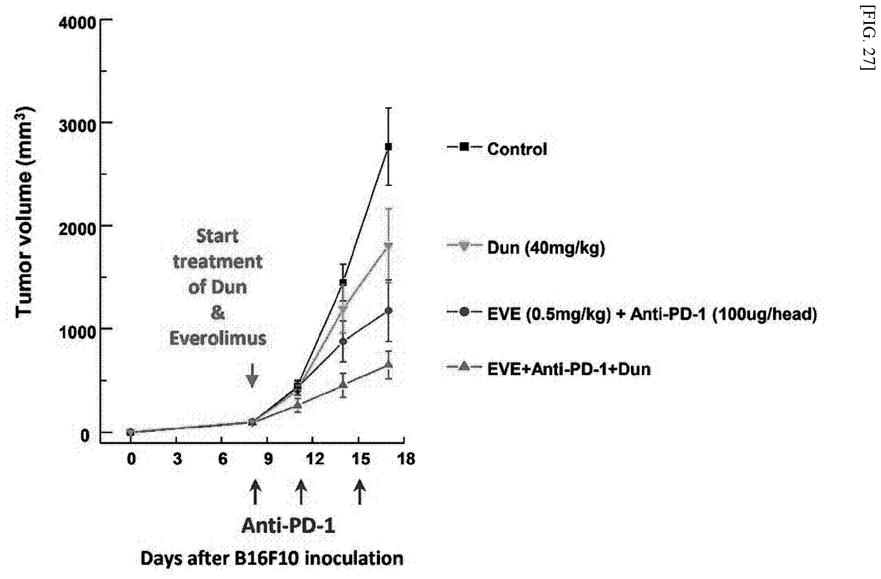

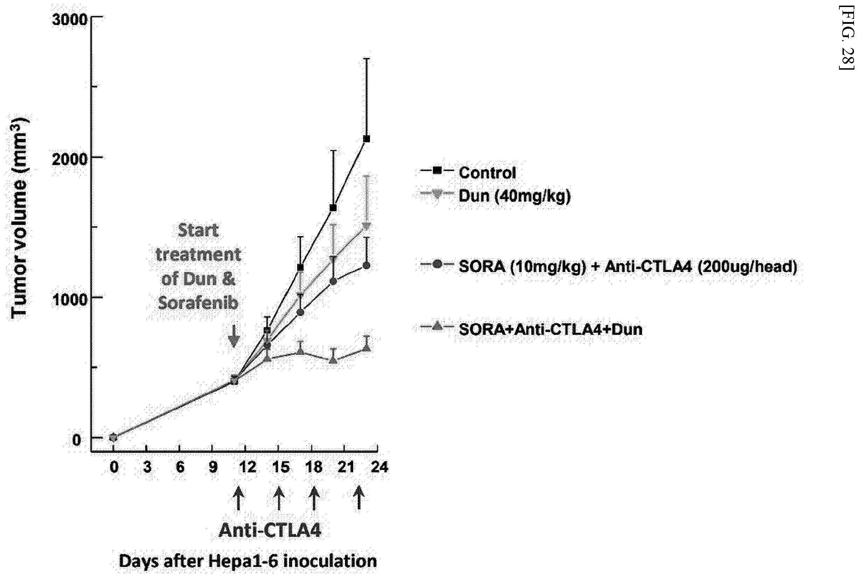

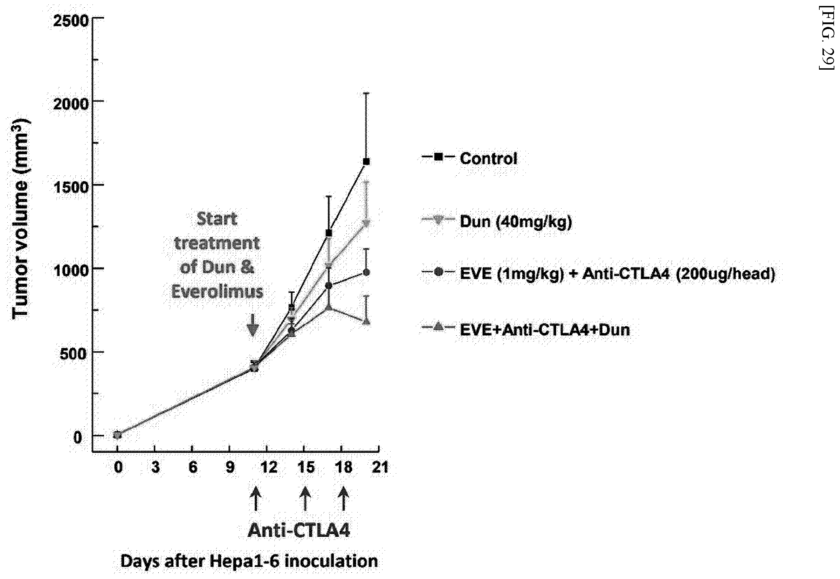

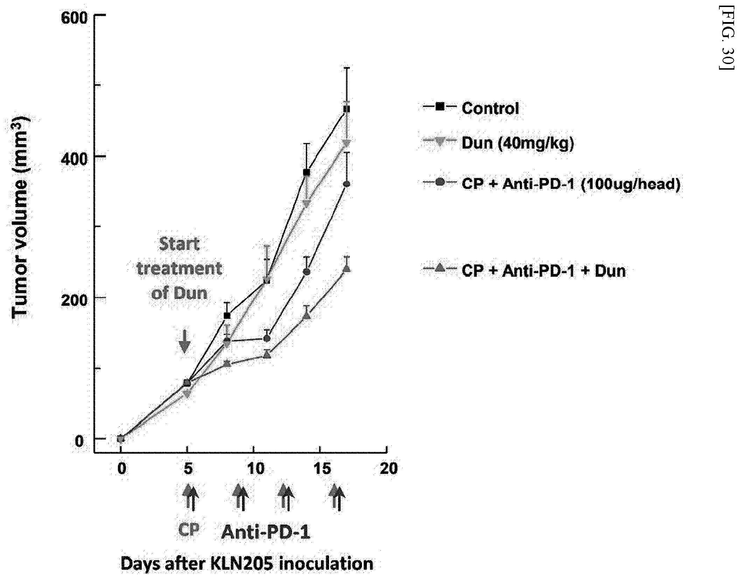

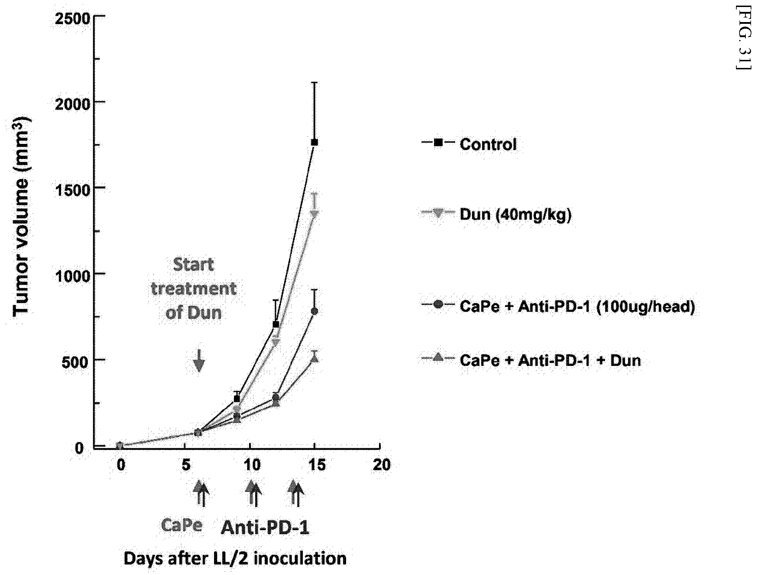

In addition, sole administration or co-administration of respective drugs was performed according to the administration schedules listed in Tables 4 to 34 below, the tumor volumes were then measured according to the tumor proliferation assessment method described in Example 1.1, the tumor growth inhibition rates (TGI (%) = (Vc - Vt)/Vc × 100 (%); Vc: tumor volume of control, Vt: tumor volume of experimental group) were calculated based the tumor volumes, and the results thereof are shown in the respective tables and FIGS. 1 to 31.

**[Table 4]**

| Experimental Example 1 | Animal model for colorectal cancer I | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (200 *µ*g/head) | Oxaliplatin (6 mg/kg) + 5-Fluorouracil (50 mg/kg) | |
| Administration cycle | Every day from 5th day of cancer cell implantation | At intervals of 3 days from 5th day, 4 times in total | Once on 5th day | |
| Administration route | Oral administration | Intraperitoneal administration | Intraperitoneal administration | |
| Experimental group | Administered drug | | | TGI (%) |
| 1 | ○ | - | - | 8.1 |
| 2 | - | ○ | - | 9.8 |
| 3 | ○ | ○ | - | 17.1 |
| 4 | - | - | ○ | 36.5 |
| 5 | ○ | - | ○ | 47.7 |
| 6 | - | ○ | ○ | 48.6 |
| 7 | ○ | ○ | ○ | 83.3 |
| Result | FIG. 1 | | | |

**[Table 5]**

| Experimental Example 2 | Animal model for colorectal cancer I | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Oxaliplatin (6 mg/kg) + 5-Fluorouracil (50 mg/kg) | |
| Administration cycle | Every day from 5th day of cancer cell implantation | At intervals of 3 days from 5th day, 4 times in total | Once on 5th day | |
| Administration route | Oral administration | Intraperitoneal administration | Intraperitoneal administration | |
| Experimental group | Administered drug | | | TGI (%) |
| 1 | - | ○ | - | 2.6 |
| 2 | ○ | - | - | 7.6 |
| 3 | ○ | ○ | - | 26.1 |
| 4 | - | - | ○ | 27.4 |
| 5 | ○ | - | ○ | 45.8 |
| 6 | - | ○ | ○ | 53.4 |
| 7 | ○ | ○ | ○ | 83.6 |
| Result | FIG. 2 | | | |

**[Table 6]**

| Experimental Example 3 | Animal model for colorectal cancer I | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (100 ug/head) + Anti-CTLA4 antibody (100 *µ*g/head) | Oxaliplatin (6 mg/kg) + 5-Fluorouracil (50 mg/kg) | |
| Administration cycle | Every day from 4th day of cancer cell implantation | At intervals of 3 days from 4th day, 4 times in total | Once on 4th day | |
| Administration route | Oral administration | Intraperitoneal administration | Intraperitoneal administration | |
| Experimental group | Administered drug | | | TGI (%) |
| 1 | ○ | - | - | 5.3 |
| 2 | - | - | ○ | 34.4 |
| 3 | - | ○ | - | 37.5 |
| 4 | ○ | ○ | - | 42.3 |
| 5 | ○ | - | ○ | 43.9 |
| 6 | - | ○ | ○ | 60.2 |
| 7 | ○ | ○ | ○ | 80.2 |
| Result | FIG. 3 | | | |

**[Table 7]**

| Experimental Example 4 | Animal model for colorectal cancer I | | | |
|---|---|---|---|---|
| | Naphthoquinone-based compound | Immune checkpoint inhibitor | Immunogenic cell death inducer | |
| Type (dosage) | Dunnione (40 mg/kg) | Anti-LAG3 antibody (200 *µ*g/head) | Oxaliplatin (6 mg/kg) | |
| Administration cycle | Every day from 7th day of cancer cell implantation | On 7th, 10th, 14th, and 17th days, 4 times in total | On 7th and 14th days, 2 times in total | |
| Administration route | Oral administration | Intraperitoneal administration | Intraperitoneal administration | |
| Experimental group | Administered drug | | | TGI (%) |
| 1 | - | - | ○ | 5.6 |
| 2 | ○ | - | - | 13.1 |
| 3 | - | ○ | ○ | 22.4 |
| 4 | ○ | - | ○ | 29.5 |
| 5 | - | ○ | - | 30.5 |
| 6 | ○ | ○ | - | 35 |
| 7 | ○ | ○ | ○ | 53.4 |
| Result | FIG. 4 | | | |

**[Table 8]**

| Experimental Example 5 | Animal model for colorectal cancer I | | |
|---|---|---|---|
| | Naphthoquinone-based compound | Immune checkpoint inhibitor | |
| Type (dosage) | Dunnione (40 mg/kg) | Anti-PD-1 antibody (200 *µ*g/head) | |
| Administration cycle | Every day from day of cancer cell implantation | At intervals of 3 days from 7th day, 4 times in total | |
| Administration route | Oral administration | Intraperitoneal administration | |
| Experimental group | Administered drug | | TGI (%) |
| 1 | ○ | - | 28.5 |
| 2 | - | ○ | 28.9 |
| 3 | ○ | ○ | 57.6 |
| Result | FIG. 5 | | |

**[Table 9]**

| Experimental Example 6 | Animal model for colorectal cancer I | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (100 *µ*g/head) | Regorafenib (10 mg/kg) | |
| Administration cycle | Every day from 7th day of cancer cell implantation | 2 times a week from 7th day, 4 times in total | Every day from 7th day | |
| Administration route | Oral administration | Intraperitoneal administration | Oral administration | |
| Experimental group | Administered drug | | | TGI (%) |
| 1 | ○ | - | - | 36.8 |
| 2 | - | ○ | ○ | 90.1 |
| 3 | ○ | ○ | ○ | 94.1 |
| Result | FIG. 6 | | | |

**[Table 10]**

| Experimental Example 7 | Animal model for colorectal cancer I | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (100 *µ*g/head) | Abemaciclib (75 mg/kg) | |
| Administration cycle | Every day from 6th day of cancer cell implantation | 2 times a week from 6th day, 2 times in total | Every day from 6th day | |
| Administration route | Oral administration | Intraperitoneal administration | Oral administration | |
| Experimental group | Administered drug | | | TGI (%) |
| 1 | ○ | - | - | 9.9 |
| 2 | - | ○ | ○ | 36.4 |
| 3 | ○ | ○ | ○ | 59.9 |
| Result | FIG. 7 | | | |

**[Table 11]**

| Experimental Example 8 | Animal model for colorectal cancer II | | |
|---|---|---|---|
| | Naphthoquinone-based compound | Immunogenic cell death inducer | |
| Type (dosage) | Dunnione (40 mg/kg) | Irinotecan (4.626 mg/kg) + Leucovorin (10.28 mg/kg) + 5-Fluorouracil (61.68 mg/kg) | |
| Administration cycle | Every day from 6th day of cancer cell implantation | 2 times a week from 6th day, 4 times in total | |
| Administration route | Oral administration | Intraperitoneal administration | |
| Experimental group | Administered drug | | TGI (%) |
| 1 | ○ | - | 21.2 |
| 2 | - | ○ | 42.9 |
| 3 | ○ | ○ | 66.3 |
| Result | FIG. 8 | | |

**[Table 12]**

| Experimental Example 9 | Animal model for colorectal cancer II | | |
|---|---|---|---|
| | Naphthoquinone-based compound | Immune checkpoint inhibitor | |
| Type (dosage) | Dunnione (40 mg/kg) | Anti-PD-1 antibody (200 *µ*g/head) | |
| Administration cycle | Every day from 6th day of cancer cell implantation | 2 times a week from 6th day, 4 times in total | |
| Administration route | Oral administration | Intraperitoneal administration | |
| Experimental group | Administered drug | | TGI (%) |
| 1 | - | ○ | 19.9 |
| 2 | ○ | - | 25.5 |
| 3 | ○ | ○ | 59.6 |
| Result | FIG. 9 | | |

**[Table 13]**

| Experimental Example 10 | Animal model for breast cancer I | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Mitoxantrone (6 mg/kg) | |
| Administration cycle | Every day from 5th day of cancer cell implantation | At intervals of 3 days from 5th day, 4 times in total | Once on 5th day | |
| Administration route | Oral administration | Intraperitoneal administration | Intraperitoneal administration | |
| Experimental group | Administered drug | | | TGI (%) |
| 1 | ○ | - | - | 8.9 |
| 2 | - | - | ○ | 16.3 |
| 3 | ○ | - | ○ | 23.8 |
| 4 | - | ○ | - | 29.6 |
| 5 | - | ○ | ○ | 35.5 |
| 6 | ○ | ○ | - | 40.5 |
| 7 | ○ | ○ | ○ | 66.9 |
| Result | FIG. 10 | | | |

**[Table 14]**

| Experimental Example 11 | Animal model for breast cancer I | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (200 *µ*g/head) | Adriamycin (6 mg/kg) | |
| Administration cycle | Every day from 14th day of cancer cell implantation | 2 times a week from 14th day, 4 times in total | Once on 14th day | |
| Administration route | Oral administration | Intraperitoneal administration | Intraperitoneal administration | |
| Experimental group | Administered drug | | | TGI (%) |
| 1 | ○ | ○ | - | 0.9 |
| 2 | - | ○ | - | 5.8 |
| 3 | - | ○ | ○ | 19.3 |
| 4 | ○ | ○ | ○ | 49 |
| Result | FIG. 11 | | | |

**[Table 15]**

| Experimental Example 12 | Animal model for breast cancer I | | |
|---|---|---|---|
| | Naphthoquinone-based compound | Immunogenic cell death inducer | |
| Type (dosage) | Dunnione (40 mg/kg) | Adriamycin (3.08 mg/kg) + Cyclophosphamide (30.8 mg/kg) + Paclitaxel (4.12 mg/kg) | |
| Administration cycle | Every day from 7th day of cancer cell implantation | At intervals of 1 week from 7th day, 3 times in total | |
| Administration route | Oral administration | Intraperitoneal administration | |
| Experimental group | Administered drug | | TGI (%) |
| 1 | ○ | - | 23.6 |
| 2 | - | ○ | 39.2 |
| 3 | ○ | ○ | 52.3 |
| Result | FIG. 12 | | |

**[Table 16]**

| Experimental Example 13 | Animal model for breast cancer I | | |
|---|---|---|---|
| | Naphthoquinone-based compound | Immunogenic cell death inducer | |
| Type (dosage) | Dunnione (40 mg/kg) | Docetaxel (4.06 mg/kg) + Adriamycin (2.7 mg/kg) + Cyclophosphamide (27 mg/kg) | |
| Administration cycle | Every day from 7th day of cancer cell implantation | At intervals of 1 week from 7th day, 4 times in total | |
| Administration route | Oral administration | Intraperitoneal administration | |
| Experimental group | Administered drug | | TGI (%) |
| 1 | ○ | - | 16.1 |
| 2 | - | ○ | 40.4 |
| 3 | ○ | ○ | 54.1 |
| Result | FIG. 13 | | |

**[Table 17]**

| Experimental Example 14 | Animal model for breast cancer II | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Lapatinib (100 mg/kg) | |
| Administration cycle | Every day from 11th day of cancer cell implantation | 2 times a week from 11th day, 2 times in total | Every day from 11th day | |
| Administration route | Oral administration | Intraperitoneal administration | Oral administration | |
| Experimental group | Administered drug | | | TGI (%) |
| 1 | ○ | - | - | 9.7 |
| 2 | - | ○ | ○ | 53.3 |
| 3 | ○ | ○ | ○ | 70.2 |
| Result | FIG. 14 | | | |

**[Table 18]**

| Experimental Example 15 | Animal model for renal cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Everolimus (250 *µ*g/kg) | |
| Administration cycle | Every day from 7th day of cancer cell implantation | At intervals of 3 days from 7th day, 7 times in total | Every day from 7th day | |
| Administration route | Oral administration | Intraperitoneal administration | Oral administration | |
| Experimental group | Administered drug | | | TGI (%) |
| 1 | ○ | - | - | 7.8 |
| 2 | - | ○ | - | 16.5 |
| 3 | ○ | ○ | - | 36.1 |
| 4 | - | - | ○ | 72.8 |
| 5 | ○ | - | ○ | 85 |
| 6 | - | ○ | ○ | 87 |
| 7 | ○ | ○ | ○ | 92 |
| Result | FIG. 15 | | | |

**[Table 19]**

| Experimental Example 16 | Animal model for renal cancer | | |
|---|---|---|---|
| | Naphthoquinone-based compound | Immunogenic cell death inducer | |
| Type (dosage) | Dunnione (40 mg/kg) | Sorafenib (10 mg/kg) | |
| Administration cycle | Every day from 7th day of cancer cell implantation | Every day from 7th day | |
| Administration route | Oral administration | Oral administration | |
| Experimental group | Administered drug | | TGI (%) |
| 1 | ○ | - | 14.5 |
| 2 | - | ○ | 29.4 |
| 3 | ○ | ○ | 53.8 |
| Result | FIG. 16 | | |

**[Table 20]**

| Experimental Example 17 | Animal model for renal cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Sorafenib (10 mg/kg) | |
| Administration cycle | Every day from 7th day of cancer cell implantation | At intervals of 3 days from 7th day, 3 times in total | Every day from 7th day | |
| Administration route | Oral administration | Intraperitoneal administration | Oral administration | |
| Experimental group | Administered drug | | | TGI (%) |
| 1 | ○ | - | - | 14.5 |
| 2 | - | ○ | ○ | 37.3 |
| 3 | ○ | ○ | ○ | 57.6 |
| Result | FIG. 17 | | | |

**[Table 21]**

| Experimental Example 18 | Animal model for renal cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (100 *µ*g/head) | Everolimus (0.1 mg/kg) | |
| Administration cycle | Every day from 7th day of cancer cell implantation | 2 times a week from 7th day, 5 times in total | Every day from 7th day | |
| Administration route | Oral administration | Intraperitoneal administration | Oral administration | |
| Experimental group | Administered drug | | | TGI (%) |
| 1 | ○ | - | - | 5.2 |
| 2 | - | ○ | ○ | 76.9 |
| 3 | ○ | ○ | ○ | 90.6 |
| Result | FIG. 18 | | | |

**[Table 22]**

| Experimental Example 19 | Animal model for renal cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Sorafenib (10 mg/kg) | |
| Administration cycle | Every day from 7th day of cancer cell implantation | 2 times a week from 7th day, 5 times in total | Every day from 7th day | |
| Administration route | Oral administration | Intraperitoneal administration | Oral administration | |
| Experimental group | Administered drug | | | TGI (%) |
| 1 | ○ | - | - | 44.6 |
| 2 | - | ○ | ○ | 45.9 |
| 3 | ○ | ○ | ○ | 66.7 |
| Result | FIG. 19 | | | |

**[Table 23]**

| Experimental Example 20 | Animal model for renal cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Vistusertib (10 mg/kg) | |
| Administration cycle | Every day from 10th day of cancer cell implantation | 2 times a week from 10th day, 5 times in total | Every day from 10th day | |
| Administration route | Oral administration | Intraperitoneal administration | Oral administration | |
| Experimental group | Administered drug | | | TGI (%) |
| 1 | ○ | - | - | 33.2 |
| 2 | - | ○ | ○ | 66.1 |
| 3 | ○ | ○ | ○ | 79.9 |
| Result | FIG. 20 | | | |

**[Table 24]**

| Experimental Example 21 | Animal model for skin cancer | | |
|---|---|---|---|
| | Naphthoquinone-based compound | Immune checkpoint inhibitor | |
| Type (dosage) | Dunnione (40 mg/kg) | Anti-PD-L1 antibody (200 *µ*g/head) | |
| Administration cycle | Every day from 6th day of cancer cell implantation | 2 times a week from 6th day, 3 times in total | |
| Administration route | Oral administration | Intraperitoneal administration | |
| Experimental group | Administered drug | | TGI (%) |
| 1 | ○ | - | 24.1 |
| 2 | - | ○ | 39.2 |
| 3 | ○ | ○ | 67 |
| Result | FIG. 21 | | |

**[Table 25]**

| Experimental Example 22 | Animal model for skin cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-L1 antibody (200 *µ*g/head) | Paclitaxel (14 mg/kg) | |
| Administration cycle | Every day from 6th day of cancer cell implantation | 2 times a week from 6th day, 3 times in total | At intervals of 1 week from 6th day, 2 times in total | |
| Administration route | Oral administration | Intraperitoneal administration | Intraperitoneal administration | |
| Experimental group | Administered drug | | | TGI (%) |
| 1 | ○ | - | - | 24.1 |
| 2 | - | ○ | ○ | 57 |
| 3 | ○ | ○ | ○ | 78 |
| Result | FIG. 22 | | | |

**[Table 26]**

| Experimental Example 23 | Animal model for skin cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Paclitaxel (14 mg/kg) | |
| Administration cycle | Every day from 6th day of cancer cell implantation | 2 times a week from 6th day, 4 times in total | Once on 6th day | |
| Administration route | Oral administration | Intraperitoneal administration | Intraperitoneal administration | |
| Experimental group | Administered drug | | | TGI (%) |
| 1 | ○ | - | - | 16.7 |
| 2 | - | ○ | ○ | 60.5 |
| 3 | ○ | ○ | ○ | 95.1 |
| Result | FIG. 23 | | | |

**[Table 27]**

| Experimental Example 24 | Animal model for skin cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (100 *µ*g/head) | Regorafenib (3 mg/kg) | |
| Administration cycle | Every day from 7th day of cancer cell implantation | 2 times a week from 7th day, 2 times in total | Every day from 7th day | |
| Administration route | Oral administration | Intraperitoneal administration | Oral administration | |
| Experimental group | Administered drug | | | TGI (%) |
| 1 | ○ | - | - | 9.2 |
| 2 | - | ○ | ○ | 61.5 |
| 3 | ○ | ○ | ○ | 84.8 |
| Result | FIG. 24 | | | |

**[Table 28]**

| Experimental Example 25 | Animal model for skin cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (100 *µ*g/head) | Vistusertib (15 mg/kg) | |
| Administration cycle | Every day from 8th day of cancer cell implantation | 2 times a week from 8th day, 3 times in total | Every day from 8th day | |
| Administration route | Oral administration | Intraperitoneal administration | Oral administration | |
| Experimental group | Administered drug | | | TGI (%) |
| 1 | ○ | - | - | 34.6 |
| 2 | - | ○ | ○ | 66.5 |
| 3 | ○ | ○ | ○ | 84.7 |
| Result | FIG. 25 | | | |

**[Table 29]**

| Experimental Example 26 | Animal model for skin cancer | | |
|---|---|---|---|
| | Naphthoquinone-based compound | Immunogenic cell death inducer | |
| Type (dosage) | Dunnione (40 mg/kg) | Everolimus (0.5 mg/kg) | |
| Administration cycle | Every day from 8th day of cancer cell implantation | Every day from 8th day | |
| Administration route | Oral administration | Oral administration | |
| Experimental group | Administered drug | | TGI (%) |
| 1 | - | ○ | 28.5 |
| 2 | ○ | - | 31.5 |
| 3 | ○ | ○ | 59.0 |
| Result | FIG. 26 | | |

**[Table 30]**

| Experimental Example 27 | Animal model for skin cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (100 *µ*g/head) | Everolimus (0.5 mg/kg) | |
| Administration cycle | Every day from 8th day of cancer cell implantation | 2 times a week from 8th day, 3 times in total | Every day from 8th day | |
| Administration route | Oral administration | Intraperitoneal administration | Oral administration | |
| Experimental group | Administered drug | | | TGI (%) |
| 1 | ○ | - | - | 34.6 |
| 2 | - | ○ | ○ | 57.4 |
| 3 | ○ | ○ | ○ | 76.4 |
| Result | FIG. 27 | | | |

**[Table 31]**

| Experimental Example 28 | Animal model for liver cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Sorafenib (10 mg/kg) | |
| Administration cycle | Every day from 11th day of cancer cell implantation | 2 times a week from 11th day, 4 times in total | Every day from 11th day | |
| Administration route | Oral administration | Intraperitoneal administration | Oral administration | |
| Experimental group | Administered drug | | | TGI (%) |
| 1 | ○ | - | - | 29.1 |
| 2 | - | ○ | ○ | 42.3 |
| 3 | ○ | ○ | ○ | 70.2 |
| Result | FIG. 28 | | | |

**[Table 32]**

| Experimental Example 29 | Animal model for liver cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Everolimus (1 mg/kg) | |
| Administration cycle | Every day from 11th day of cancer cell implantation | 2 times a week from 11th day, 3 times in total | Every day from 11th day | |
| Administration route | Oral administration | Intraperitoneal administration | Oral administration | |
| Experimental group | Administered drug | | | TGI (%) |
| 1 | ○ | - | - | 22.4 |
| 2 | - | ○ | ○ | 40.5 |
| 3 | ○ | ○ | ○ | 58.6 |
| Result | FIG. 29 | | | |

**[Table 33]**

| Experimental Example 30 | Animal model for lung cancer I | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (100 *µ*g/head) | Carboplatin (25 mg/kg) + Paclitaxel (10 mg/kg) | |
| Administration cycle | Every day from 5th day of cancer cell implantation | 2 times a week from 5th day, 4 times in total | 2 times a week from 5th day, 4 times in total | |
| Administration route | Oral administration | Intraperitoneal administration | Intraperitoneal administration | |
| Experimental group | Administered drug | | | TGI (%) |
| 1 | ○ | - | - | 10.2 |
| 2 | - | ○ | ○ | 22.7 |
| 3 | ○ | ○ | ○ | 48.6 |
| Result | FIG. 30 | | | |

**[Table 34]**

| Experimental Example 31 | Animal model for lung cancer II | | | |
|---|---|---|---|---|
| | Naphthoquinone-based compound | Immune checkpoint inhibitor | Immunogenic cell death inducer | |
| Type (dosage) | Dunnione (40 mg/kg) | Anti-PD-1 antibody (100 *µ*g/head) | Carboplatin (25 mg/kg) + Pemetrexed (100 mg/kg) | |
| Administration cycle | Every day from 6th day of cancer cell implantation | 2 times a week from 6th day, 3 times in total | 2 times a week from 6th day, 3 times in total | |
| Administration route | Oral administration | Intraperitoneal administration | Intraperitoneal administration | |
| Experimental group | Administered drug | | | TGI (%) |
| 1 | ○ | - | - | 23.5 |
| 2 | - | ○ | ○ | 55.8 |
| 3 | ○ | ○ | ○ | 71.5 |
| Result | FIG. 31 | | | |

With reference to Tables 4 to 34 and FIGS. 1 to 31, it was confirmed that when the naphthoquinone-based compound was administered in combination with the immune checkpoint inhibitor and/or the immunogenic cell death inducer, the effects inhibiting the cancer cell proliferation were significantly superior compared to the case where each substance was administered alone.

### Example 2. Analysis of tumor metastasis

### Example 2.1. Preparation of tumor metastasis animal model and tumor metastasis analysis method

4T1-Luc (2.5 × 10⁶ cells) as a mouse breast cancer cell line overexpressing a luciferase was implanted into mammary fat pads of BALB/c mice, and the tumor was removed through surgical resection when the size of the cancer reached 150 to 200 mm³. The administration of drugs was started from the day after the tumor removal procedure, and the degree of cancer metastasis was measured by measuring the photon emission amount at intervals of 1 week from the 3rd day after the tumor removal procedure.

### Example 2.2. Assessment of tumor metastasis according to sole administration and co-administration of respective drugs

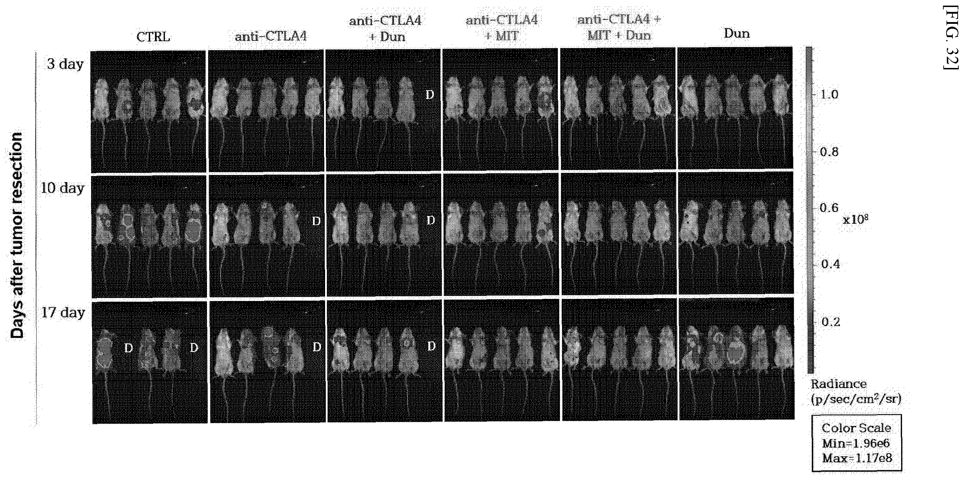

After sole administration or co-administration of respective drugs was performed according to the administration schedule listed in Table 35 below, the photon emission amount (fold) was measured on the 17th day according to the tumor metastasis assessment method described in Example 2.1, and the results thereof are shown in Table 35 and FIG. 32.

**[Table 35]**

| Experimental Example 32 | Animal model for breast cancer | | | |
|---|---|---|---|---|
| | Naphthoquinone-based compound | Immune checkpoint inhibitor | Immunogenic cell death inducer | |
| Type (dosage) | Dunnione (40 mg/kg) | Anti-CTLA4 antibody (200 *µ*g/head) | Mitoxantrone (6 mg/kg) | |
| Administration cycle | Every day from 1 st day after primary cancer removal | At intervals of 3 days from 1st day | Once on 1 st day | |
| Administration route | Oral administration | Intraperitoneal administration | Intraperitoneal administration | |
| Experimental group | Administered drug | | | Photon emission amount |
| 1 | - | - | - | 3028.2 ± 2961 |
| 2 | - | ○ | - | 1615.3 ± 1514 |
| 3 | ○ | ○ | - | 94.1 ± 66.8 |
| 4 | - | ○ | ○ | 11.1 ± 6.6 |
| 5 | ○ | ○ | ○ | 1.7 ± 0.7 |
| 6 | ○ | - | - | 2805.3 ± 2352.3 |
| Result | FIG. 32 | | | |

With reference to Table 35 and FIG. 32, it was confirmed that when the naphthoquinone-based compound was administered in combination with the immune checkpoint inhibitor or when the naphthoquinone-based compound and the immunogenic cell death inducer as well as the immune checkpoint inhibitor, that is, the three substances were co-administered, the cancer cell metastasis was significantly reduced compared to the case where each substance was administered alone.

### Example 3. Analysis of Survival rate

### Example 3.1. Survival rate analysis method

After subcutaneous implantation of the cancer cell line, the survival rate was measured by recording the number of mice that died in each group every day. The measurement of survival rate was performed until the death of all mice in all groups or the death of all mice in the control. The average survival day was calculated by obtaining a survival curve using the survival rate.

### Example 3.2. Analysis of survival rate according to sole administration and co-administration of respective drugs

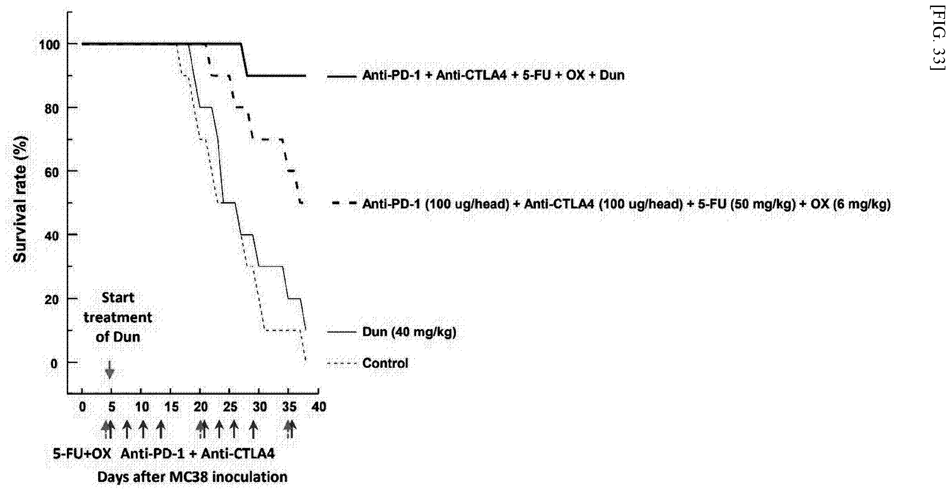

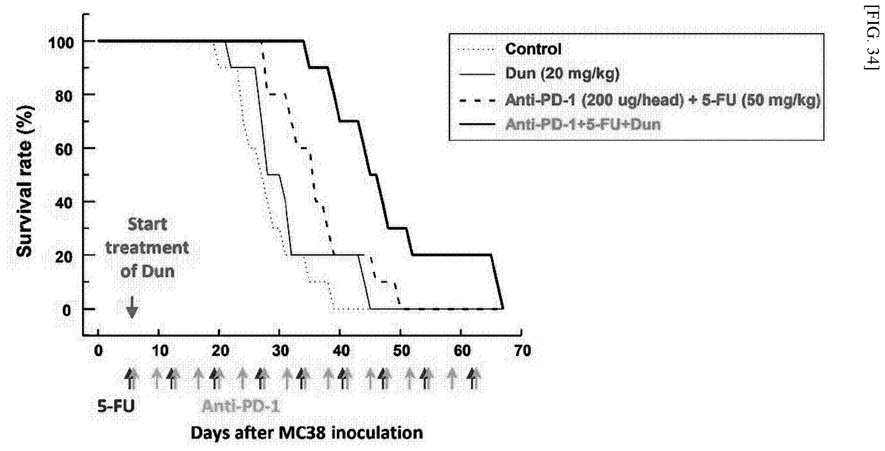

After sole administration or co-administration of respective drugs was performed according to the administration schedules listed in Tables 36 and 37 below, the survival rates and the average survival days were calculated according to the survival rate analysis method described in Example 3.1, and the results thereof are respectively shown in Tables 36 and 37 and FIGS. 33 and 34.

**[Table 36]**

| Experimental Example 33 | Animal model for colorectal cancer I | | | |
|---|---|---|---|---|
| | Naphthoquinone-based compound | Immune checkpoint inhibitor | Immunogenic cell death inducer | Survival rate (%) on 38th day after cancer implantation |
| Type (dosage) | Dunnione (40 mg/kg) | Anti-PD-1 antibody (100 *µ*g/head) + Anti-CTLA4 antibody (100 *µ*g/head) | Oxaliplatin (6 mg/kg) + 5-Fluorouracil (50 mg/kg) | |
| Administration cycle | Every day from 4th day of cancer cell implantation | On 4th, 7th, 10th, 13th, 19th, 22nd, 25th, 28th, and 34th days (2 cycles + once) | At intervals of 15 days from 4th day, 3 times in total | |
| Administration route | Oral administration | Intraperitoneal administration | Intraperitoneal administration | |
| Experimental group | Administered drug | | | |
| 1 | - | - | - | 0 |
| 2 | ○ | - | - | 10 |
| 3 | - | ○ | ○ | 50 |
| 4 | ○ | ○ | ○ | 90 |
| Result | FIG. 33 | | | |

**[Table 37]**

| Experimental Example 34 | Animal model for colorectal cancer I | | | | |
|---|---|---|---|---|---|
| | Naphthoquinone-based compound | Immune checkpoint inhibitor | Immunogenic cell death inducer | | |
| Type (dosage) | Dunnione (40 mg/kg) | Anti-PD-1 antibody (200 *µ*g/head) | 5-Fluorouracil (50 mg/kg) | | |
| Administration cycle | Every day from 6th day of cancer cell implantation | 2 times a week from 6th day to 62nd day | Once on 6th day | | |
| Administration route | Oral administration | Intraperitoneal administration | Intraperitoneal administration | | |
| Experimental group | Administered drug | | | Average survival day | Total survival period |
| 1 | - | - | - | 27 | 20 to 39 |
| 2 | ○ | - | - | 28 | 22 to 45 |
| 3 | - | ○ | ○ | 36 | 28 to 50 |
| 4 | ○ | ○ | ○ | 45 | 35 to 67 |
| Result | FIG. 34 | | | | |

With reference to Tables 36 and 37 and FIGS. 33 and 34, it may be seen that when the naphthoquinone-based compound is administered alone, the effect of increasing the survival rate of mice is insignificant, whereas when the naphthoquinone-based compound is administered in combination with the immune checkpoint inhibitor and the immunogenic cell death inducer, the survival rate and the average number of survival days of mice are significantly increased.

### Example 4. Tumor proliferation assessment using genetically engineered mouse models (GEMMs)

### Example 4.1. Construction of models for spontaneous non-small cell lung cancer, and drug administration

Models for spontaneous non-small cell lung cancer were induced by constructing conditional mutant mice (Kras^{LSL-G12D/+}; Trp53 ^{flox/flox} mice) having Ras activation and p53 variation through crossbreeding of conditional K-Ras activation variation-genetically engineered mice and conditional p53 variation-genetically engineered mice, and then performing intratracheal instillation of adenoviruses (Ad5-CMV-Cre; 2.5 × 10⁷ PFU/infectious units), into which Cre genes were introduced, into the lung. 12 weeks after induction of Cre protein expression using an adenoviral vector, the generation of cancer in the lungs of the mice were checked through Micro-CT imaging, test groups were set to a control, a dunnione (40 mg/kg) administration group, a carboplatin (25 mg/kg) + paclitaxel (10 mg/kg) administration group, a carboplatin (25 mg/kg) + paclitaxel (10 mg/kg) + dunnione (40 mg/kg) administration group, an anti-PD-1 antibody (200 *µ*g/head) administration group, an anti-PD-1 antibody (200 *µ*g/head) + dunnione (40 mg/kg) administration group, a carboplatin (25 mg/kg) + paclitaxel (10 mg/kg) + anti-PD-1 antibody (200 *µ*g/head) administration group, and a carboplatin (25 mg/kg) + paclitaxel (10 mg/kg) + anti-PD-1 antibody (200 *µ*g/head) + dunnione (40 mg/kg) administration group, and then administration of the drugs was started (see Table 38).

### Example 4.2. Analysis of tumor proliferation according to sole administration and co-administration of respective drugs

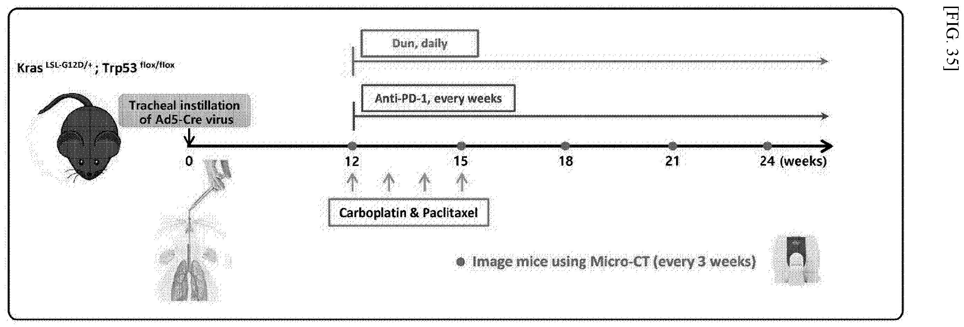

After sole administration or co-administration of respective drugs was performed according to the administration schedule listed in Table 38 below and FIG. 35, the tumor growth rate for each group compared to before the drug administration was measured at the 6th week through Micro-CT imaging, and the results thereof were summarized in Table 39.

**[Table 38]**

| Experimental Example 35 | Animal model for lung cancer | | |
|---|---|---|---|
| | Naphthoquinone-based compound | Immune checkpoint inhibitor | Immunogenic cell death inducer |
| Type (dosage) | Dunnione (40 mg/kg) | Anti-PD-1 antibody (200 *µ*g/head) | Carboplatin (25 mg/kg) + Paclitaxel (10 mg/kg) |
| Administration cycle | Every day | Every week | Every week (4 cycles) |
| Administration route | Oral administration | Intraperitoneal administration | Intraperitoneal administration |
| Experimental group | Administered drug | | |
| 1 | ○ | - | - |
| 2 | - | - | ○ |
| 3 | - | ○ | - |
| 4 | - | ○ | ○ |
| 5 | ○ | ○ | ○ |

**[Table 39]**

| Group | Tumor growth rate (fold) at 6th week |
|---|---|
| Control | 244.2 ± 374.1 |
| Dunnione (40 mg/kg) | 34.6 ± 54 |
| Carboplatin (25 mg/kg) + paclitaxel (10 mg/kg) | 63 ± 98.7 |
| Carboplatin (25 mg/kg) + paclitaxel (10 mg/kg) + dunnione (40 mg/kg) | 28.1 ± 23.2 |
| Anti-PD-1 antibody (200 *µ*g/head) | 80.1 ± 82.4 |
| Anti-PD-1 antibody (200 *µ*g/head) + dunnione (40 mg/kg) | 21 ± 14.4 |
| Carboplatin (25 mg/kg) + paclitaxel (10 mg/kg) + anti-PD-1 antibody (200 *µ*g/head) | 8.7 ± 4.3 |
| Carboplatin (25 mg/kg) + paclitaxel (10 mg/kg) + anti-PD-1 antibody (200 *µ*g/head) + dunnione (40 mg/kg) | 2.5 ± 2 |

### Example 5. Analysis of immune-related adverse events (irAEs) according to administration of anticancer agent

### Example 5.1. Analysis of adverse reactions according to administration of immune checkpoint inhibitor

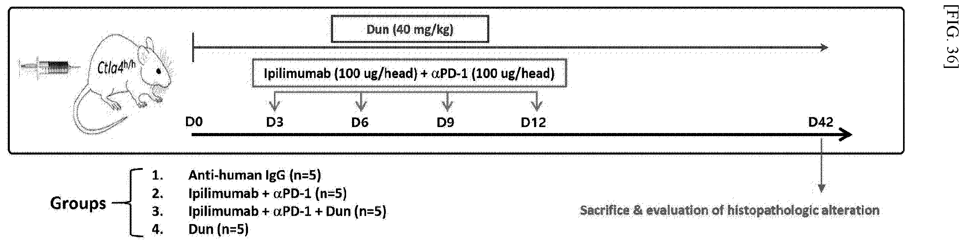

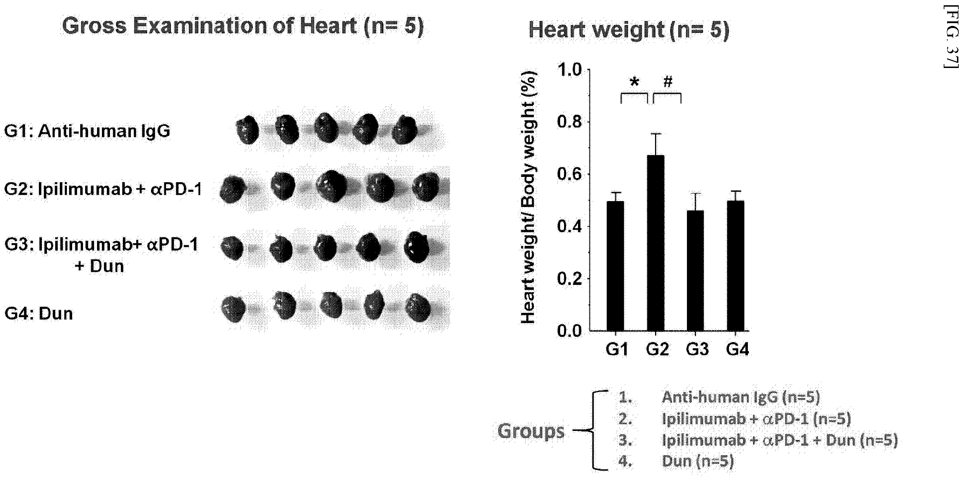

The immune checkpoint inhibitor is known to cause mild adverse reactions compared to existing cytotoxic anticancer agents, but may infrequently cause fatal or permanent functional impairment. Accordingly, sole administration or co-administration of respective drugs was performed according to the administration schedule listed in Table 40 below and FIG. 36, adverse reactions for each organ were then analyzed, and the results thereof are respectively shown in FIGS. 37 to 40.

**[Table 40]**

| Experimental | CTLA4 knock-in mouse animal model (female, 8 weeks old) | | |
|---|---|---|---|
| Example 36 | Naphthoquinone-based compound | Immune checkpoint inhibitor | |
| Type (dosage) | Dunnione (40 mg/kg) | Anti-PD-1 antibody (100 *µ*g/head) | Anti-CTLA4 antibody; Ipilimumab (100 *µ*g/head) |
| Administration cycle | Every day | At intervals of 3 days from 3rd day of dunnione administration, 4 times in total | |
| Administration route | Oral administration | Intraperitoneal administration | Intraperitoneal administration |
| Experimental group | Administered drug | | |
| 1 (Control; antihuman IgG) | - | - | - |
| 2 | - | ○ | ○ |
| 3 | ○ | ○ | ○ |

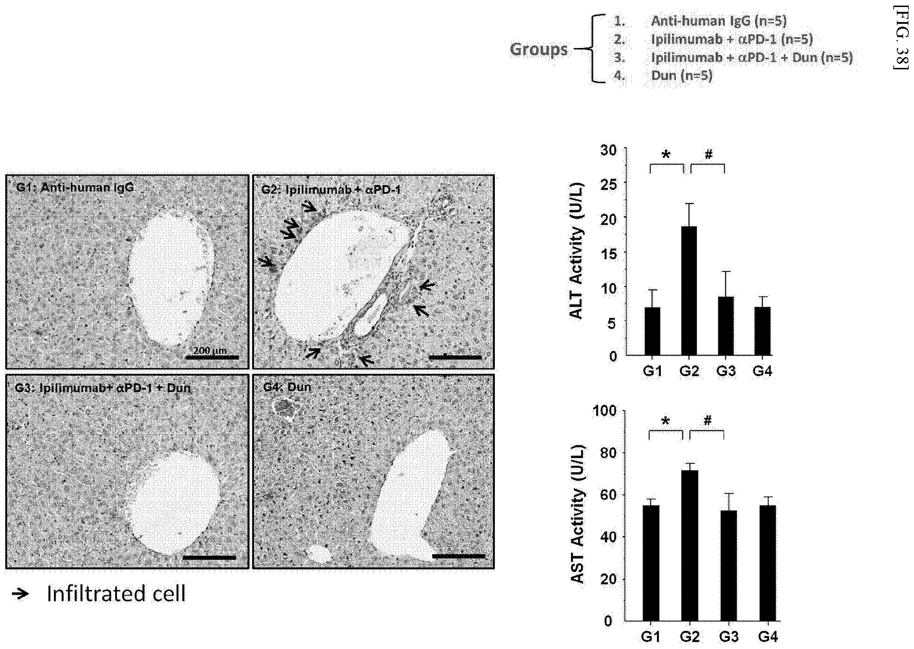

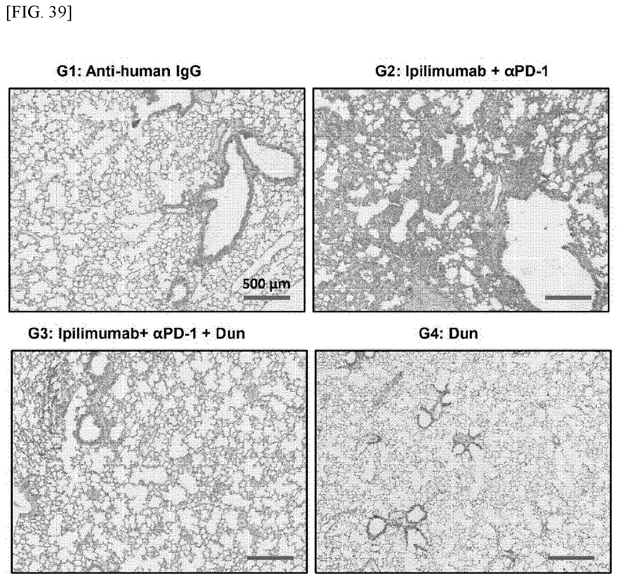

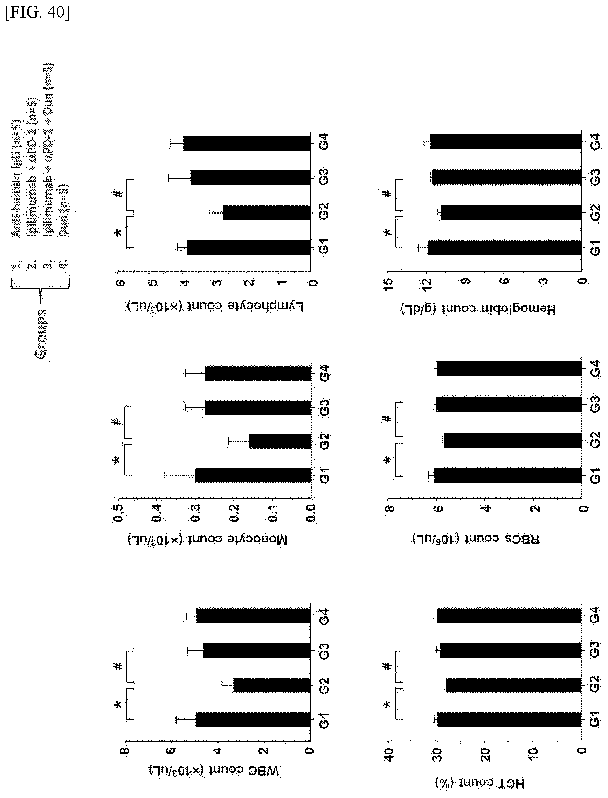

With reference to FIGS. 37 to 40, it may be seen that in the cases of cardiac hypertrophy (FIG. 37), hepatitis (FIG. 38), pneumonia (FIG. 39), and pancytopenia (FIG. 40) corresponding to adverse reactions induced by the administration of the immune checkpoint inhibitor, the incidence of such adverse reactions is significantly reduced by sole administration or co-administration of the naphthoquinone-based compound.

### Example 5.2. Analysis of adverse reactions according to administration of immune checkpoint inhibitor and immunogenic cell death inducer

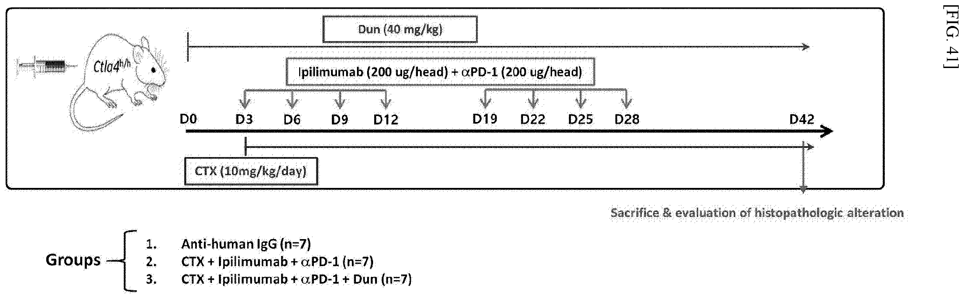

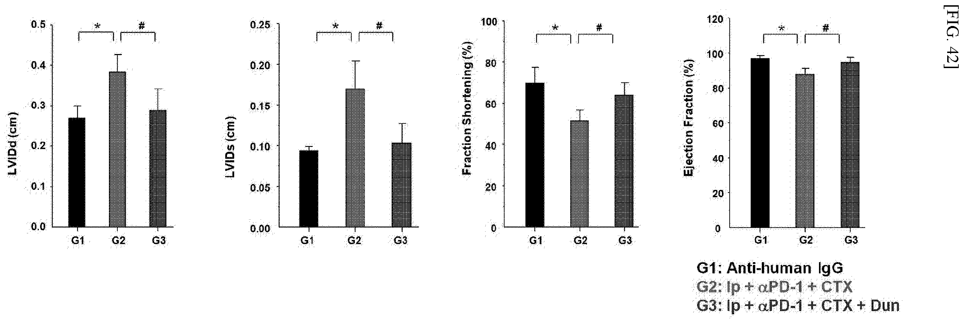

Adverse reactions for each organ by administration of immune checkpoint inhibitors and existing chemical anticancer agents, which accompany various immune-related adverse reactions, were analyzed. After sole administration or co-administration of respective drugs was performed according to the administration schedule listed in Table 41 below and FIG. 41, adverse reactions for each organ were analyzed, and the results thereof are respectively shown in FIGS. 42 to 46. Here, for the existing chemical anticancer agents, in order to minimize the side effects, metronomic anticancer therapy (metronomic treatment), in which low-dose anticancer agents were regularly administered, was performed.

**[Table 41]**

| Experimental Example 37 | CTLA4 knock-in mouse animal model (female, 8 weeks old) | | |
|---|---|---|---|
| | Naphthoquinone-based compound | Immune checkpoint inhibitor | Immunogenic cell death inducer |
| Type (dosage) | Dunnione (40 mg/kg) | Anti-PD-1 antibody (200 *µ*g/head) + Anti-CTLA4 antibody; Ipilimumab (200 *µ*g/head) | Cyclophosphamide (10 mg/kg) |
| Administration cycle | Every day | On 3rd, 6th, 9th, 12th, 19th, 22nd, 25th, and 28th days | Every day from 3rd day of dunnione administration |
| Administration route | Oral administration | Intraperitoneal administration | Intraperitoneal administration |
| Experimental group | Administered drug | | |
| 1 (Control; antihuman IgG) | - | - | - |
| 2 | - | ○ | ○ |
| 3 | ○ | ○ | ○ |

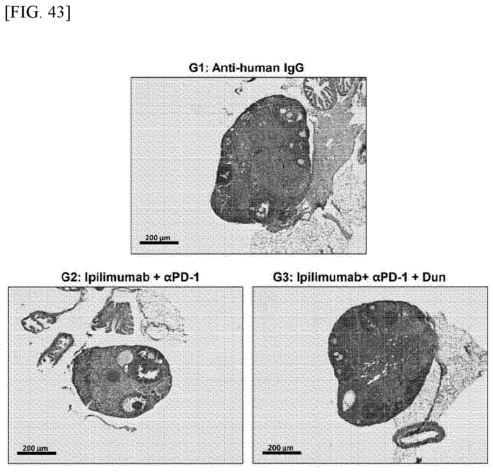

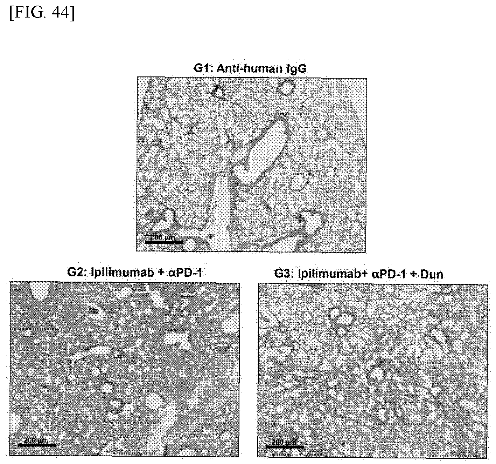

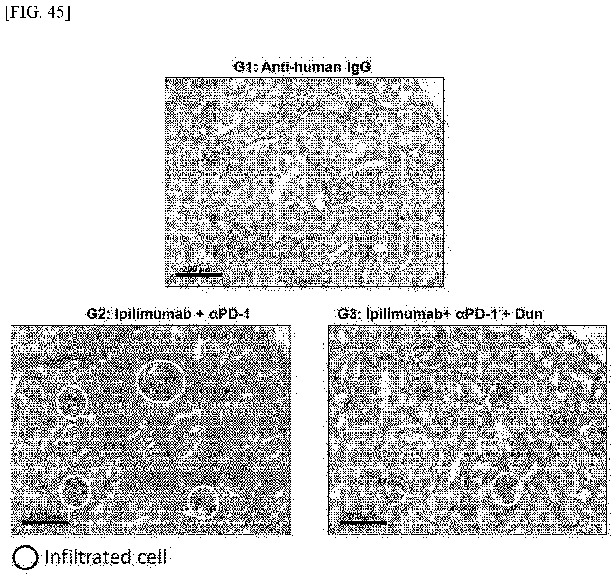

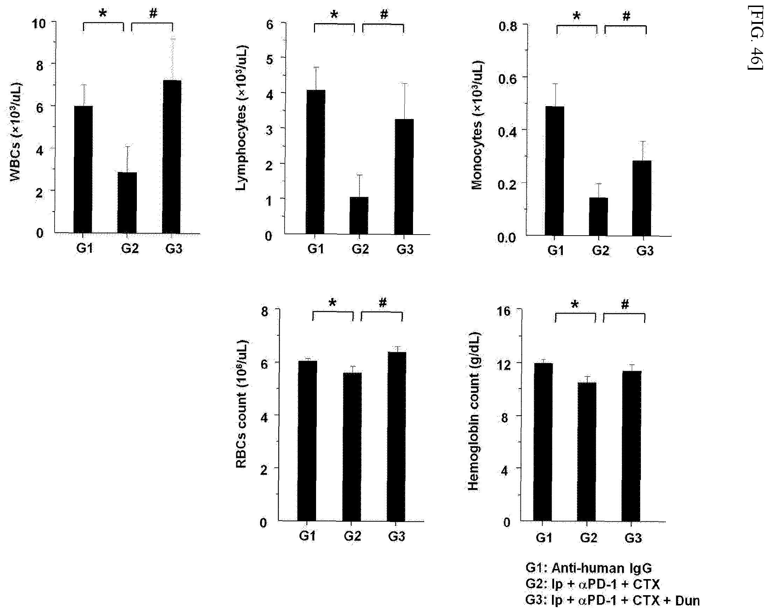

With reference to FIGS. 42 to 46, it may be seen that in the cases of heart failure (FIG. 42), a decrease in ovary size (FIG. 43), pneumonia (FIG. 44), nephritis (FIG. 45), and pancytopenia (FIG. 46) corresponding to adverse reactions induced by the administration of the immune checkpoint inhibitor and the immunogenic cell death inducer, the incidence of such adverse reactions is significantly reduced by co-administration of the naphthoquinone-based compound.

### Example 6. Analysis of immunogenic cell death of tumor cells

### Example 6.1. Immunogenic cell death analysis method

Calreticulin is one of the most important molecules in the immunogenic cell death process. When the calreticulin appears on membranes of tumor cell, a strong signal for phagocytotic cells including dendritic cells may be generated to cause the phagocytotic cells to attack dying tumor cells. This is the most significant "eat me" signal for immune cells. Whether or not immunogenic cell death was induced by the anticancer agents used in the present invention was assessed through the quantitative change of calreticulin expressed on the surface of cancer cells after the treatment with the anticancer agents. Single cells isolated from the tumor were incubated with anti-calreticulin antibody (ratio of 1:50) as a primary antibody at 4°C for 30 minutes, and then washed 2 times with PBS. After the washing, the cells were stained with Alexa Fluor 488 anti-goat IgG antibody (ratio of 1:500) as a secondary antibody, and then fixed with a fixation solution, and the level of calreticulin (ecto-CRT) expressed on the surfaces of the cells was observed using a flow cytometer. The case where the expression of calreticulin was increased by 20% or greater compared to the control was considered that immunogenic cell death was induced.

**[Table 42]**

| Tumor model | Cell line | Treatment drug | Expression rate (%) of calreticulin on cell surface |
|---|---|---|---|
| Lung cancer | LL/2 | Carboplatin + Paclitaxel | 247 |
| | | Pemetrexed + Carboplatin | 122 |
| | KLN205 | Carboplatin + Paclitaxel | 790 |
| Breast cancer | EMT6 | Lapatinib | 169 |
| | | Abemaciclib | 263 |
| | 4T1 | Adriamycin | 307 |
| | | Mitoxantrone | 385 |
| Liver cancer | Hapa1-6 | Sorafenib | 435 |
| | | Everolimus | 892 |
| Colorectal cancer | MC38 | Regorafenib | 134 |
| | | Vistusertib | 175 |
| | | 5-Fluorouracil + Oxaliplatin | 181 |
| Renal cancer | RENCA | Vistusertib | 341 |
| | | Everolimus | 134 |
| | | Sorafenib | 158 |
| Skin cancer (melanoma) | B16F10 | Everolimus | 216 |
| | | Vistusertib | 145 |
| | | Paclitaxel | 186 |
| | | Regorafenib | 166 |

With reference to Table 42, it was confirmed that the expression rates of calreticulin on the cell surfaces of various carcinomas were increased by the anticancer agents used in the present invention. Accordingly, it may be seen that immunogenic cell death is induced by various anticancer agents used in the present invention.

## Claims

1. A pharmaceutical composition for preventing or treating cancer, comprising, as active ingredients:
a naphthoquinone-based compound; and
at least one selected from among an immune checkpoint inhibitor (ICI) and an immunogenic cell death (ICD) inducer.

2. The pharmaceutical composition of claim 1, wherein the composition comprises, as active ingredients, the naphthoquinone-based compound, the immune checkpoint inhibitor, and the immunogenic cell death inducer.

3. The pharmaceutical composition of claim 1, wherein the composition is in the form of a complex dosage form in which the naphthoquinone-based compound is mixed with at least one selected from among the immune checkpoint inhibitor and the immunogenic cell death inducer.

4. The pharmaceutical composition of claim 1, wherein the composition is in the form in which the naphthoquinone-based compound and at least one selected from among the immune checkpoint inhibitor and the immunogenic cell death inducer are each formulated, and simultaneously or sequentially administered.

5. The pharmaceutical composition of claim 1, wherein the naphthoquinone-based compound is orally administered, and the immune checkpoint inhibitor and the immunogenic cell death inducer are intravenously, orally, or intraperitoneally administered.

6. The pharmaceutical composition of claim 1, wherein, based on a single dosage of the composition, the naphthoquinone-based compound and the immune checkpoint inhibitor are co-administered in a weight ratio of 1:0.001 to 1:0.1, and the naphthoquinone-based compound and the immunogenic cell death inducer are co-administered in a weight ratio of 1:0.2 to 1:5.

7. The pharmaceutical composition of claim 1, wherein the immune checkpoint inhibitor comprises two different kinds of immune checkpoint inhibitors.

8. The pharmaceutical composition of claim 1, wherein the immunogenic cell death inducer comprises two different kinds of immunogenic cell death inducers.

9. The pharmaceutical composition of any one of claims 1 to 8, wherein the naphthoquinone-based compound is at least one selected from among compounds represented by Chemical Formulae 1 to 5, pharmaceutically acceptable salts thereof, prodrugs thereof, solvates thereof, or isomers thereof: 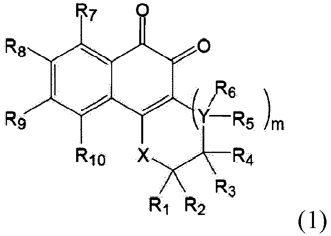 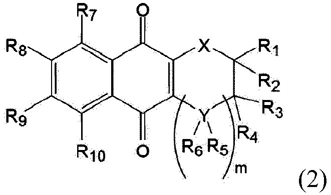 in Chemical Formulae 1 and 2,
R₁ to R₆ are each independently selected from the group consisting of hydrogen, hydroxy, halogen, amino, substituted or unsubstituted C₁-C₁₀ alkylamino, substituted or unsubstituted C₁-C₁₀ dialkylamino, substituted or unsubstituted C₁-C₁₀ alkyl, substituted or unsubstituted C₂-C₁₀ alkenyl, substituted or unsubstituted C₂-C₁₀ alkynyl, substituted or unsubstituted C₁-C₁₀ alkoxy, substituted or unsubstituted C₁-C₁₀ alkoxycarbonyl, substituted or unsubstituted C₁-C₁₀ acyl, substituted or unsubstituted C₃-C₈ cycloalkyl, substituted or unsubstituted 3- to 10-membered heterocycloalkyl, substituted or unsubstituted C₆-C₁₀ aryl, substituted or unsubstituted 5- to 10-membered heteroaryl, and substituted or unsubstituted - (CH₂)ₙ-aryl, or may be each substituted or unsubstituted double bond or C₃-C₆ ring structure, which is formed by mutually linking two substituents among them, and the ring structure may be a saturated structure or a partially or fully unsaturated structure, wherein the substituent may be one or more selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, 3- to 10-membered heterocycloalkyl, C₆-C₁₀ aryl, and 5- to 10-membered heteroaryl;
R₇ to R₁₀ are each independently selected from the group consisting of hydrogen, hydroxy, halogen, amino, substituted or unsubstituted C₁-C₁₀ alkylamino, substituted or unsubstituted C₁-C₁₀ dialkylamino, substituted or unsubstituted C₁-C₁₀ alkyl, substituted or unsubstituted C₂-C₁₀ alkenyl, substituted or unsubstituted C₂-C₁₀ alkynyl, substituted or unsubstituted C₁-C₁₀ alkoxy, substituted or unsubstituted C₁-C₁₀ alkoxycarbonyl, substituted or unsubstituted C₁-C₁₀ acyl, substituted or unsubstituted C₃-C₈ cycloalkyl, substituted or unsubstituted 3- to 10-membered heterocycloalkyl, substituted or unsubstituted C₆-C₁₀ aryl, substituted or unsubstituted 5- to 10-membered heteroaryl, and substituted or unsubstituted - (CH₂)ₙ-aryl, or may be each substituted or unsubstituted C₃-C₆ ring structure which is formed by mutually linking two substituents among them, and the ring structure may be a saturated structure or a partially or fully unsaturated structure, wherein the substituent may be one or more selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₁-C₁₀ alkylamino, C₃-C₈ cycloalkyl, 3- to 10-membered heterocycloalkyl, C₆-C₁₀ aryl, and 5- to 10-membered heteroaryl;
X is O, S, or NR', where R' is hydrogen or C₁-C₆ alkyl;
Y is C, S, N, or O, where when Y is S or O, R₅ and R₆ are not any substituents, and when Y is N, R₅ is hydrogen or C₁-C₆ alkyl and R₆ is not any substituent; and
m is 0 or 1, and when m is 0, adjacent carbon atoms of m are directly boned to each other to form a cyclic structure; and n is an integer of 0 to 10, 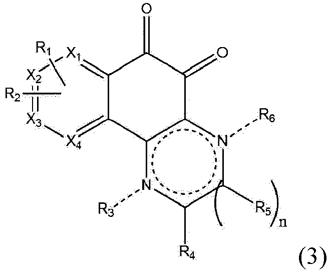 in Chemical Formula 3,
R₁ and R₂ are each independently hydrogen, halogen, substituted or unsubstituted C₁-C₂₀ alkoxy, substituted or unsubstituted C₁-C₆ alkyl, substituted or unsubstituted C₆-C₁₀ aryl, substituted or unsubstituted C₆-C₁₀ aryloxy, substituted or unsubstituted 5- to 10-membered heteroaryl, -NO₂, -NR'₁R'₂, -NR'₁(CO(O)R'₂), - NR'₁(C(O)NR'₁R'₂), -C(O)NR'₁R'₂, -CN, -SO(O)R'₁, -SO(O)NR'₁R'₂, - NR'₁(SO(O)R'₂), or -CSNR'₁R'₂, or R₁ and R₂ may be bonded to each other to form a cyclic structure of substituted or unsubstituted C₆-C₁₀ aryl or a cyclic structure of substituted or unsubstituted 5- to 10-membered heteroaryl;
wherein R'₁ and R'₂ are each independently hydrogen, substituted or unsubstituted C₁-C₆ alkyl, substituted or unsubstituted C₃-C₈ cycloalkyl, substituted or unsubstituted C₆-C₁₀ aryl, substituted or unsubstituted C₆-C₁₀ aryloxy, substituted or unsubstituted 5- to 10-membered heteroaryl, substituted or unsubstituted - (CR"₁R"₂)ₘ^{,}-C₆-C₁₀ aryl, or substituted or unsubstituted NR"₁R"₂; wherein R"₁ and R"₂ are each independently hydrogen or C₁-C₃ alkyl, or R"₁ and R"₂ may be bonded to each other to form a cyclic structure of substituted or unsubstituted C₆-C₁₀ aryl;
R₃, R₄, R₅, and R₆ are each independently hydrogen, halogen, substituted or unsubstituted C₁-C₉ alkyl, substituted or unsubstituted C₂-C₂₀ alkene, substituted or unsubstituted C₁-C₂₀ alkoxy, substituted or unsubstituted C₃-C₈ cycloalkyl, substituted or unsubstituted 3- to 10-membered heterocycloalkyl, substituted or unsubstituted C₆-C₁₀ aryl, substituted or unsubstituted C₆-C₁₀ aryloxy, substituted or unsubstituted 5- to 10-membered heteroaryl, substituted or unsubstituted - (CR'₅R'₆)ₘ-C₆-C₁₀ aryl, substituted or unsubstituted -(CR'₅R'₆)ₘ-C₆-C₁₀ aryloxy, substituted or unsubstituted -(CR'₅R'₆)ₘ-5- to 10-membered heteroaryl, substituted or unsubstituted -(CR'₅R'₆)ₘ-3- to 10-membered heterocycloalkyl, substituted or unsubstituted -(CR'₅R'₆)ₘ-NR'₃R'₄, substituted or unsubstituted -(CR'₅R'₆)ₘ-OR'₃, - CO(O)R'₃, -CONR'₃R'₄, -NR'₃R'₄, -NR'₃(C(O)R'₄), -SO(O)R'₃, -SO(O)NR'₃R'₄, - NR'₃(SO(O)R'₄), -CSNR'₃R'₄, -CH₂A when the compound represented by Chemical Formula 3 is "A", or -A when the compound represented by Chemical Formula 3 is "A";
wherein R'₃ and R'₄ are each independently hydrogen, substituted or unsubstituted C₁-C₆ alkyl, substituted or unsubstituted C₃-C₈ cycloalkyl, substituted or unsubstituted C₆-C₁₀ aryl, substituted or unsubstituted -(CH₂)ₘ-C₆-C₁₀ aryl, substituted or unsubstituted -(CH₂)ₘ-C₆-C₁₀ aryloxy, or -CO(O)R"₃, or R'₃ and R'₄ may be bonded to each other to form a cyclic structure of substituted or unsubstituted 3- to 10-membered heterocycloalkyl, or a cyclic structure of substituted or unsubstituted 5- to 10-membered heteroaryl;
R'₅ and R'₆ are each independently hydrogen or C₁-C₃ alkyl; and R"₃ is C₁-C₆ alkyl;
wherein the substituent is one or more selected from the group consisting of hydroxy, halogen, C₁-C₁₀ alkyl, C₂-C₁₀ alkenyl, C₂-C₁₀ alkynyl, C₁-C₁₀ alkoxy, C₁-C₁₀ alkoxycarbonyl, C₃-C₈ cycloalkyl, 3- to 10-membered heterocycloalkyl, C₆-C₁₀ aryl, and 5- to 10-membered heteroaryl;
with a proviso that structures in which R₃ and R₄ are each independently C₆-C₁₀ aryl are excluded, structures in which R₄ and R₆ are each independently C₆-C₁₀ aryl are excluded, structures in which R₄ is hydrogen, methyl, halogen-substituted methyl, or piperidinylmethyl when R₃ has the structure defined above are excluded, and structures in which R₅ is phenyl are excluded;
m and m' are each independently a natural number of 1 to 4;
the heteroatom is one or more selected from among N, O, and S;
X₁, X₂, X₃, and X₄ are each independently CH or N; and
n is 0 or 1, and when n is 0, adjacent carbon atoms of n are directly boned to each other to form a cyclic structure, 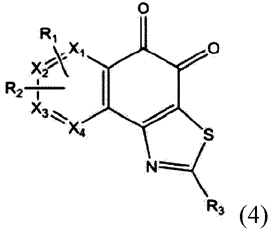 in Chemical Formula 4,
R₁ and R₂ are each independently hydrogen, a halogen atom, hydroxy, substituted or unsubstituted C₁-C₂₀ alkoxy, substituted or unsubstituted C₁-C₁₀ alkyl, substituted or unsubstituted C₄-C₁₀ aryl, substituted or unsubstituted C₄-C₁₀ aryloxy, substituted or unsubstituted C₂-C₁₀ heteroaryl, -NO₂, -NR'₁R'₂, -NR'₁(CO(O)R'₂), - NR'₁(C(O)NR'₁R'₂), -CO(O)R'₁, -C(O)NR'₁R'₂, -CN, -SO(O)R'₁, -SO(O)NR'₁R'₂, - NR'₁(SO(O)R'₂), or -CSNR'₁R'₂, or R₁ and R₂ may be bonded to each other to form a cyclic structure of substituted or unsubstituted C₄-C₁₀ aryl or a cyclic structure of substituted or unsubstituted C₂-C₁₀ heteroaryl;
wherein R'₁ and R'₂ are each independently hydrogen, substituted or unsubstituted C₁-C₆ alkyl, substituted or unsubstituted C₃-C₈ cycloalkyl, substituted or unsubstituted C₄-C₁₀ aryl, substituted or unsubstituted C₄-C₁₀ aryloxy, substituted or unsubstituted C₁-C₈ heteroaryl, substituted or unsubstituted -(CR"₁R"₂)ₘ^{,}-C₄-C₁₀ aryl, substituted or unsubstituted -(CR"₁R"₂)ₘ^{,}-C₄-C₁₀ heteroaryl, or substituted or unsubstituted NR"₁R"₂; wherein R"₁ and R"₂ are each independently hydrogen or C₁-C₃ alkyl, or R"₁ and R"₂ may be bonded to each other to form a cyclic structure of substituted or unsubstituted C₄-C₁₀ aryl;
R₃ is hydrogen, hydroxy, a halogen atom, substituted or unsubstituted C₁-C₁₀ alkyl, substituted or unsubstituted C₂-C₂₀ alkene, substituted or unsubstituted C₁-C₂₀ alkoxy, substituted or unsubstituted C₃-C₈ cycloalkyl, substituted or unsubstituted C₂-C₈ heterocycloalkyl, substituted or unsubstituted C₄-C₁₀ aryl, substituted or unsubstituted C₄-C₁₀ aryloxy, substituted or unsubstituted C₁-C₁₀ heteroaryl, substituted or unsubstituted -(CR'₅R'₆)ₘ-C₄-C₁₀ aryl, substituted or unsubstituted - (CR'₅R'₆)ₘ-C₄-C₁₀ aryloxy, substituted or unsubstituted -(CR'₅R'₆)ₘ-C₁-C₁₀ heteroaryl, substituted or unsubstituted -(CR'₅R'₆)ₘ-NR'₃R'₄, substituted or unsubstituted -(CR'₅R'₆)ₘ-C₂-C₁₀ heterocycloalkyl, substituted or unsubstituted - (CR'₅R'₆)ₘ-OR'₃, substituted or unsubstituted -(CR'₅R'₆)ₘ(O)COR'₃, -CO(O)R'₃, - CONR'₃R'₄, -NR'₃R'₄, -NR'₃(C(O)R'₄), -CH₂A when the compound represented by Chemical Formula 4 is "A", or -A when the compound represented by Chemical Formula 4 is "A";
wherein R'₃ and R'₄ are each independently hydrogen, substituted or unsubstituted C₁-C₆ alkyl, substituted or unsubstituted C₃-C₈ cycloalkyl, substituted or unsubstituted C₄-C₁₀ aryl, substituted or unsubstituted -(CR'₅R'₆)ₘ-C₄-C₁₀ aryl, substituted or unsubstituted -(CR'₅R'₆)ₘ-C₄-C₁₀ aryloxy, substituted or unsubstituted -(CR'₅R'₆)ₘ-C₁-C₁₀ heteroaryl, or -CO(O)R‴₃, or R'₃ and R'₄ may be bonded to each other to form a cyclic structure of substituted or unsubstituted C₄-C₁₀ heterocycloalkyl or a cyclic structure of substituted or unsubstituted C₁-C₁₀ heteroaryl;
R'₅ and R'₆ are each independently hydrogen or C₁-C₃ alkyl; and R‴₃ is C₁-C₆ alkyl;
wherein the substituent may be one or more selected from the group consisting of hydroxy, a nitro group, a halogen atom, C₁-C₁₀ alkyl, C₂-C₁₀ alkenyl, C₂-C₁₀ alkynyl, C₁-C₁₀ alkoxy, C₁-C₁₀ alkoxycarbonyl, C₃-C₈ cycloalkyl, C₃-C₈ heterocycloalkyl, C₄-C₁₀ aryl, and C₅-C₁₀ heteroaryl;
X₁, X₂, X₃, and X₄ are each independently CH or N;
m and m' are each independently a natural number of 1 to 4; and the heteroatom is one or more selected from among N, O, and S, 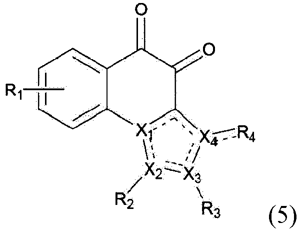 in Chemical Formula 5,
X₁, X₂, X₃, and X₄ are each independently selected from the group consisting of carbon, nitrogen, oxygen, and sulfur atoms, and at least two among X₁, X₂, X₃, and X₄ are heteroatoms selected from among nitrogen, oxygen, and sulfur atoms, with a proviso that when X₁ and X₂ are each carbon atom, X₃ and X₄ is not be nitrogen atoms at the same time;
R₁ is one or more selected from the group consisting of hydrogen, alkyl, alkyloxy, halo, nitro, hydroxy, cyano, and -NR₅R₆;
R₂ is absent or selected from the group consisting of hydrogen, oxygen, alkyl, alkyloxy, C₆₋₁₀ aryl, and heterocyclyl, wherein the alkyl may be substituted with C₆-₁₀ aryl, and the heterocyclyl may be substituted with -C(O)Rs;
R₃ is absent or selected from the group consisting of hydrogen, oxygen, halo, alkyl, alkyloxy, C₆-₁₀ aryl, heterocyclyl, -SO₂NR₇R₁₂, -NR₉R₁₀, and -C(O)R₁₁, wherein when the alkyl is substituted, the substituent is selected from the group consisting of halo, alkyloxy, C₆-₁₀ aryl, C₆₋₁₀ aryloxy, heterocyclyl, -C(O)R₈, R₁₂C(O)O-, and -NR₁₃R₁₄, and the heterocyclyl may be substituted with -C(O)Rs;
R₄ is absent or selected from the group consisting of hydrogen, oxygen, alkyl, alkyloxy, C₆₋₁₀ aryl, C₆-₁₀ aryloxy, heterocyclyl, and -C(O)R₁₅, wherein when the alkyl is substituted, the substituent is selected from the group consisting of halo, C₆-₁₀ aryl, heterocyclyl, and -C(O)R₈, and the heterocyclyl may be substituted with - C(O)R₈;
R₅ and R₆ are each independently selected from the group consisting of hydrogen, alkyl, and -C(O)R₇;
R₇ and R₁₂ are each alkyl;
R₁₁ is heterocyclyl or -NR₁₃R₁₄;
R₁₅ is alkyl, alkyloxy, C₆-₁₀ aryloxy, heterocyclyl, or -NR₁₃R₁₄;
R₉, R₁₀, R₁₃, and R₁₄ are each independently selected from the group consisting of hydrogen, alkyl, unsubstituted or halo-substituted C₆-₁₀ aryl, and - C(O)R₈;
Rs's are each alkyloxy;
wherein the alkyl may be linear or branched alkyl having 1 to 10 carbon atoms or cyclic alkyl having 3 to 7 carbon atoms, the heterocyclyl is a 3- to 7-membered heterocyclic group having, in the ring, one or more heteroatoms selected from the group consisting of a nitrogen atom, an oxygen atom, and a sulfur atom, and when the aryl is substituted, the substituents are each one or more selected from the group consisting of halo, C₁-₆ alkyl, halo-substituted alkyl, and alkyloxy; and
- - - is a single bond or a double bond depending on R₂, R₃, R₄, X₁, X₂, X₃, and X₄,
with a proviso that when X₁ and X₄ are each carbon atom and X₂ and X₃ are each nitrogen atom, one among R₂ and R₄ is not alkyl, aryl, or heterocyclyl, wherein when R₂ is alkyl, aryl, or heterocyclyl, R₄ is not -C(O)R₁₅.

10. The pharmaceutical composition of claim 9, wherein the compound represented by Chemical Formula 1 is a compound represented by Chemical Formula 1-3: 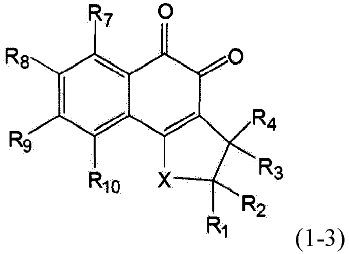 in Chemical Formula 1-3,
X, R₁, R₂, R₃, R₄, R₇, R₈, R₉, and R₁₀ in the formula are as defined in Chemical Formula 1 of claim 9.

11. The pharmaceutical composition of claim 1, wherein the naphthoquinone-based compound is dunnione (2,3,3-trimethyl-2H-benzo[g][1]benzofuran-4,5-dione).

12. The pharmaceutical composition of any one of claims 1 to 8, wherein the immune checkpoint inhibitor targets any one immune checkpoint selected from the group consisting of CTLA4, PD-1, PD-L1, LAG3, B7-H3, B7-H4, KIR, OX40, IgG, IDO-1, IDO-2, CEACAM1, BTLA, OX40L, TIM3, and combinations thereof.

13. The pharmaceutical composition of claim 12, wherein the immune checkpoint inhibitor is any one selected from the group consisting of: an anti-CTLA4 antibody, an antigen-binding fragment thereof, or a variant thereof; an anti-PD-L1 antibody, an antigen-binding fragment thereof, or a variant thereof; an anti-PD-1 antibody, an antigen-binding fragment thereof, or a variant thereof; an anti-LAG3 antibody, an antigen-binding fragment thereof, or a variant thereof; and combinations thereof.

14. The pharmaceutical composition of any one of claims 1 to 8, wherein the immunogenic cell death inducer is any one selected from the group consisting of capecitabine, 5-fluorouracil, thioguanine, chlorambucil, oxaliplatin, cisplatin, carboplatin, paclitaxel, docetaxel, irinotecan, doxorubicin, vinorelbine, gemcitabine, pemetrexed, adriamycin, etoposide, vincristine, cytarabine, cyclophosphamide, ifosfamide, tamoxifen, anastrozole, letrozole, exemestane, fulvestrant, temozolomide, carmustine, lomustine, epirubicin, eribulin, toremifene, goserelin, megestrol, vinblastine, bendamustine, thiotepa, bleomycin, topotecan, leucovorin, trifluridine, tipiracil, mitoxantrone, mitomycin C, aldesleukin, temsirolimus, everolimus, mechlorethamine, methotrexate, pemetrexed, trastuzumab, bevacizumab, cetuximab, aflibercept, pertuzumab, ramucirumab, panitumumab, nivolumab, necitumumab, pembrolizumab, obinutuzumab, ofatumumab, erlotinib, gefitinib, sorafenib, lapatinib, dinaciclib, palbociclib, regorafenib, imatinib, sunitinib, axitinib, pazopanib, afatinib, ceritinib, crizotinib, osimertinib, bosutinib, dasatinib, nilotinib, ponatinib, hydroxyurea, procarbazine, abemaciclib, vistusertib, and combinations thereof.

15. The pharmaceutical composition of claim 1, wherein the pharmaceutical composition comprises:
dunnione;
any one immune checkpoint inhibitor selected from the group consisting of an anti-CTLA4 antibody, an anti-PD-L1 antibody, an anti-PD-L1 antibody, an anti-PD-L2 antibody, an anti-LAG3 antibody, an anti-B7-H3 antibody, an anti-B7-H4 antibody, an anti-HVEM antibody, an anti-KIR antibody, an anti-OX40 antibody, an anti-IgG antibody, an anti-IDO-1 antibody, an anti-IDO-2 antibody, an anti-CEACAM1 antibody, an anti-BTLA antibody, an anti-OX40L antibody, an anti-TIM3 antibody, an anti-GAL9 antibody, an anti-VISTA antibody, an anti-TIGIT antibody, and combinations thereof; and
any one immunogenic cell death inducer selected from the group consisting of capecitabine, 5-fluorouracil, thioguanine, chlorambucil, oxaliplatin, cisplatin, carboplatin, paclitaxel, docetaxel, irinotecan, doxorubicin, vinorelbine, gemcitabine, pemetrexed, adriamycin, etoposide, vincristine, cytarabine, cyclophosphamide, ifosfamide, tamoxifen, anastrozole, letrozole, exemestane, fulvestrant, temozolomide, carmustine, lomustine, epirubicin, eribulin, toremifene, goserelin, megestrol, vinblastine, bendamustine, thiotepa, bleomycin, topotecan, leucovorin, trifluridine, tipiracil, mitoxantrone, mitomycin C, aldesleukin, temsirolimus, everolimus, mechlorethamine, methotrexate, pemetrexed, trastuzumab, bevacizumab, cetuximab, aflibercept, pertuzumab, ramucirumab, panitumumab, nivolumab, necitumumab, pembrolizumab, obinutuzumab, ofatumumab, erlotinib, gefitinib, sorafenib, lapatinib, dinaciclib, palbociclib, regorafenib, imatinib, sunitinib, axitinib, pazopanib, afatinib, ceritinib, crizotinib, osimertinib, bosutinib, dasatinib, nilotinib, ponatinib, hydroxyurea, procarbazine, abemaciclib, vistusertib, and combinations thereof.

16. The pharmaceutical composition of any one of claims 1 to 8, wherein the cancer is any one selected from the group consisting of liver cancer, gastric cancer, colon cancer, breast cancer, lung cancer, non-small cell lung cancer, bone cancer, pancreatic cancer, skin cancer, head or neck cancer, skin or intraocular melanoma, uterine cancer, ovarian cancer, colorectal cancer, small intestine cancer, rectal cancer, perianal cancer, fallopian tube carcinoma, endometrial carcinoma, cervical carcinoma, vaginal carcinoma, vulva carcinoma, Hodgkin's disease, esophageal cancer, lymph gland cancer, bladder cancer, gallbladder cancer, endocrine gland cancer, thyroid cancer, parathyroid cancer, adrenal cancer, soft tissue sarcoma, urethral cancer, penile cancer, prostate cancer, adenocarcinoma, chronic or acute leukemia, lymphocytic lymphoma, kidney or ureter cancer, renal cell carcinoma, renal pelvic carcinoma, central nervous system (CNS) tumor, primary CNS lymphoma, spinal cord tumor, brainstem glioma, pituitary adenoma, and combinations thereof.

17. A use of a composition for preventing or treating cancer, wherein the composition contains a naphthoquinone-based compound, and at least one selected from among an immune checkpoint inhibitor (ICI) and an immunogenic cell death (ICD) inducer.
